# EUROPEAN PATENT APPLICATION

(11) **EP 3 680 338 A1**
(43) Date of publication of application: **15.07.2020**
(21) Application number: 18854823.4
(22) Date of filing: 07.09.2018
(51) Int. Cl.: C12N 15/87, A61K 48/00, A61P 35/00

(54) **GENETICALLY ENGINEERED T CELL AND APPLICATION THEREOF**

(30) Priority: 08.09.2017 CN 201710805991; 22.09.2017 CN 201710867308; 07.12.2017 CN 201711287300
(71) Applicant: Carsgen Therapeutics Co., Ltd., Xuhui District Shanghai 200231 (CN); Shanghai Cancer Institute, Shanghai 200032 (CN)
(72) Inventor: LI, Zonghai, Shanghai 200231 (CN); GUO, Xingliang, Shanghai 200231 (CN)
(74) Representative: Lavoix
(86) International application number: PCT/CN2018/104669
(87) International publication number: WO 2019/047932

(57) **Abstract**

Provided are a genetically engineered T cell and a pharmaceutical composition using the same. The T cell has an endogenous T cell receptor (TCR) that is inactivated or inactive, comprises encoding of an exogenous receptor bindable to a target antigen, and contains an open reading frame regulated by the exogenous receptor. When the exogenous receptor binds to the target antigen, expression of the open reading frame can be initiated. The T cell has a killing effect on tumor cells and has reduced side effects and enhanced safety.

## Description

### Technical field

The invention belongs to the field of biomedicine, and in particular relates to a T lymphocyte that specifically induces the expression of cytokines, such as IL-12.

### Background

Tumor immunotherapy has received extensive attention and application in recent years, especially the CAR-T technology, which represents a landmark of tumor control for human.

Some antitumor active substances, such as IL12 have good antitumor activity, but at the same time have strong toxic effects and may even be fatal (Cohen, J (1995). IL-12 deaths: explanation and a puzzle. Science 270: 908). Therefore, antitumor active substances, such as IL12 are selectively and highly expressed in local area of a tumor by using T lymphocytes through inducible promoters is undoubtedly a way to improve efficacy and reduce toxic and side effects. For example, the expression of IL12 can be regulated using NFAT6 promoter to in T cells, and then only when T cells are activated, can NFAT6 be effectively activated to allow IL12 to be expressed (Zhang L1, Kerkar SP Improving adoptive T cell therapy by targeting and controlling IL-12 expression to the tumor environment. Mol Ther. 2011 Apr; 19 (4): 751-9). However, there are reports in the literature that when TIL carrying IL12 gene under the control of NFAT6 promoter was clinically studied, strong toxic side effects were found, although this treatment method was found to have good antitumor activity (Zhang L1, Morgan RA1, Tumor-infiltrating lymphocytes genetically engineered with an induciblegene encoding interleukin-12 for the immunotherapy of metastaticmelanoma. Clin Cancer Res. 2015 May 15; 21 (10): 2278-88). The reason may be that IL-12 expression is caused by non-target activation of T cells.

Therefore, there is an urgent need in the art for a technical means to well control the expression of a target gene, such as IL12.

### Summary of the invention

An object of the present invention is to provide a T lymphocyte that specifically induces the expression of an exogenous gene, such as a cytokine, and simultaneously expresses an exogenous receptor that can bind a target antigen and can trigger activation of CD3 signal.

In a first aspect, a genetically engineered T cell is provided in the present invention, wherein the endogenous T cell receptor (TCR) in said T cell is inactivated or inactive; said T cell comprises an open reading frame, the open reading frame encodes an exogenous receptor capable of binding to a target antigen and is regulated by the exogenous receptor, and when the exogenous receptor binds to the target antigen, the expression of the open reading frame can be initiated.

In a specific embodiment, the open reading frame is regulated by the exogenous receptor through a promoter.

In a specific embodiment, the exogenous receptor can trigger the activation of CD3 signal, and the promoter activity is regulated by TCR signal activation.

In a preferred embodiment, the exogenous receptor binds to the target antigen to trigger the activation of CD3 signal.

In a specific embodiment, the T cell comprises the nucleic acid of an open reading frame operably linked to the promoter, the promoter activity is regulated by the activation of TCR signal, the exogenous receptor can trigger the activation of CD3 signal, and when the exogenous receptor binds to the target antigen, the promoter regulates the expression of the open reading frame.

In a specific embodiment, the T cell does not express an endogenous T cell receptor (TCR).

In a specific embodiment, the endogenous TCR is treated by using gene knockout technology or gene silencing technology, so that the endogenous TCR is inactive; preferably, the endogenous TCR gene is knocked out by using gene site-directed knockout technology. The gene site-directed knockout technology includes CRISPR / Cas9 technology, Zinc Finger Nucleases (ZFN) technology, transcription activator-like effector (TALE) technology, or TALE-CRISPR / Cas9 technology; and more preferably, CRISPR / Cas9 technology is used.

In a specific embodiment, the gene knockout or gene silencing is performed in the constant region of one or both of the α and β chains of the TCR;
preferably, the exon of the corresponding encoding gene in the constant region of one or both of the α and β chains of the TCR is subjected to gene knockout or gene silencing.

In a specific embodiment, gene knockout or gene silencing is performed in the constant region of the α chain of the TCR,
preferably, the first exon in the constant region of the endogenous TCRα chain is subjected to gene knockout or gene silencing.

In a specific embodiment, the promoter comprises a binding motif of a transcription factor that depends on the activation of TCR signal.

In a specific embodiment, the promoter comprises a minimal promoter operably linked to a binding motif of a transcription factor that depends on the activation of TCR signal.

In a specific embodiment, the minimal promoter is a minimal promoter of cytokine, including a minimal promoter of interleukin, interferon, tumor necrosis factor superfamily, colony stimulating factor, chemokine, and growth factor; preferably minimal promoter of IFN -γ, TNF-α, or IL-2, more preferably minimal promoter of IL-2.

In a specific embodiment, the binding motif includes a NFAT, NF-κB or AP-1 binding motif or a combination thereof; preferably a NFAT binding motif, more preferably two or more NFAT binding motifs; most preferably 6 NFAT-binding motifs.

In a specific embodiment, the minimal promoter is encoded by a nucleotide sequence having 90% identity with SEQ ID NO: 8.

In a specific embodiment, the open reading frame includes an open reading frame of cytokine, immunotoxin, cytotoxic protein, antibody drug, or bifunctional antibody; and the cytokine is preferably IFN-α, IFN-β, IFN -γ; Interleukin 2, 3, 4, 5, 6, 8, 12, 13, 22, 23, 24; TNF-α, GM-CSF, CD40L, CTLA-4, FLT3L, TRAIL or LIGHT, more preferably IL-12.

In a specific embodiment, the exogenous receptor includes chimeric antigen receptor (CAR), modified T cell (antigen) receptor (TCR), T cell fusion protein (TFP), and T cell antigen coupler (TAC) or a combination thereof.

In a specific embodiment, the modified TCR is selected from wild TCR, high affinity TCR, or chimeric TCR.

In a specific embodiment, the chimeric antigen receptor comprises an antigen-binding domain, transmembrane domain and intracellular domain.

In a specific embodiment, the chimeric antigen receptor includes:
(i) an antibody that specifically binds to said antigen, a transmembrane region of CD28, a costimulatory signal domain of CD28, and a CD3ζ fusion peptide; or
(ii) an antibody that specifically binds to said antigen, a transmembrane region of CD28, a co-stimulatory signal domain of CD137, and a CD3ζ fusion peptide; or
(iii) an antibody that specifically binds to the antigen, a transmembrane region of CD28, a costimulatory signal domain of CD28, a costimulatory signal domain of CD137, and a CD3ζ fusion peptide.

In a specific embodiment, the TFP includes:
(a) a TCR subunit comprising:
   Part of TCR extracellular domain, transmembrane domain, and TCR intracellular domain, and said intracellular domain includs a stimulatory signaling domain;
(b) an antibody domain having an antigen-binding domain;
wherein the TCR subunit is operably connected to the antibody domain, the extracellular, transmembrane, and intracellular signaling domains of the TCR subunit are derived from CD3ε or CD3y, and the TFP is integrated into the TCR expressed on T cells.

In a specific embodiment, the TAC includes:
(a) an extracellular domain: the extracellular domain includes an antibody domain having an antigen-binding domain, and a single-chain antibody that binds to CD3;
(b) a transmembrane region;
(c) an intracellular domain connected to the protein kinase LCK.

In a specific embodiment, the target antigen includes a tumor antigen, a pathogen antigen.

In a preferred embodiment, the tumor antigen includes a solid tumor antigen or a liquid tumor antigen, preferably a solid tumor antigen.

In a preferred example, the solid tumor includes, but is not limited to, sarcoma and cancer (such as fibrosarcoma, myxosarcoma, liposarcoma, chondrosarcoma, osteosarcoma, chordoma, endothelial sarcoma, lymphangiosarcoma, angiosarcoma, lymphangiothelioma, synovial tumor, mesothelioma, Ewing's tumor, leiomyosarcoma, rhabdomyosarcoma, colon cancer, pancreatic cancer, breast cancer, ovarian cancer, prostate cancer, squamous cell carcinoma, basal cell carcinoma, adenocarcinoma, sweat adenocarcinoma, sebaceous adenocarcinoma, papillary carcinoma, papillary adenocarcinoma, carcinoma, bronchial carcinoma, myeloid cancer, renal cell carcinoma, liver cancer, nile duct cancer, choriocarcinoma, seminal cell tumor, embryo cancer, nephroblastoma, cervical cancer, uterine cancer, testicular cancer, lung cancer, small cell lung cancer, bladder cancer, epithelial cancer, glue plasma tumors, astrocytomas, medulloblastomas, craniopharyngiomas, ependymal tumors, pineal tumors, hemangioblastomas, acoustic neuromas, oligodendroglioma, schwannomas, meningiomas, melanoma, neuroblastoma, retinoblastoma), esophageal cancer, gallbladder cancer, kidney cancer, multiple myeloma; preferably pancreatic cancer , liver cancer, lung cancer, stomach cancer, esophageal cancer, head and neck squamous cell carcinoma, prostate cancer, colon cancer, breast cancer, lymphoma, gallbladder cancer, kidney cancer, leukemia, multiple myeloma, ovarian cancer, cervical cancer and glioma; more preferably pancreatic cancer, liver cancer, lung cancer and gastric cancer.

In a preferred example, the tumor antigen is selected from the group consisting of: Thyroid stimulating hormone receptor (TSHR); CD171; CS-1; C-type lectin-like molecule-1 (CLL-1); ganglioside GD3; Tn antigen; CD19; CD20; CD22; CD30; CD70; CD123; CD138; CD33; CD44; CD44v7 / 8; CD38; CD44v6; B7H3 (CD276), B7H6; KIT (CD117); Interleukin 13 receptor subunit α (IL-13Ra); Interleukin 11 receptor α (IL-11Ra); prostate stem cell antigen (PSCA); prostate-specific membrane antigen; carcinoembryonic antigen; NY-ESO-1; HIV-1 Gag; MART-1; gp100; tyrosinase; mesothelin; EpCAM; protease serine 21 (PRSS21); Vascular endothelial growth factor receptor; vascular endothelial growth factor receptor 2 (VEGFR2); Lewis (Y) antigen; CD24; platelet-derived growth factor receptor β (PDGFR-β); stage-specific embryonic antigen-4 (SSEA -4); MUC1; MUC6; epidermal growth factor receptor family and mutants thereof (EGFR, EGFR2, ERBB3, ERBB4, EGFRvIII); nerve cell adhesion molecule (NCAM); carbonic anhydrase IX (CAIX); proteasome (Prosome, Macropain) subunit, beta type, 9 (LMP2); hepatin A receptor 2 (EphA2); fucosyl GM1; sialic acid Lewis adhesion molecule (sLe); Ganglioside GM3; high molecular weight melanoma-associated antigen (HMWMAA); o-acetyl GD2 ganglioside (OAcGD2); folate receptor; tumor vascular endothelial marker 1 (TEM1 / CD248); tumor vascular endothelial marker 7 (TEM7R); claudin 6 (CLDN6), Claudin 18.2, Claudin 18.1; ASGPR1; CDH16; 5T4; 8H9; αvβ6 integrin; B cell mature antigen (BCMA); CA9; κ light chain; CSPG4; EGP2, EGP40 FAP; FAR; FBP; embryonic AchR; HLA-A1; HLA-A2; MAGE A1, MAGE3; KDR; Lambda; MCSP; NKG2D ligand; PSC1; ROR1; Sp17; SURVIVIN; TAG72; TEM1; fibronectin; tenascin; carcinoembryonic variant of tumor necrosis zone; G protein-coupled receptor class C group 5, member D (GPRC5D); X chromosome open reading frame 61 (CXORF61); CD97; CD179a; anaplastic lymphoma kinase (ALK); polysialic acid; placental specificity 1 (PLAC1); hexose moiety of GloH glycoceramide (GloboH); breast differentiation antigen (NY-BR-1); uroplakin 2 (UPK2); hepatitis A virus cell receptor 1 (HAVCR1); epinephrine receptor β3 (ADRB3); pannexin 3 (PANX3); G protein coupled receptor 20 (GPR20); lymphocytes Pro 6 complex, locus K9 (LY6K); olfactory receptor 51E2 (OR51E2); TCRy alternate reading frame protein (TARP); nephroblastoma protein (WT1); ETS translocation variant gene 6, located on chromosome 12p (ETV6 -AML); sperm protein 17 (SPA17); X antigen family, member 1A (XAGE1); angiopoietin-binding cell surface receptor 2 (Tie2); melanoma cancer testis antigen-1 (MAD-CT-1); Melanoma cancer testis antigen-2 (MAD-CT-2); Fos-related antigen 1; p53 mutant; human telomerase reverse transcriptase (hTERT); sarcoma translocation breakpoint; melanoma inhibition of apoptosis Agent (ML-IAP); ERG (transmembrane protease, serine 2 (TMPRSS2) ETS fusion gene); N-acetylglucosaminyl transferase V (NA17); paired box protein Pax-3 (PAX3); androgen receptor Cyclin B1; V-myc avian myeloma disease virus oncogene neuroblastoma-derived homolog (MYCN); Ras homolog family member C (RhoC); cytochrome P450 1B1 (CYP1B1); CCCTC-binding factor (zinc finger protein) -like (BORIS); squamous cell carcinoma antigen 3 (SART3) recognized by T cells; paired box protein Pax-5 (PAX5); proacrosin-binding protein sp32 (OYTES1); Lymphocyte-specific protein tyrosine kinase (LCK); A kinase anchor protein 4 (AKAP-4); synovial sarcoma, X breakpoint 2 (SSX2); CD79a; CD79b; CD72; leukocyte-associated immunoglobulin-like receptor 1 (LAIR1); Fc fragment of IgA receptor (FCAR); leukocyte immunoglobulin-like receptor subfamily member 2 (LILRA2); CD300 molecular-like family member f (CD300LF); type C lectin domain family 12 member A (CLEC12A); Bone marrow stromal cell antigen 2 (BST2); mucin-like hormone receptor-like 2 (EMR2) containing EGF-like modules; lymphocyte antigen 75 (LY75); phosphatidylinositol proteoglycan-3 (GPC3); Fc receptor Body-like 5 (FCRL5); immunoglobulin lambda-like polypeptide 1 (IGLL1);

In a preferred example, the pathogen antigen is selected from the group consisting of an antigen from virus, bacteria, fungus, protozoan, or parasite; preferably, the virus antigen is selected from the group consisting of a cytomegalovirus antigen, Epstein-Barr virus antigen, or human immunodeficiency virus antigen or influenza virus antigen.

In a specific embodiment, the nucleic acid of the exogenous receptor that can bind to the target antigen and trigger the activation of CD3 signal are linked together with the nucleic acid of the open reading frame operably connected to the promoter directly or through a linker molecule.

In a specific embodiment, the amino acid sequence of the exogenous receptor that can bind to the target antigen and trigger the activation of CD3 signal is a sequence having at least 90% identity with the sequence as shown in SEQ ID NO: 24, 25, 26, 27, 29, 30, 31, 32, 33, 34, 35, 36, 37, 38, 39, 40, 41, 42, or 43.

In a specific embodiment, the nucleic acid of the open reading frame operably connected to the promoter is encoded by a nucleotide sequence having 90% identity with SEQ ID NO: 44, 45, or 46.

In a second aspect, the use of the T cells according to the first aspect is provided in the present invention for preparing a pharmaceutical composition for treating a tumor, a pathogen infection in an individual in need thereof, or enhancing the immune tolerance of an individual.

In a third aspect, an expression construct is provided in the present invention, comprising: the expression cassette of the exogenous receptor that can bind to the target antigen and trigger the activation of CD3 signal according to the first aspect linked together with the expression cassette of the open reading frame operably connected to the promoter directly or through a linker molecule.

In a fourth aspect, the method for preparing T cells according to the first aspect is provided in the present invention, including following steps:
1) the endogenous TCR is treated by using gene knockout technology or gene silencing technology, so that the endogenous TCR is inactive; preferably, the endogenous TCR gene is knocked out by using a gene site-directed knockout technology; the gene site-directed knockout technology includes CRISPR / Cas9 technology, Zinc Finger Nucleases (ZFN) technology, transcription activator-like effector (TALE) technology, or TALE-CRISPR / Cas9 technology; and more preferably, CRISPR / Cas9 technology is used;
2) the T cells obtained as said above are infected by a virus carrying a nucleic acid encoding an exogenous receptor capable of binding to a target antigen and a nucleic acid of an open reading frame regulated by the exogenous receptor, wherein when the exogenous receptor binds to the target antigen, the expression of the open reading frame can be initiated.

In a preferred embodiment, in step 2), the T cells obtained as said above are infected by a virus carrying a nucleic acid encoding an exogenous receptor that binds to the target antigen and trigger the activation of CD3 signal and a nucleic acid of the open reading frame operably linked to the promoter.

In a preferred embodiment, the open reading frame is regulated by the exogenous receptor via the promoter.

In a preferred embodiment, the exogenous receptor can trigger the activation of CD3 signal, and the promoter activity is regulated by the activation of TCR signal.

In a preferred embodiment, binding of the exogenous receptor to the target antigen triggers activation of CD3 signal.

In a preferred embodiment, the T cell comprises a nucleic acid of the open reading frame operably linked to the promoter, the promoter activity is regulated by the activation of TCR signal, the exogenous receptor can trigger the activation of CD3 signal, and when the exogenous receptor binds to the target antigen, the promoter regulates the expression of the open reading frame.

In a specific embodiment, the exon of the corresponding encoding gene in the constant region of one or both of the α and β chains of the TCR is subjected to gene site-directed knockout technology, so that the endogenous TCR is inactive, and preferably the first exon in the constant region of α chain of the endogenous TCR is site-directed knocked out.

In a specific embodiment, the T cells are derived from PBMC, umbilical cord blood cells, purified T cell population, T cell line, and / or genetically engineered T cells.

In a fifth aspect, a method for treating tumors, pathogen infections, or enhancing immune tolerance in an individual in need thereof is provided in the present invention, comprising administering a therapeutically effective amount of the T cells according to the first aspect, or administering a therapeutically effective amount of a pharmaceutical composition comprising the T cells according to the first aspect.

In a specific embodiment, the subject is a human.

In a specific embodiment, the tumor includes leukemia (such as acute leukemia, acute lymphocytic leukemia, acute myeloid leukemia, acute myeloid leukemia, acute promyelocytic leukemia, acute myelo-monocytic leukemia, acute monocyte leukemia, acute leukemia, chronic leukemia, chronic myelogenous leukemia, chronic lymphocytic leukemia, polycythemia vera), lymphoma (Hodgkin's disease, non-Hodgkin's disease), primary macroglobulinemia, heavy chain disease, solid tumors such as sarcomas and cancers (such as fibrosarcoma, myxosarcoma, liposarcoma, chondrosarcoma, osteosarcoma, chordoma, endothelial sarcoma, lymphangiosarcoma, angiosarcoma, lymphangiothelioma, synovial tumor, mesothelioma, Ewing's tumor, leiomyosarcoma, rhabdomyosarcoma, colon cancer, pancreatic cancer, breast cancer, ovarian cancer, prostate cancer, squamous cell carcinoma, basal cell carcinoma, adenocarcinoma, sweat adenocarcinoma, sebaceous adenocarcinoma, papillary carcinoma, papillary adenocarcinoma, carcinoma, bronchial carcinoma, myeloid cancer, renal cell carcinoma, liver cancer, nile duct cancer, choriocarcinoma, seminal cell tumor, embryo cancer, nephroblastoma, cervical cancer, uterine cancer, testicular cancer, lung cancer, small cell lung cancer, bladder cancer, epithelial cancer, glue Plasma tumors, astrocytomas, medulloblastomas, craniopharyngiomas, ependymal tumors, pineal tumors, hemangioblastomas, acoustic neuromas, oligodendroglioma, schwannomas, meningiomas, melanoma, neuroblastoma, retinoblastoma), esophageal cancer, gallbladder cancer , kidney cancer, multiple myeloma; preferably, the "tumor" includes but is not limited to: pancreatic cancer, liver cancer, lung cancer, gastric cancer, esophageal cancer, head and neck squamous cell carcinoma, prostate cancer, colon cancer, breast cancer, lymphoma, gallbladder cancer, kidney cancer, leukemia, multiple myeloma, ovarian cancer, cervical cancer, and glioma, and any combination thereof; or
the pathogens include: viruses, bacteria, fungi, protozoa or parasites; preferably, the viruses include: cytomegalovirus, Epstein-Barr virus, human immunodeficiency virus or influenza virus.

In a sixth aspect, a pharmaceutical composition is provided in the present invention, comprising: the T cells according to the first aspect; and a pharmaceutically acceptable carrier or excipient.

In a seventh aspect, a kit is provided in the present invention, comprising:
the T cells according to the first aspect; and
an instruction on how to administer the immune effector cells to an individual.

In a specific embodiment, the immune effector cells include, but are not limited to, T lymphocytes, plasma cells, NK cells, and the like; preferably T lymphocytes.

It should be understood that, within the scope of the present invention, the technical features specifically mentioned above and below (such as in the Examples) can be combined with each other, thereby constituting a new or preferred technical solution which needs not be individually described.

### Description of drawings

Figure 1 is a plasmid map prepared for CAR-T cells;
Figure 2 shows the positive rate of chimeric antigen receptor infection;
Figure 3 shows the results of T7EN1 nuclease assay;
Figure 4 shows the results of sequencing analysis on gene editing sites;
Figure 5 shows the results of expression analysis for the CD3ε chain on the surface of IL12-GPC3-CAR T cells;
Figure 6 shows the cytotoxicity of TCR+ or TCR- IL12-GPC3-CAR T cells;
Figure 7 shows the results of cytokines release from IL12-GPC3-CAR T of TCR + and TCR-;
Figure 8 shows the secretion of IL12 detected by ELISA.

### Modes for carrying out the invention

After extensive and in-depth research, the inventors unexpectedly discovered that the inducive expression of a target gene against a target antigen can be regulated by knocking out endogenous TCR in T lymphocytes in combination with an inducible promoter regulated by a TCR receptor signaling pathway, thereby solving the activation of target gene due to non-target antigen substances, and enhancing its safety while retaining its activity. The present invention has been completed based on the above findings.

### Definitions on terms

Unless specifically defined herein, all of the used technical and scientific terms have the same meaning as commonly understood by ae skilled person in the fields of gene therapy, biochemistry, genetics, and molecular biology.

All methods and materials similar or equivalent to those described herein can be used in the practice of or testing the present invention, wherein suitable methods and materials are described herein. All of publications, patent applications, patents and other references mentioned herein are incorporated by reference in their entirety. In case of conflict, the present specification, including definitions, will control. In addition, the materials, methods, and examples are illustrative only and are not intended to be limiting, unless otherwise specified.

Unless otherwise stated, traditional techniques of cell biology, cell culture, molecular biology, transgenic biology, microbiology, recombinant DNA, and immunology will be adopted in the practice of the present invention, all of which belong to the technical scope of the field. Such techniques are explained in detail in the literature. See, for example, Current Protocols in Molecular Biology (FrederickM.AUSUBEL, 2000, Wileyand sonInc, Library of Congress, USA); Molecular Cloning: A Laboratory Manual, Third Edition, (Sambrooketal, 2001, Cold Spring Harbor, NewYork: Cold Spring Harbor Laboratory Press); Oligonucleotide Synthesis (M.J.Gaited., 1984); Mullis et al. U.S. Pat. No. 4,683,195; Nucleic Acid Hybridization (B. D. Harries & S. J. Higginseds. 1984); Transcription And Translation (B. D. Hames & S. J. Higginseds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the series, Methods In ENZYMOLOGY (J. Abelson 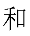 M. Simon, eds.-in-chief, Academic Press, Inc., New York), especially Vols. 154 and 155 (Wuetal. eds.) and Vol.185, "Gene Expression Technology" (D. Goeddel, ed.); Gene Transfer Vectors For Mammalian Cells (J. H. Miller 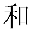 M. P. Caloseds., 1987, Cold Spring Harbor Laboratory); Immunochemical Methods In Cell And Molecular Biology (Mayer and Walker, eds., Academic Press, London, 1987); Hand book Of Experimental Immunology, Vol. I-IV (D. M. Weir and C. C. Blackwell, eds., 1986); 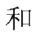 Manipulating the Mouse Embryo (Cold Spring Harbor Laboratory Press, Cold Spring Harbor, N.Y., 1986).

The term "T cell receptor (TCR)" is a characteristic marker on the surface of all T cells and binds to CD3 with non-covalent bonds to form a TCR-CD3 complex. TCR is responsible for identifying an antigen that binds to major histocompatibility complex molecules. TCR is a heterodimer consisting of two different peptide chains, peptide chains α and β. Each peptide chain can be divided into variable region (V region), constant region (C region), transmembrane region and cytoplasmic region, characterized in that the cytoplasmic region is short. The TCR molecule belongs to the immunoglobulin superfamily, and its antigen specificity depends on the V region; each V region (Vα, Vβ) has three hypervariable regions CDR1, CDR2, and CDR3, among which, CDR3 has the largest variation and directly determines the binding specificity to TCR antigen. When TCR recognizes the MHC-antigen-peptide complex, CDR1 and CDR2 recognize and bind to the side walls of the antigen-binding groove of the MHC molecule, while CDR3 directly binds to the antigen peptide. TCR is divided into two categories: TCR1 composed of two chains of γ and δ and TCR2 composed of two chains of α and β. These natural (or manufactured by other means) "anti-cancer" T cells often have weaker recognition capabilities and therefore cannot form a favorable attack on cancer cells. In this case, it is possible to improve the "affinity" and combat effectiveness of these TCRs to the corresponding TAA by means of partial genetic modification, that is, high-affinity TCRs. Therefore, the "genetically modified TCR" technology is also named as the "affinity-enhanced TCR" technology. In the gene modified T cell receptor, the constant region domains of the heavy and light chains of an antibody which also belongs to the immunoglobulin superfamily are used to replace the constant region domains of the B chain and a chain of the TCR, respectively, thereby forming a chimeric TCR molecule (chim-TCR).

The co-receptor of CD3 (differentiation cluster 3) T cells is a protein complex consisting of four different chains. In mammals, the complex contains a CD3y chain, a CD3δ chain, and two CD3ε chains. These chains have a T cell receptor (TCR) and a zeta-chain to generate an activation signal. The TCR, ζ chain and CD3 molecule together constitute a T cell receptor complex. CD3 molecule is connected to T cell receptor (TCR) via salt bridge to form TCR-CD3 complex, which is involved in T cell signal transduction. It is mainly used to label thymocytes, T lymphocytes and T cell lymphomas. The cytoplasmic segment of CD3 contains immunoreceptor tyrosine-based activation motif (ITAM). TCR recognizes and binds to an antigen peptide presented by the major histo-compatibility complex (MHC) molecule, thereby leading to phosphorylation of the tyrosine residues in conserved sequence of CD3 ITAM by T56 tyrosine protein kinase p56lck, and then recruiting other tyrosine protein kinases (such as ZAP-70) containing SH2 (Scr homology 2) domain. ITAM phosphorylation and binding to ZAP-70 is one of the important biochemical reactions in the early stages of T cell activation signaling. Therefore, the function of the CD3 molecule is to transduce the activation signal generated by the recognition of an antigen by TCR. In the present application, the exogenous receptor that can bind to a target antigen and trigger the activation of CD3 signal comprises at least one CD3 binding site and at least one additional antigen binding site specific to bacterial substances, viral proteins, autoimmune markers, or antigens present on certain cells (such as cell surface proteins of B cells, T cells, natural killer (NK) cells, bone marrow cells, phagocytic cells, or tumor cells). Such exogenous receptor can cross-link two cells and can be used to direct T cells to specific targets and trigger the cytotoxic activity of T cells on target cells. Examples of such targets may be tumor cells or infectious agents, such as viral or bacterial pathogens, such as dengue virus, herpes simplex, influenza virus, HIV, or cells carrying an autoimmune target (such as IL-2, an autoimmune marker, or autoimmune antigen).

*In vivo* transduction pathways for T-cell activation signal mainly include PLC-γ activation pathway and Ras-MAP kinase activation pathway. After a series of cascades of signal transduction molecules, transcription factors (NFAT, NF-kb, AP-1, etc.) are eventually activated and enter the nucleus to regulate the transcription of related target genes.

The term "inactivated" or "inactive" means that the gene of interest (such as the TCR gene) is no longer expressed as a functional protein. In a specific embodiment, the method of inactivating a gene of interest may include deleting, frameshifting, or mutating the gene of interest, such as introducing a rare endonuclease that can break the gene of interest into a cell. In a specific embodiment, the cell can be transfected with a nucleic acid encoding a rare endonuclease capable of cleaving the gene of interest, so that the rare endonuclease is expressed in the cell. The rare endonuclease may be a giant nuclease, a zinc finger nuclease, a CRISPR / Cas9 nuclease, an MBBBD-nuclease, or a TALEN-nuclease. In a preferred embodiment, the rare endonuclease is a CRISPR / Cas9 nuclease or a TALEN-nuclease.

The "TCR inactivation" or "TCR inactivity" refers to an endogenous TCR, in which a gene of at least one subunit, especially a TCRα and / or TCRβ gene, and more preferably a TCRα gene is inactivated.

The term "NF-kb (Nuclear factor kB)" is a member of the transcription factor family and is the most important nuclear transcription factor in a cell. It plays a central role in the transcriptional regulation of cellular information stimulus-mediated in cells, participates in the expression and regulation of various genes and is a sign of cell activation. NF-κB generally exists as a homo- or heterodimer. In resting cells, NF-kB dimers are dispersed in the cytoplasm by binding to their inhibitory protein IkB through a non-covalent bond. Many factors, including endoplasmic reticulum stress, can activate NF-kB. Upon activation, NF-kB enters the nucleus, binds to specific proteins on the DNA module, induces the production of specific mRNAs, and finally can transcribe, produce and release various cytokines.

The term "AP-1 (Activator protein 1)" is a transcriptional activator in the cell and is a heterodimer composed of c-Fos and c-Jun. It responds to various stimuli by regulating gene expression, including cytokines, growth factors, stress, bacterial and viral infections; therefore AP-1 controls many cellular processes, including differentiation, proliferation and apoptosis. AP-1 up-regulates the transcription of a gene containing TPA DNA response element (TRE; 5'-TGAG/CTCA-3'). The AP-1 heterodimer is formed by a leucine zipper and initiates the expression of a gene by binding a specific conserved sequence to the gene.

The term "Nuclear factor of activated T cells (NFAT)" plays an important role in the transcriptional regulation of cytokine genes. NFAT protein plays an important role in the transcriptional regulation of varioys cytokines and cell surface receptors that can regulate important immune functions (such as, interleukin-2, interleukin-4, interleukin-5, interleukin-13, interferon-y, tumor necrosis factor-a, GM-CSF, CD40L, and CTLA-4). NFAT proteins that have been discovered so far can be divided into: NFAT1, NFAT2, NFAT3, NFAT4 and NFAT5. Among them, the activation of NFATc1-4 depends on the intracellular calcium signaling pathway.

Activation of NFAT protein is regulated by a process including NFAT protein dephosphorylation, nuclear translocation and DNA binding. In resting cells, phosphorylated NFAT protein resides in the cytoplasm and has a lower DNA-binding affinity. Various stimuli that can trigger calcium movement can cause rapid dephosphorylation of NFAT proteins through a process regulated by Ca2+/calmodulin-dependent protein phosphatase, i.e., calcineurin. A dephosphorylated NFAT protein with an exposed nuclear localization signal is shifted into the nucleus, which binds to DNA with high affinity and regulates the transcription of the target gene.

The term "promoter" as used herein is defined as a DNA sequence recognized by a synthetic mechanism of a cell required for initiating specific transcription of a polynucleotide sequence or an introduced synthetic mechanism.

A typical eukaryotic promoter consists of a minimal promoter and other cis-elements. The minimal promoter is essentially a TATA box region, where RNA polymerase II (polII), TATA binding protein (TBP), and TBP related factor (TAF) can be combined to initiate transcription. Such sequence elements (e.g., enhancers) have been found to increase, often in a position and / or orientation-independent manner, the overall expression level of nearby genes. The construction of a chimeric promoter obtained by combining a minimal promoter with different cis-regulatory elements can be found in, for example, US Patent 6,565,673.

In some embodiments, NFAT plays an important role in the transcriptional expression of cytokines during T cell activation. Based on such consideration, the encoding sequence of the cytokine is placed under the regulation of the minimal promoter containing NFAT-binding motif by the inventors. In addition, in order to further improve the specificity of CAR-T cells expressing cytokines, the endogenous TCRα chain of CAR-GPC3 T cells carrying a cytokine gene, the expression of which is NFAT-regulated, can also be knocked out by gene editing technology to eliminate the expression of cytokines induced by non-tumor target antigens (such as antigens other than GPC3) through TCR/CD3 signaling pathway, therefor only tumor target antigens can specifically induce CAR-GPC3 T cells to express cytokines, such as IL12. Gene editing technology can adopt TALEN technology, CRISPR / Cas9, or ZFN.

In the Example, the IL2 minimal promoter containing 6 NFAT binding motifs is a promoter composed of six NFAT binding sites in tandem with the minimal promoter of IL2 (Hooijberg E, Bakker AQ, Ruizendaal JJ, Spits H. NFAT-controlled expression of GFP permits visualization and isolation of antigen-stimulated primary human Tcells. Blood. 2000 Jul 15; 96(2): 459-66), which can be used to regulate the expression of cytokines such as IL12 in T lymphocytes, such as TCR-T (Zhang L, Kerkar SP, Yu Z, Zheng Z, Yang S, RestifoNP, Rosenberg SA, Morgan RA. Improving adoptive T cell therapy by targeting and controlling IL-12 expression to the tumor environment. Mol Ther. 2011 Apr; 19 (4): 751-9).

The term "Open Reading Frame (ORF)" is the normal nucleotide sequence of a structural gene. The reading frame from the start codon to the stop codon can encode a complete polypeptide chain, and there is no stop codon that can interrupt translation.

The term "knockout" is a technique for integrating a foreign gene into a certain location on the genome of a target cell through homologous recombination to achieve the purpose of modifying a certain gene on a chromosome by site-directed modification. It overcomes the blindness and contingency of random integration, and is an ideal method for modifying and transforming biological genetic materials, however its target shooting efficiency is extremely low. In recent years, nuclease-directed genomic targeted modification technology has developed rapidly. Such nuclease usually consists of a DNA recognition domain and a non-specific endonuclease domain. The DNA recognition domain recognizes the target site, locates the nuclease to the genomic region to be edited, and then the non-specific endonuclease will cleave the DNA double-strand and lead to self-repair mechanism of DNA breakage, thereby triggering mutations in the gene sequence and promoting the occurrence of homologous recombination. The latest developped technology includes CRISPR/Cas9 technology, ZFN technology, TALE technology and TALE-CRISPR/Cas9 technology.

The term "gene silencing" refers to a phenomenon in which a gene is not expressed or underexpressed without damaging the original DNA due to various reasons. Gene silencing occurs at two levels, one is gene silencing at the transcription level due to DNA methylation, heterochromatinization, and position effects, and the other is post-transcriptional gene silencing, that is, inactivating genes by specifically suppressing target RNAs at the post-transcriptional level, including antisense RNA, co-suppression, gene suppression, RNA interference, and microRNA-mediated translation suppression, etc.

The term "artificial Zinc Finger Nucleases (ZFN)" technology is the first generation of nuclease site-directed modification technology, which can specifically recognize the zinc finger motif of the triplet DNA fragment, instead of bases as a basic unit for the specific identification of DNA sequence. The most classic zinc finger nuclease is a fusion of a non-specific endonuclease FokI with a zinc finger-containing domain, the purpose of which is to cleave specific sequences. The single-stranded part of the cleaved DNA can be deleted by the repair mechanism, and then reconnected together. ZFN technology has been used as the main method to induce genomic mutation and improve the efficiency of homologous recombination. However, the binding to the target DNA sequence is unstable, it is impossible to locate all target sequences, a large amount of screening and identification works are required, and it is prone to off-target mutations, since ZFN is affected by the upstream and downstream-dependent effects. In addition, the complete set of commercial ZFNs is expensive, tedious in design, and unstable in effect.

The term "transcription activator-like effector (TALE)" exhibits the specificity of DNA binding, and the modular operation of the identification code is simple and convenient. The TALE-DNA binding domain consists of tandem repeat units, most of which contain 34 amino acids. The amino acids 12 and 13 of the unit are designed as repeat variable residues (RVD). RVD of TALE recognizes 4 bases on a DNA sequence with highly specificity, and the 13^{th} amino acid directly binds specifically to the bases of DNA. Based on a DNA sequence, specific TALEDN recognition binding domains can be constructed at any site, which can be widely used for gene sequence mutation modification and gene targeting. TALE nucleases (tanscription activator-like effector nucleases, TALENs) were assembled by setting DNA target sequences, assembling TALE-DNA binding domains and non-specific DNA cleavage domains with Fok I endonucleases being fused. TALENs target DNA and produce DNA double-srand breaks (DSBs). DSBs activate two conserved DNA repair pathways in eukaryotic cells. Broken chromosomes can be reconnected through non-homologous end joining (NHEJ). During the joining process, the loss or insertion of small fragments of bases may be introfuced at the break site, thereby affecting gene function or generating gene knockout effects. If a homologous recombination vector is introduced at this time, homology-directed repair (HDR) can guide repair by using similar DNA templates, and replace the DNA sequence around the break site, thereby achieving specific mutations or site-directed introduction of foreign DNA.

CRISPER / Cas9 is the third-generation gene editing technology, which has obvious advantages over ZEN and TALEN technologies. Its construction is simple and convenient, and the gene editing efficiency is high and the cost is low. In general, "CRISPR systems" are collectively referred to as transcripts and other elements involved in the expression of the CRISPR-related ("Cas") genes or directing the activities thereof, including a sequences encoding Cas gene, tracr (transactivating CRISPR) sequence (e.g., tracrRNA or active part of tracrRNA), tracr pairing sequences (covering "repeats in the same direction" and parts of repeats in the same direction of tracrRNA processing in the context of an endogenous CRISPR systems), guide sequences (also named as "spacers" in the context of endogenous CRISPR systems), or other sequences and transcripts from a CRISPR locus. In general, CRISPR systems are characterized by elements that facilitate the formation of a CRISPR complex (also named as anterior compartment in the context of endogenous CRISPR systems) at the site of a target sequence. In the context of the formation of CRISPR complex, a "target sequence" refers to a sequence that is designed to be complementary to the guide sequence, where hybridization between the target sequence and the guide sequence promotes the formation of a CRISPR complex. Complete complementarity is not necessary, provided that there is sufficient complementarity to initiate hybridization and promote the formation of a CRISPR complex. A target sequence may comprise any polynucleotide, such as a DNA or RNA polynucleotide. In some embodiments, the target sequence is located in the nucleus or cytoplasm of a cell.

In general, a guide sequence is any polynucleotide sequence that is sufficiently complementary to a target polynucleotide sequence as to hybridize to the target sequence and direct the CRISPR complex to specifically bind to the target sequence. In some embodiments, when optimal alignment is performed using a suitable alignment algorithm, the degree of complementarity between the guide sequence and its corresponding target sequence is about or higher than about 50%, 60%, 75%, 80% , 85%, 90%, 95%, 97.5%, 99%, or higher. Any suitable algorithm for aligning sequences can be used to determine the optimal alignment, non-limiting examples of which include the Smith-Waterman algorithm, the Needleman-Wunsch algorithm, Algorithms based on Burrows-Wheeler Transform (e.g. Burrows Wheeler Aligner), ClustalW, Clustal X, BLAT, Novoalign (Novocraft Technologies), ELAND Corporation (Illumina), San Diego, California), SOAP (available at soap.genomics.org.cn), and Maq (available at maq.sourceforge.net).

In general, a tracr pairing sequence includes any sequence that is sufficiently complementary to the tracr sequence to facilitate one or more of the following: (1) the removal of a guide sequence flanked by the tracr pairing sequence in a cell containing the corresponding tracr sequence; and (2) the formation of a CRISPR complex at the target sequence, wherein the CRISPR complex comprises a tracr pairing sequence that hybridizes to the tracr sequence. Generally, the degree of complementarity is in terms of the best alignment between the tracr pairing sequence and tracr sequence along the length of the shorter of the two sequences. The optimal alignment can be determined by any suitable alignment algorithm, and the secondary structure can be further explained, such as self-complementarity within the tracr sequence or tracr pairing sequence. In some embodiments, when performing an optimal alignment, the degree of complementarity between the tracr sequence and the tracr pairing sequence along the length of the shorter of the two is about or higher than about 25%, 30%, 40%, 50%, 60%, 70%, 80%, 90%, 95%, 97.5%, 99%, or higher.

In some embodiments, the CRISPR enzyme is a part of a fusion protein comprising one or more heterologous protein domains (e.g., about or more than about 1, 2, 3, 4, 5, 6, 7, 8, 9, 10 or more domains). A CRISPR enzyme fusion protein may comprise any other protein, and optionally a linker sequence between any two domains. Examples of protein domains that can be fused to CRISPR enzymes include, but are not limited to, epitope tags, reporter gene sequences, and protein domains having one or more of the following activities: methylase activity, demethylase activity, transcription activation activity, transcription repression activity, transcription release factor activity, histone modification activity, RNA cleavage activity and nucleic acid binding activity. Non-limiting examples of epitope tags include histidine (His) tag, V5 tag, FLAG tag, influenza virus hemagglutinin (HA) tag, Myc tag, VSV-G tag, and thioredoxin (Trx) tag. Examples of reporter genes include, but are not limited to, glutathione-S-transferase (GST), horseradish peroxidase (HRP), chloramphenicol acetyltransferase (CAT), β-galactosidase, β-glucuronidase, luciferase, green fluorescent protein (GFP), HcRed, DsRed, green fluorescent protein (CFP), yellow fluorescent protein (YFP), spontaneous fluorescent protein including blue fluorescent protein (BFP). CRISPR enzymes can be fused to a gene sequence encoding a protein or protein fragment that binds to a DNA molecule or to other cellular molecules, including, but not limited to, maltose binding protein (MBP), S-tag, Lex A DNA binding domain (DBD) fusion, GAL4 DNA binding domain fusion, and herpes simplex virus (HSV) BP16 protein fusion. Additional domains that can form part of a fusion protein comprising a CRISPR enzyme are described in US 20110059502, which is incorporated herein by reference. In one embodiment, exons of corresponding encoding genes in the constant regions of one or both of the α and β chains of the TCR are knocked out using CRISPER / Cas technology, so that the endogenous TCR is inactive. Preferably, the first exon of the constant region of the α chain of the endogenous TCR is site-directed knocked out.

The term "down-regulation" refers to the reduction or elimination of gene expression of one or more genes.

As used herein, the term "modification" refers to a change in the state or structure of a protein or polypeptide of the invention. Modifications can be chemical, structural, and functional modifications.

The term "T CELL FUSION PROTEIN (TFP)" includes various polypeptide-derived recombinant polypeptides constituting TCR, which can bind to surface antigens on target cells, interact with other polypeptides of an intact TCR complex, and be usually co-located on the surface of T cells. TFP consists of an antigen-binding domain consisting of a TCR subunit and a human or humanized antibody domain, wherein the TCR subunit includes at least part of a TCR extracellular domain, a transmembrane domain, and a TCR intracellular signal domain; and the TCR subunit is operatively connected to the antibody domain, wherein the extracellular, transmembrane, and intracellular signal domains of the TCR subunit are derived from CD3 ε or CD3y, and the TFP is integrated into TCR expressed on T cells.

The term "T CELL ANTIGEN COUPLER (TAC)" includes three functional domains: a tumor-targeting domain, including single-chain antibody, designed ankyrin repeat protein (DARPin), or other targeting groups; an extracellular domain, single-chain antibody that binds to CD3, so that the TAC receptor is close to other TCR receptors; the transmembrane region and intracellular region of the CD4 co-receptor, wherein the intracellular region is linked to the protein kinase LCK, which catalyzes the phosphorylation of immune receptor tyrosine activation motifs (ITAMs) of the TCR complex as an initial step in T cell activation.

The term "tumor antigen" refers to a molecule (usually a protein, carbohydrate, or lipid) that is expressed on the surface of a tumor cell in whole or in a form of a fragment (e.g., MHC / peptide) and is used to target a pharmacological agent preferentially to tumor cells. Tumor antigens can be markers expressed by normal cells and cancer cells, such as lineage markers, such as CD19 on B cells; tumor antigens can also be cell surface molecules that are overexpressed in cancer cells compared with normal cells, such as 1-fold over-expressed, 2-fold over-expressed, 3-fold or more over-expressed compared with normal cells; tumor antigens can also be cell surface molecules inappropriately synthesized in cancer cells, for example, molecules containing deletions, additions or mutations as compared with molecules expressed on normal cells; tumor antigens can also be specifically expressed on the cell surface of cancer cells in whole or in a form of fragments (e.g., MHC / peptides), rather than synthesized or expressed on the surface of normal cells. In certain embodiments, a CAR of the invention includes a CAR comprising an antigen binding domain (e.g., an antibody or antibody fragment) that binds to an MHC presenting peptide. Generally, endogenous protein-derived peptides fill the pockets of Class I molecules of the major histocompatibility complex (MHC) and are recognized by T cell receptors (TCR) on CD8+ T lymphocytes. The MHC class I complex is constitutively expressed by nucleated cells. In tumors, virus-specific and / or tumor-specific peptide / MHC complexes represent a unique type of cell surface target for immunotherapy.

The term "pathogen" refers to a protozoan capable of causing a disease and includes: viruses, bacteria, fungi or parasites. The "virus antigen" refers to a polypeptide expressed by a virus and capable of inducing an immune response.

In the context of the present invention, "tumor antigen" or "hyperproliferative disorder antigen" or "antigen associated with a hyperproliferative disorder" refers to an antigen common to a particular hyperproliferative disorder. In certain aspects, the hyperproliferative disorder antigen of the invention is derived from cancer, including but not limited to primary or metastatic melanoma, thymoma, lymphoma, sarcoma, lung cancer, liver cancer, non-Hodgkin's lymphoma, Hodgkin's lymphoma, leukemia, uterine cancer, cervical cancer, bladder cancer, kidney cancer and adenocarcinoma such as breast cancer, prostate cancer, ovarian cancer, pancreatic cancer, etc.

As used herein, the terms "activation" and "activating" are used interchangeably, and they and their grammatically other forms can refer to a process by which a cell changes from a resting state to an active state. The process may include a response to a phenotypic or genetic change in the state of antigen, migration, and / or functional activity. For example, the term "activation" may refer to a process in which T cells are gradually activated. For example, T cells may require at least two signals to be completely activated. The first signal may occur after the TCR is engaged with the antigen-MHC complex, and the second signal may occur through the engagement of a co-stimulatory molecule (the co-stimulatory molecules listed in Table 1). Anti-CD3 can mimic the first signal and anti-CD28 can mimic the second signal, *in vitro.* For example, engineered T cells can be activated by an expressed CAR. T cell activation or T cell triggering as used herein may refer to a state of T cells that have been sufficiently stimulated to induce detectable cell proliferation, cytokine production, and / or detectable effector function.

The term "receptor" as used herein is a type of special protein that exists in the cell membrane or inside the cell and can bind to a specific signal molecule outside the cell to activate a series of biochemical reactions in the cell, so that the cell has a corresponding effect on external stimuli. The biologically active substances that bind to the receptor are collectively named as ligands. Receptor-ligand binding causes changes in molecular conformation, so that cause cellular responses, such as mediating intercellular signal transduction, intercellular adhesion, and endocytosis, and the like.

The term "co-stimulatory ligand" as used herein includes a molecule on an antigen-presenting cell (e.g., aAPC, dendritic cell, B cell, etc.) that specifically binds to an identical costimulatory molecule on a T cell, thereby providing a signal, and mediating a T cell response together with the first signal provided by, for example, the TCR / CD3 complex in combination with a peptide-loaded MHC molecule, including but not limited to proliferation, activation, differentiation, and the like. Co-stimulatory ligands can include, but are not limited to, CD7, B7-1 (CD80), B7-2 (CD86), PD-L, PD-L2, 4-1BBL, OX40L, inducible costimulatory ligand (ICOS-L), Intercellular adhesion molecule (ICAM), CD30L, CD40, CD70, CD83, HLA-G, MICA, MICB, HVEM, lymphotoxin beta receptor, 3 / TR6, ILT3, ILT4, HVEM, an agonist or antibody binding Toll ligand receptor and a ligand that specifically binds to B7-H3. Co-stimulatory ligands also specifically include antibodies that specifically bind to co-stimulatory molecules present on T cells, for example, but not limited to, CD27, CD28, 4-1BB, OX40, CD30, CD40, PD-1, ICOS, lymphocyte function-related antigen-1 (LFA-1), CD2, CD7, LIGHT, NKG2C, B7-H3, and ligands that specifically bind to CD83.

The term "co-stimulatory molecule" as used herein refers to an identity-binding partner on a T cell that specifically binds to a co-stimulatory ligand, thereby mediating the co-stimulatory response of a T cell, such as, but not limited to, proliferation. Co-stimulatory molecules include, but are not limited to, MHC class I molecules, BTLA, and Toll ligand receptors.

As used herein, a "co-stimulatory signal" refers to a signal that, in combination with a cell-stimulating signal molecule, such as TCR / CD3, results in T cell proliferation and / or up- or down-regulation of key molecules.

The term "chimeric antigen receptor" or "CAR" as used herein refers to an engineered molecule that can be expressed by immune cells, including but not limited to T cells. CAR is expressed in T cells and can redirect T cells to induce specifically killing of target cells determined by artificial receptors. The extracellular binding domain of CAR can be derived from a murine, humanized or fully human monoclonal antibody. When it is in an immune effector cell, the cell is given specificity for a target cell (usually a cancer cell) and intracellular signal is produced. A CAR typically includes at least one extracellular antigen-binding domain, a transmembrane domain, and a cytoplasmic signaling domain (also referred to herein as an "intracellular signaling domain"), including functional signaling domain derived from stimulatory molecules and / or costimulatory molecule as defined below. In certain aspects, the polypeptide groups are adjacent to each other. A polypeptide group includes a dimerization switch that can couple polypeptides to each other in the presence of a dimerization molecule. For example, an antigen binding domain can be coupled to an intracellular signaling domain. In one aspect, the stimulatory molecule is a ζ chain that binds to a T cell receptor complex. In one aspect, the cytoplasmic signaling domain further comprises one or more functional signaling domains derived from at least one costimulatory molecule as defined below. In one aspect, the costimulatory molecule is selected from the costimulatory molecules described herein, such as 4-1BB (i.e., CD137), CD27, and / or CD28. In one aspect, the CAR includes a chimeric fusion protein comprising an extracellular antigen-binding domain, a transmembrane domain, and an intracellular signaling domain comprising a functional signaling domain derived from a stimulatory molecule. In one aspect, the CAR comprises a chimeric fusion protein comprising an extracellular antigen-binding domain, a transmembrane domain, a functional signaling domain derived from a co-stimulatory molecule and an intracellular signaling domain of a functionality signaling domain derived from a stimulatory molecule. In one aspect, the CAR comprises a chimeric fusion protein comprising an extracellular antigen-binding domain, a transmembrane domain, and two functional signaling domains derived from one or more costimulatory molecules.

The term, "signaling domain" refers to a functional moiety of a protein that functions by transmitting information within a cell and is used to generate a second messenger or to respond to such a messenger to act as an effector, thereby regulating cell viability via a defined signal transduction pathway.

As used herein, the term "engineered" and other grammatical forms thereof may refer to one or more alterations of a nucleic acid, such as a nucleic acid within the organism's genome. The term "engineered" may refer to alterations, additions and / or deletions of genes. Engineered cells can also refer to cells with genes added, deleted, and / or altered.

As used herein, the term "cell" or "engineered cell" and other grammatical forms thereof may refer to a cell of human or non-human animal origin. Engineered cells can also refer to cells that express CAR.

The term "transfection" as used herein refers to the introduction of exogenous nucleic acid into a eukaryotic cell. Transfection can be achieved by various means known in the art, including calcium phosphate-DNA co-precipitation, DEAE-dextran-mediated transfection, polybrene-mediated transfection, electroporation, microinjection, liposome fusion, lipid transfection, protoplast fusion, retroviral infection and biolistics.

The term "stable transfection" or "stably transfect" refers to the introduction and integration of exogenous nucleic acid, DNA or RNA into the genome of a transfected cell. The term "stable transfectant" refers to a cell, in which a foreign DNA is stably integrated into genomic DNA.

As used herein, the terms "nucleic acid molecule encode", "encoding DNA sequence" and "encoding DNA" refer to the order or sequence of deoxyribonucleotides along a deoxyribonucleotide strand. The order of these deoxyribonucleotides determines the order of amino acids along a polypeptide (protein) chain. Therefore, a nucleic acid sequence encodes an amino acid sequence.

The term "individual" as used herein refers to any animal, such as a mammal or marsupial. Individuals of the invention include, but are not limited to, humans, non-human primates (such as rhesus monkeys or other types of macaques), mice, pigs, horses, donkeys, cattle, sheep, rats, and any kind of poultry.

As used herein, the term "peripheral blood lymphocyte" (PBL) and other grammatical forms thereof may refer to lymphocytes that circulate in the blood (e.g., peripheral blood). Peripheral blood lymphocytes can refer to (but not limited to) lymphocytes of organs. Peripheral blood lymphocytes may comprise T cells, NK cells, B cells, or any combination thereof.

As used herein, the term "peripheral blood mononuclear cell" (PBMC) refers to cells with a single nucleus in peripheral blood, including lymphocytes, monocytes, and the like.

As used herein, the term "T cell" and other grammatical forms thereof can refer to T cells of any origin. For example, the T cells may be primary T cells, such as autologous T cells and the like. T cells can also be of human or non-human.

The term "T cell activation" or "activation of T cell" and other grammatical forms thereof as used herein may refer to a T cell in a status where the T cell is sufficiently stimulated to induce detectable cell proliferation, cytokine production, and / or detectable effector function. In some cases, "full T cell activation" may be similar to a status where cytotoxicity of T cells is triggered. Various assays known in the art can be used to measure T cell activation. The assay may be ELISA, ELISPOT for measuring cytokine secretion, flow cytometry assay for measuring intracellular cytokine expression (CD107), flow cytometry assay for measuring proliferation, and cytotoxicity assay for determining target cell elimination (51Cr release assay). The assays typically compared controls (non-engineered cells) with engineered cells (CAR T) to determine the relative activation of engineered cells as compared with controls. In addition, the assay can be performed to compare with engineered cells that are incubated with or contact target cells which do not express the target antigen. For example, the comparison may be a comparison with GPC3-CART cells incubated with target cells which do not express GPC3.

The term "sequence" and other grammatical forms thereof used herein, when used in reference to a nucleotide sequence, may include DNA or RNA, and may be single-stranded or double-stranded. Nucleic acid sequences can be mutated. The nucleic acid sequence can be of any length.

The term "effective amount" as used herein refers to an amount that provides a therapeutic or prophylactic benefit.

The term "expression vector" as used herein refers to a vector comprising a recombinant polynucleotide, which comprises an expression control sequence operably linked to a nucleotide sequence to be expressed. An expression vector comprises sufficient cis-acting elements for expression; and other elements for expression can be provided by a host cell or an *in vitro* expression system. Expression vectors include all those known in the art, such as cosmids, plasmids (e.g., naked or contained in liposomes), and viruses (e.g., lentivirus, retrovirus, adenovirus, and adeno-associated virus).

The term "lentivirus" as used herein refers to a genus of the retroviridae family. Retroviruses are unique in infecting non-dividing cells; they can deliver large amounts of genetic information into the DNA of a host cell, therefore, they are one of the most effective methods for gene delivery. HIV, SIV and FIV are examples of lentiviruses. Lentivirus-derived vectors provide a means to achieve significant levels of gene transfer *in vivo.*

The term "operably linked" as used herein refers to a functional linkage between a regulatory sequence and other nucleic acid sequences, and such linkage results in the expression of the latter. For example, when a first nucleic acid sequence is functionally related to a second nucleic acid sequence, the first nucleic acid sequence is operably linked to the second nucleic acid sequence. For example, if a promoter affects the transcription or expression of an encoding sequence, the promoter is operably linked to the encoding sequence. Generally, the operably linked DNA sequences are continuous and, if necessary, two protein encoding regions are linked in the same reading frame.

The term "vector" as used herein is a composition which comprises an isolated nucleic acid and can be used to deliver the isolated nucleic acid inside a cell. Many vectors are known in the art, including, but not limited to linear polynucleotides, polynucleotides associated with ionic or amphiphilic compounds, plasmids, and viruses. Therefore, the term "vector" includes autonomously replicated plasmids or viruses. The term should also be interpreted to include non-plasmid and non-viral compounds, such as polylysine compounds, liposomes and the like, which can facilitate the transfer of nucleic acids into cells. Examples of viral vectors include, but not limited to, adenovirus vectors, adeno-associated virus vectors, retroviral vectors, and the like.

The term sequence "identity" as used herein means that the identity percentage is determined by comparing two best-matched sequences over a comparison window (e.g., at least 20 positions), where a portion of a polynucleotide or polypeptide sequence in the comparison window may contain additions or deletions (i.e., gaps), for example, 20% or less gaps (e.g., 5 to 15%, or 10 to 12%) compared with the reference sequence (which does not contain additions or deletions) for the two best-matched sequences. The percentage is usually calculated by determining the number of positions where the same nucleic acid base or amino acid residue occurs in both sequences, so as to produce the number of correctly matched positions, and the number of correctly matched positions is divided by the total number of positions in the reference sequence (i.e., Window size) and the obtained result is multiplied by 100 to produce a percentage of sequence identity.

As used herein, the terms "IL12" and "interleukin-12" are used interchangeably and have the same meaning. As used herein, the term "IL12" is an immunomodulatory factor with multiple biological activities. For example, the term "IL12" may refer to human IL12 as defined in SEQ ID NO: 26 or an active fragment thereof, or it may refer to murine IL12 as defined in SEQ ID NO: 27 or an active fragment thereof, alternatively, it may also be of another species.

In some embodiments, the element used to construct the receptor that specifically binds to GPC3 or IL12 may be naturally occurring. For example, it may be isolated or purified from mammals; or may also be artificially prepared. For example, recombinant elements or IL12 can be produced according to conventional genetic engineering recombination techniques. Preferably, the present invention can use the recombined elements or IL12.

An amino acid sequence formed by substitution, deletion, or addition of one or more amino acid residues based on each element or IL12 polypeptide sequence is also included in the present invention. Appropriate substitution of amino acids is a technique well known in the art that can be readily implemented and ensures that the biological activity of the resulting molecule is not altered. Based on these techniques, a skilled person will appreciate that, in general, changing a single amino acid in a non-essential region of a polypeptide will not substantially alter biological activity thereof.

The biologically active fragments of each element or the IL12polypeptide can be applied in the present invention. Herein, the meaning of the biologically active fragment means that a polypeptide, as a part of a full-length polypeptide, can still maintain all or part of the function of the full-length polypeptide. Generally, the biologically active fragment retains at least 50% of the activity of the full-length polypeptide. Generally, the biologically active fragment is capable of retaining 60%, 70%, 80%, 90%, 95%, 99%, or 100% of the activity of the full-length polypeptide.

Based on the elements or the IL12 polypeptide sequence, modified or improved polypeptides can also be applied in the present invention. For example, for promoting the half-life, effectiveness, metabolism, and / or the effectiveness of a polypeptide, a modified or improvded polypeptide can be used. That is, any variation that does not affect the biological activity of a polypeptide can be applied in the present invention.

The term, "tumor" refers to a disease characterized by the pathological proliferation of cells or tissues, and subsequent migration or invasion into other tissues or organs. The growth of a tumor is usually uncontrolled and progressive, and does not induce or inhibit normal cell proliferation. Tumors can affect various cells, tissues or organs, including, but not limited to, bladder, bone, brain, breast, cartilage, glial cells, esophagus, fallopian tube, gallbladder, heart, intestine, kidney, liver, lung, lymph nodes, nerve tissue, ovary, pancreas, prostate, skeletal muscle, skin, spinal cord, spleen, stomach, testis, thymus, thyroid, trachea, urethra, ureter, urethra, uterus, vaginal organ, or tissue or corresponding cell. Tumors include cancers, such as sarcomas, carcinomas, or plasmacytomas (malignant tumors of plasma cells). The tumor according to the present invention may include, but is not limited to, leukemia (such as acute leukemia, acute lymphocytic leukemia, acute myeloid leukemia, acute myeloid leukemia, acute promyelocytic leukemia, acute myelo-monocytic leukemia, acute monocytic leukemia, acute leukemia, chronic leukemia, chronic myelogenous leukemia, chronic lymphocytic leukemia, polycythemia vera), lymphoma (Hodgkin's disease, non-Hodgkin's disease), primary macroglobulinemia, heavy chain disease, solid tumors such as sarcomas and cancers (such as fibrosarcoma, myxosarcoma, liposarcoma, chondrosarcoma, osteosarcoma, chordoma, endothelial sarcoma, lymphangiosarcoma, angiosarcoma, lymphangiothelioma, synovial tumor, mesothelioma, Ewing's tumor, leiomyosarcoma, rhabdomyosarcoma, colon cancer, pancreatic cancer, breast cancer, ovarian cancer, prostate cancer, squamous cell carcinoma, basal cell carcinoma, adenocarcinoma, sweat adenocarcinoma, sebaceous adenocarcinoma, papillary carcinoma, papillary adenocarcinoma, carcinoma, bronchial carcinoma, myeloid cancer, renal cell carcinoma, liver cancer, nile duct cancer, choriocarcinoma, seminal cell tumor, embryo cancer, nephroblastoma, cervical cancer, uterine cancer, testicular cancer, lung cancer, small cell lung cancer, bladder cancer, epithelial cancer, glue Plasma tumors, astrocytomas, medulloblastomas, craniopharyngiomas, ependymal tumors, pineal tumors, hemangioblastomas, acoustic neuromas, oligodendroglioma, schwannomas, meningiomas, melanoma, neuroblastoma, retinoblastoma), esophageal cancer, gallbladder cancer , kidney cancer, multiple myeloma. Preferably, the "tumor" includes, but not limited to, pancreatic cancer, liver cancer, lung cancer, gastric cancer, esophageal cancer, head and neck squamous cell carcinoma, prostate cancer, colon cancer, breast cancer, lymphoma, gallbladder cancer, kidney cancer, leukemia, multiple myeloma, ovarian cancer, cervical cancer and glioma.

Preferably, the "tumor" includes, but not limited to, liver cancer, gastric cancer, lung cancer, and breast cancer.

As used herein, the term "enhancing T cell function" includes inducing, causing, or stimulating T cells to have a sustained or enhanced biological function, or to renew or reactivate exhausted or inactive T cells. Examples of enhancing T cell functions include increased interferon secretion, increased proliferation, and increased antigen reactivity of CD8 + T cells (e.g., virus or pathogen clearance), compared with pre-intervention levels. In one embodiment, the enhancement level is at least 50%, or 60%, 70%, 80%, 90%, 100%, 120%, 150%, 200%. The means for measuring such enhancement is known to a skilled person in the art.

As used herein, the term "exogenous" refers to a nucleic acid molecule or polypeptide that are not endogenously expressed in a cell, or the expression level of which is insufficient to achieve the function achieved when the nucleic acid molecule or polypeptide is overexpressed. Therefore, "exogenous" includes recombinant nucleic acid molecules or polypeptides expressed within a cell, such as an exogenous, heterologous, and overexpressed nucleic acid molecule and polypeptide.

In some embodiments, the antigen-binding receptor of the invention is a chimeric antigen receptor. As used herein, the term "Chimeric Antigen Receptor (CAR)" refers to a tumor antigen binding domain fused to an intracelluar signal transduction domain within a cell and capable of activating T cells. The extracellular binding domain of CAR is generally derived from a mouse or humanized or human monoclonal antibody.

Chimeric antigen receptors typically comprise an extracellular antigen-binding region. In some embodiments, the extracellular antigen binding region can be of fully human. In other cases, the extracellular antigen binding region can be humanized. In other cases, the extracellular antigen binding region may be of mouse origin, or the chimera in the extracellular antigen binding region consists of amino acid sequences derived from at least two different animals. In some embodiments, the extracellular antigen binding region may be of non-human.

A variety of antigen-binding regions can be designed. Non-limiting examples include a single chain variable fragment (scFv) derived from an antibody, a fragment antigen binding region (Fab) selected from a library, a single domain fragment, or a natural ligand associated with its cognate receptor. In some embodiments, the extracellular antigen binding region may comprise scFv, Fab or a natural ligand, as well as any derivatives thereof. The extracellular antigen-binding region may refer to a molecule other than an intact antibody, which may comprise a portion of an intact antibody and may bind to an antigen to which the intact antibody binds. Examples of antibody fragments may include, but not limited to, Fv, Fab, Fab', Fab'-SH, F(ab')2; bifunctional antibodies, linear antibodies; single chain antibody molecules (e.g., scFv); and multispecific antibodies formed from antibody fragments.

The extracellular antigen-binding regions, such as scFv, Fab, or natural ligands, can be a part of a CAR that determines antigen specificity. The extracellular antigen-binding region can bind to any complementary target. The extracellular antigen-binding region may be derived from an antibody with known variable region sequences. The extracellular antigen-binding regions can be obtained from antibody sequences obtained from available mouse hybridomas. Alternatively, the extracellular antigen-binding regions can be obtained from total exo-cleavage sequencing of tumor cells or primary cells, such as tumor infiltrating lymphocytes (TIL).

In some cases, the binding specificity of an extracellular antigen-binding region can be determined by a complementarity determining region or CDR, such as a light chain CDR or a heavy chain CDR. In many cases, binding specificity can be determined by light chain CDRs and heavy chain CDRs. A given combination of given heavy chain CDRs and light chain CDRs can provide a given binding pocket compared with other reference antigens, which can confer greater affinity and / or specificity to an antigen (e.g., GPC3). For example, CDRs specific to phosphatidylinosin-3 can be expressed in the extracellular binding region of CAR, so that a CAR targeting GPC3 can target T cells to tumor cells expressing GPC3.

In certain aspects of any of the embodiments disclosed herein, an extracellular antigen binding region, such as scFv, can comprise a light chain CDR specific for an antigen. The light chain CDR may be the complementarity determining region of scFv light chain of an antigen binding unit such as CAR. A light chain CDR may comprise a sequence of consecutive amino acid residues, or two or more sequences of consecutive amino acid residues separated by non-complementarity determining regions (e.g., framework regions). In some cases, a light chain CDR may comprise two or more light chain CDRs, which may be referred to as light chain CDR-1, CDR-2, and the like. In some cases, a light chain CDR may comprise three light chain CDRs, which may be referred to as light chain CDR-1, light chain CDR-2, and light chain CDR-3, respectively. In some examples, a group of CDRs present on a same light chain may be collectively referred to as a light chain CDR.

In certain aspects of any of the embodiments disclosed herein, an extracellular antigen binding region, such as a scFv, can comprise an antigen-specific heavy chain CDR. The heavy chain CDR may be a heavy chain complementarity determining region of an antigen binding unit, such as scFv. The heavy chain CDR may comprise a continuous sequence of amino acid residues, or a continuous sequence of two or more amino acid residues separated by a non-complementarity determining region (e.g., a framework region). In some cases, a heavy chain CDR may comprise two or more heavy chain CDRs, which may be referred to as heavy chain CDR-1, CDR-2, and the like. In some cases, a heavy chain CDR may comprise three heavy chain CDRs, which may be referred to as heavy chain CDR-1, heavy chain CDR-2, and heavy chain CDR-3, respectively. In some cases, a group of CDRs present on a same heavy chain may be collectively referred to as a heavy chain CDR.

By using genetic engineering, extracellular antigen-binding regions can be modified in various ways. In some cases, the extracellular antigen-binding region can be mutated so that the extracellular antigen-binding region with a higher affinity for its target can be selected. In some cases, the affinity of an extracellular antigen-binding region for its target can be optimized for a target that can be expressed at low levels on normal tissue. This optimization can be performed to minimize potential toxicity. In other cases, the clone of an extracellular antigen-binding region with a higher affinity for the membrane-bound form of a target may be superior to its soluble counterpart. This modification can be made since different levels of soluble forms of the target can also be detected and targeting to them can cause undesired toxicity.

In some cases, the extracellular antigen-binding region includes a hinge or a spacer region. The terms hinge and spacer region are used interchangeably. The hinge can be considered as a part of a CAR that provides flexibility to extracellular antigen binding regions. In some cases, hinges can be used to detect CAR on the cell surface, especially when antibodies that detect extracellular antigen-binding regions are ineffective or not available. For example, it may be necessary to optimize the length of the hinge derived from an immunoglobulin, depending on where the extracellular antigen-binding region targets the epitope on the target.

In some cases, the hinge may not belong to the immunoglobulin, but belong to another molecule, such as the natural hinge of CD8α molecule. The CD8α hinge may contain cysteine and proline residues known to play a role in the interaction of CD8 co-receptors and MHC molecules. The cysteine and proline residues can affect the performance of the CAR.

The CAR hinge can be adjustable in size. The morphology of the immune synapse between T cells and target cells also defines the distance that cannot be functionally bridged by CAR due to the distal membrane epitope on the cell surface of the target molecule. The synaptic distance cannot reach the approximate value of signal transmission even using short hinge CAR. Similarly, signal output of CAR target antigen epitope proximal to membrane can be observed only in the context of a long hinge CAR. The hinge can be adjusted according to the used extracellular antigen binding region. The hinge can be of any length.

A transmembrane domain can anchor CAR to the plasma membrane of a cell. The natural transmembrane portion of CD28 can be used for CAR. In other cases, the natural transmembrane portion of CD8α can also be used in CAR. "CD8" may be a protein that has at least 85, 90, 95, 96, 97, 98, 99, or 100% identity to the NCBI reference number: NP_001759 or a fragment thereof having stimulating activity. "CD8 nucleic acid molecule" may be a polynucleotide encoding a CD8 polypeptide. In some cases, the transmembrane region may be a natural transmembrane portion of CD28. "CD28" may refer to a protein that has at least 85, 90, 95, 96, 97, 98, 99, or 100% identity to the NCBI reference number: NP_006130 or a fragment thereof having stimulating activity. A "CD28 nucleic acid molecule" may be a polynucleotide encoding a CD28 polypeptide. In some cases, the transmembrane portion may contain a CD8α region.

The intracellular signal domain of a CAR may be responsible for activating at least one of the effector functions of the T cell in which the CAR has been placed. CAR can induce effector functions of T cells, for example, cytolytic activity or auxiliary activity, including secretion of cytokines. Therefore, the term "intracellular signal domain" refers to a portion of a protein that transduces effector function signals and directs cells to perform specific functions. The entire intracellular signaling region can generally be used, however, in many cases, it is not necessary to use the entire chain of a signal domain. In some cases, a truncated portion of the intracellular signaling region is used. In some cases, the term intracellular signal domain is therefore intended to include any truncated portion of the intracellular signal domain sufficient to transduce effector functional signals.

Preferred examples of the signal domain used in CAR may include the cytoplasmic sequence of T cell receptors (TCRs) and co-receptors that act synergistically to initiate signal transduction after target-receptor binding, as well as any derivatives or variant sequences thereof and any synthetic sequences having the same functionality as these sequences.

In some cases, the intracellular signal domain may comprise a signal motif of a known immune receptor tyrosine activation motif (ITAM). Examples of ITAM comprising cytoplasmic signaling sequences include functional signaling domains of protein DAP10 or DAP12 derived from TCRζ, FcRγ, FcRβ, CD3γ, CD3δ, CD3ε, CD5, CD22, CD79a, CD79b, CD66d. However, in a preferred embodiment, the intracellular signal domain is derived from CD3ζ chain.

An example of a T cell signal domain containing one or more ITAM motifs is CD3ζ domain, also known as T cell receptor T3ζ chain or CD247. This domain is a part of the T cell receptor-CD3 complex and plays an important role in combining the antigen recognition of several intracellular signal transduction pathways with the main effector activation of T cells. As used herein, CD3ζ refers primarily to human CD3ζ and isoforms thereof, as known from Swissprot entry P20963, and includes proteins with substantially the same sequence. As part of the chimeric antigen receptor, it is reiterated that full T cell receptor T3ζ chain is not necessary, and any derivative comprising the signal domain of T cell receptor T3ζ chain is suitable, including any functional equivalent thereof.

The intracellular signaling domain may be selected from any one of the domains of Table 1. In some cases, the domain can be modified such that the identity to the reference domain can be about 50% to about 100%. Any one of the domains of Table 1 may be modified such that the modified form may comprise about 50, 60, 70, 80, 90, 95, 96, 97, 98, 99 or up to about 100% identity.

The intracellular signaling region of a CAR may further comprise one or more co-stimulatory domains. The intracellular signaling region may comprise a single co-stimulatory domain, such as ζ chain (first generation of CAR) or with CD28 or 4-1BB (second generation of CAR). In other examples, the intracellular signaling region may comprise two co-stimulatory domains, such as CD28 / OX40 or CD28 / 4-1BB (third generation).

Together with an intracellular signal domain, such as CD8, these co-stimulatory domains can generate downstream activation of kinase pathways, thereby supporting gene transcription and functional cellular responses. The co-stimulatory domain in a CAR can activate CD28 (phosphatidylinositol-4,5-bisphosphate 3-kinase) or 4-1BB / OX40 (TNF-receptor-associated factor adaptor) pathways as well as MAPK and Akt activation-associated proximal signal protein.

In some cases, signals generated by CAR may be combined with auxiliary or co-stimulatory signals. For co-stimulatory signal domains, chimeric antigen receptor-like complexes can be designed to comprise several possible co-stimulatory signal domains. As is well known in the art, in naive T cells, individual engagment of T cell receptors are not sufficient to induce complete activation of T cells into cytotoxic T cells. A second costimulatory signal is necessary for complete activation of T cells. Several receptors have been reported to provide co-stimulation to the activation of T cells, including, but not limited to CD28, OX40, CD27, CD2, CD5, ICAM-1, LFA-1 (CD11a / CD18), 4-1BBL, MyD88, and 4- 1BB. All of the signal transduction pathways used by these co-stimulatory molecules can synergize with primary T cell receptor activation signals. The signals provided by these co-stimulatory signaling regions can synergize with the main effect activation signals derived from one or more ITAM motifs (such as CD3zeta signaling domain), and can fulfill the requirements of T cell activation.

In some cases, a co-stimulatory domain can be added to a chimeric antigen receptor-like complex for enhancing the efficacy and durability of engineered cells. In another embodiment, the T cell signal domain and the co-stimulatory domain are fused to each other to form a signaling region.

**Table 1. Co-stimulatory domains**

| Gene marker | Abbreviation | Name |
|---|---|---|
| CD27 | CD27; T14; S152; Tp55; TNFRSF7; S152. LPFS2 | CD27 molecule |
| CD28 | Tp44; CD28; CD28 antigen | CD28 molecule |
| TNFRSF9 | ILA; 4-1BB; CD137; CDw137 | Tumor necrosis factor receptor superfamily member 9 |
| TNFRSF4 | OX40; ACT35; CD134; IMD16; TXGP1L | Tumor necrosis factor receptor superfamily member 4 |
| TNFRSF8 | CD30; Ki-1; D1S166E | Tumor necrosis factor receptor superfamily member 8 |
| CD40LG | IGM; IMD3; TRAP; gp39; CD154; CD40L; HIGM1; T-BAM; TNFSF5; hCD40L | CD40 ligand |
| ICOS | AILIM; CD278; CVID1 | Inducible T cell co-stimulator |
| ITGB2 | LAD; CD18; MF17; MFI7; LCAMB; LFA-1; MAC-1 | Integrin β2 (Complement component 3 receptor 3 and 4 subunits) |
| CD2 | T11; SRBC; LFA-2 | CD2 molecule |
| CD7 | GP40; TP41; Tp40; LEU-9 | CD7 molecule |
| KLRC2 | NKG2C; CD159c; NKG2-C | Killer lectin-like receptor subfamily |
| | | C, member 2 |
| TNFRSF18 | AITR; GITR; CD357; GITR-D | Tumor necrosis factor receptor superfamily member 18 |
| TNFRSF14 | TR2; ATAR; HVEA; HVEM; CD270; LIGHTR | Tumor necrosis factor receptor superfamily member 14 |
| HAVCR1 | TIM; KIM1; TIM1; CD365; HAVCR; KIM-1; TIM-1; TIMD1; TIMD-1; HAVCR-1 | Hepatitis A virus cell receptor 1 |
| LGALS9 | HUAT; LGALS9A, Galectin-9 | Lectin, galactoside binding, soluble 9 |
| CD83 | BL11; HB15 | CD83 molecule |

The term "adjustment" as used herein refers to a positive or negative change. Examples of adjustment include changes of 1%, 2%, 10%, 25%, 50%, 75%, or 100%.

The term "treatment" as used herein refers to a clinical intervention in an attempt to alter an individual or treat a disease caused by a cell, both for prevention and for intervention in a clinical pathological process. Therapeutic effects include, but are not limited to, preventing the occurrence or recurrence of the disease, reducing symptoms, reducing the direct or indirect pathological consequences of any disease, preventing metastasis, slowing the progress of the disease, improving or alleviating the condition, and alleviating or improving the prognosis.

As used herein, the term "immunocompromised" refers to a subject having an immune deficiency that is easily infected. Organisms that cause opportunistic infections usually do not cause illness in peoples with healthy immune systems, but can infect peoples with weakened immune systems or suppressed immune systems.

The term "constitutive expression" as used herein refers to expression under all physiological conditions.

As used herein, the term "inducible expression" refers to expression under conditions, for example, when T cells bind to an antigen. a skilled person will know how to perform conventional "inducible expression".

### Genetically engineered T cells

The genetically engineered T cells described herein refer to T cells that have been engineered by means of genetic engineering. In particular, the genetically engineered T cell of the present invention refers to a T cell that co-expresses an antigen (such as a tumor antigen)-specific chimeric receptor and exogenous open reading frame (for example, open reading frame for a cytokine), thereby exerting targeting and killing effects; and the genetically engineered T cells of the present invention especially refer to a T cell that co-expresses an antigen (such as tumor antigens)-specific chimeric receptors and (exogenous) cytokines, while the endogenous T cell receptor (TCR) is inactive or inactivated.

In some embodiments, the transmembrane region of the antigen-binding receptor can be selected from the transmembrane region of a protein such as CD8 or CD28. Human CD8 protein is a heterodimer, consisting of two chains of αβ or γδ. In some embodiments, the transmembrane region is selected from the transmembrane region of CD8α or CD28. In addition, the CD8α hinge is a flexible region. Therefore, the transmembrane region of CD8 or CD28 and the hinge region are used to connect the target recognition domain scFv of the antigen-binding receptor CAR with the intracellular signal region.

The intracellular signal domain of the present invention may be selected from the group consisting of CD3ζ, FcεRIγ, CD28 co-stimulatory signal domain, CD137 co-stimulatory signal domain, and combinations thereof. The CD3 molecule consists of five subunits, in which the CD3ζ subunit (also known as CD3 zeta, abbreviated as Z) comprises 3 ITAM motifs, which is an important signal transduction region in the TCR-CD3 complex. In addition, as mentioned above, CD28 and CD137 are co-stimulatory signal molecules, and after binding to their respective ligands, the co-stimulatory effect produced by the intracellular signal segment leads to the continuous proliferation of T lymphocytes, can increase the secretion levels of cytokines such as IL-2 and IFN-γ and increase the survival cycle and antitumor effects of CAR T cells *in vivo.* In some embodiments, the intracellular signaling domain is CD3ζ signal domain or a combination of CD3ζ signal domain and other co-stimulatory signal domain, such as CD28.

In some embodiments, the engineered T cells of the present invention may include an expression construct, in which there are elements connected in the following order: an antibody, CD28 co-stimulatory signal domain, CD3ζ and NFAT6 binding motif reversely linked to the aforementioned element, IL12 expression unit. Preferably, the antibody and the CD28 co-stimulatory signal domain are connected through a CD8α transmembrane region and a CD8α hinge region.

According to one aspect of the invention, the invention also includes a nucleic acid encoding the antigen-binding receptor. The present invention also relates to a variant of the above-mentioned polynucleotide, which encodes a polypeptide having the same amino acid sequence as the present invention or fragments, analogs and derivatives of the polypeptide.

The present invention also provides a vector comprising the above-mentioned nucleic acid encoding an antigen-binding receptor protein expressed on the surface of T cells. In a specific embodiment, the vector used in the present invention is a lentiviral plasmid vector pRRLSIN-cPPT.PGK-GFP.WPRE. It should be understood that other types of viral vectors and non-viral vectors are also applicable.

The invention also includes viruses comprising the vectors described above. The virus of the present invention includes an infectious virus after being packaged, and also includes a virus to be packaged which contains necessary components for being packaged as an infectious virus. Other viruses known in the art that can be used to transduce foreign genes into T cells and the corresponding plasmid vectors can also be used in the present invention.

Based on the teachings of the present invention, a skilled person in the art can know that the idea of the present invention can also be applied to other immune effector cells, that is, immune effector cells co-expressing antigen (such as tumor antigen)-specific chimeric receptors and (exogenous) cytokines. And the expression of the exogenous cytokines is not non-specifically activated, that is, it is not activated by a non-tumor target antigen. The other immune effector cells include, but not limited to, NK cells, plasma cells, and the like.

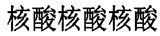 **Nucleic acid**

The nucleic acids described herein have meanings as commonly understood by a skilled person in the art. In particular, the nucleic acid of the present invention refers to a nucleic acid used for genetically engineering a T cell, including a nucleic acid encoding an antigen-binding receptor, such as a chimeric antigen receptor, a nucleic acid encoding the open reading frame for an exogenous, such as cytokines, and a nucleic acid for inactivating the endogenous T cell receptor (TCR), such as for knocking out the endogenous T cell receptor.

Many virus-based systems have been developed for transferring genes into mammalian cells. For example, retroviruses provide a convenient platform for gene delivery systems. The selected gene can be inserted into a vector and packaged in a retroviral particle using techniques known in the art. Vectors derived from retroviruses such as lentivirus are suitable tools to achieve long-term gene transfer, as they allow the long-term stable integration and reproduction in daughter cells of a transgene. Lentiviral vectors have additional advantages over vectors derived from retroviruses such as murine leukemia virus, as they can transduce non-proliferating cells. They also have the additional advantage of low immunogenicity. The advantage of adenoviral vectors is that they do not fuse into the genome of a target cell, thereby bypassing negative integration-related events.

Cells can be transfected with a transgene encoding the antigen-binding receptor. The concentration of a transgene can be from about 100 picograms to about 50 micrograms. In some cases, the amount of nucleic acid (e.g., ssDNA, dsDNA, or RNA) introduced into a cell can be altered to optimize transfection efficiency and / or cell viability. For example, 1 microgram of dsDNA can be added to each cell sample for electroporation. In some cases, the amount of nucleic acid (e.g., double-stranded DNA) required for optimal transfection efficiency and / or cell viability will vary depending on the cell type. In some cases, the amount of nucleic acid (e.g., dsDNA) used for each sample can directly correspond to transfection efficiency and / or cell viability, for example, a range of transfection concentrations. The transgene encoded by the vector can be integrated into the genome of a cell. In some cases, the transgene encoded by the vector is forward integrated. In other cases, the transgene encoded by the vector is reverse integrated.

In some cases, the immunoreactive cells may be stem memory T cells TSCM cells consisting of CD45RO(-), CCR7(+), CD45RA(+), CD62L+(L-selectin), CD27+, CD28+, and / or IL-7Rα+, which can also express CD95, IL-2Rβ, CXCR3, and / or LFA-1, and show many functional properties different from the stem memory cells. Alternatively, the immunoreactive cells may be central memory TCM cells comprising L-selectin and CCR7, wherein the central memory cells may secrete, for example, IL-2, instead of IFNγ or IL-4. The immunoreactive cells may also be effector memory TEM cells comprising L-selectin or CCR7, and produce, for example, effector cytokines such as IFNγ and IL-4.

The delivery vehicle is typically delivered systemically (e.g., intravenously, intraperitoneally, intramuscularly, subcutaneously, or intracranially) or by topical administration to an individual patient, as described below. Alternatively, the vector may be delivered to cells *ex vivo,* such as cells removed from an individual patient (e.g., lymphocytes, T cells, bone marrow aspirate, tissue biopsy), and then the cells are usually implanted into the patient after the cells that incorporate the vector are selected. Cells can be expanded before or after selection.

Suitable immunoreactive cells for expressing antigen-binding receptors may be cells that are autologous or non-autologous to the individual in need thereof.

The T cells can be obtained from many sources, including PBMC, bone marrow, lymph node tissue, umbilical cord blood, thymus tissue, and tissue from infected sites, ascites, pleural effusion, spleen tissue, and tumors. In some cases, T cells can be obtained from blood collected from the individual using any number of techniques known to a skilled person in the art, such as FicollTM isolation. In one embodiment, cells from a subject's circulating blood are obtained by apheresis. Apheresis products usually contain lymphocytes, including T cells, monocytes, granulocytes, B cells, other nucleated white blood cells, red blood cells, and platelets. In one embodiment, the cells collected by apheresis can be washed to remove the plasma fraction and placed in a suitable buffer or medium for subsequent processing steps.

Alternatively, cells can be derived from healthy donors, patients diagnosed with cancer, or patients diagnosed with infection. In some embodiments, the cells can be a part of a mixed cell population with different phenotypic characteristics. Cell lines can also be obtained from transformed T cells according to the aforementioned methods. Cells can also be obtained from cell therapy libraries. Modified cells resistant to immunosuppressive therapy can be obtained by any of the methods described herein. It is also possible to select a suitable cell population before modification. Engineered cell populations can also be selected after modification. Engineering cells can be used for autologous transplantation. Alternatively, the cells can be used for allogeneic transplantation. In some cases, the cells are administered to a sample for identifying the same patient of cancer-related target sequences. In other cases, the cells are administered to a different sample for identifying a patient of cancer-related target sequences.

In some cases, suitable primary cells include peripheral blood mononuclear cells (PBMC), peripheral blood lymphocytes (PBL), and other subpopulations of blood cells, such as, but not limited to, T cells, natural killer cells, monocytes, natural killer T cells, monocyte precursor cells, hematopoietic stem cells or non-pluripotent stem cells. In some cases, the cells can be any immune cells, including any T cells, such as tumor infiltrating cells (TIL), such as CD3+ T cells, CD4+ T cells, CD8+ T cells, or any other type of T cell. T cells can also include memory T cells, memory stem T cells, or effector T cells. T cells can also be selected from a large population, such as from whole blood. T cells can also be expanded from a large population. T cells may also tend to specific populations and phenotypes. For example, T cells can tend to a phenotype comprising CD45RO(-), CCR7(+), CD45RA(+), CD62L(+), CD27(+), CD28(+), and / or IL-7Rα(+). Suitable cells can be selected from one or more markers from the following list: CD45RO(-), CCR7(+), CD45RA(+), CD62L(+), CD27(+), CD28(+), and / or IL-7Rα(+). Suitable cells also include stem cells, such as, embryonic stem cells, induced pluripotent stem cells, hematopoietic stem cells, neuronal stem cells, and mesenchymal stem cells. Suitable cells may include any number of primary cells, such as human cells, non-human cells, and / or mouse cells. Suitable cells may be progenitor cells. Suitable cells may be derived from a subject (e.g., a patient) to be treated.

The amount of therapeutically effective cells required in a patient may vary depending on the viability of the cells and the efficiency with which the cells are genetically modified (e.g., the efficiency with which a transgene is integrated into one or more cells, or the expression level of a protein encoded by the transgene). In some cases, the product (e.g., doubling) of viability of a genetically modified cell and the efficiency of transgene integration may correspond to the amount of treatment of the cells available to the subject. In some cases, an increase in cell viability after genetic modification may correspond to a decrease in the amount of necessary cells that is effective for treating the patient. In some cases, an increase in the efficiency of integration of a transgene into one or more cells may correspond to a decrease in the amount of necessary cells that is effective for treating the patient. In some cases, determining the necessary amount of therapeutically effective cells may include determining functions related to changes in cells over time. In some cases, determining the necessary amount of therapeutically effective cells can include determining functions corresponding to changes in efficiency in integrating the transgene into one or more cells based on time-dependent variables (e.g., cell culture time, electroporation time, stimulation time for cells). In some cases, the therapeutically effective cells can be a population of cells that comprises from about 30% to about 100% expression of an antigen-binding receptor on the cell surface. In some cases, the therapeutically effective cells can express about 30%, 35%, 40%, 45%, 50%, 55%, 60%, 65%, 70%, 75% 80%, 85%, 90%, 91%, 92%, 93%, 94%, 95%, 96%, 97%, 98%, 99%, 99.5%, 99.9% or more than about 99.9% of the antigen-binding receptor on the cell surface, as measured by flow cytometry.

In some cases, when the antigen-binding receptor is present on the plasma membrane of a cell, and activated by binding to a target, it will be toxic to cells expressing on the surface a target, to which the antigen-binding receptor will bind. For example, in some cases, when the antigen-binding receptor described herein is present in the cell's plasma membrane, the cell can be a cytotoxic cell (e.g., a NK cell or a cytotoxic T lymphocyte), and when the cell is activated by binding to its target, it can increase the cytotoxic activity of cytotoxic cells on target cells. For example, in some cases, an antigen-binding receptor described herein, when activated by the binding of its target, can increase cytotoxicity by at least 10%, at least 15%, at least 20%, at least 25%, at least 30%, at least 40%, at least 50%, at least 75%, at least 2 times, at least 2.5 times, at least 5 times, at least 10 times or more than 10 times, as compared with cytotoxicity in the absence of cells binding to the target.

### Pharmaceeutical composition

The T cells of the present invention can be used to prepare a pharmaceutical composition. In addition to an effective amount of T cells, the pharmaceutical composition may further include a pharmaceutically acceptable carrier. The term "pharmaceutically acceptable" means that when the molecular itself and composition are properly administered to an animal or human, they do not cause adverse, allergic or other adverse reactions.

Specific examples of some substances that can be used as a pharmaceutically acceptable carrier or a component thereof are antioxidants; preservatives; pyrogen-free water; isotonic saline solutions; and phosphate buffers and the like.

The composition of the present invention can be prepared into various dosage forms as necessary, and can be administered by a physician according to the patient's type, age, weight, general disease status, administration mode and other factors. The method of administration may be, for example, parenteral administration (such as injection) or other treatment methods.

"Parenteral" administration of immunogenic compositions includes, for example, subcutaneous (s.c.), intravenous (i.v.), intramuscular (i.m.) or intrasternal injection or infusion techniques.

A formulation comprising an immunoreactive cell population administered to an individual comprises a plurality of immunoreactive cells effective to treat and / or prevent a particular indication or disease. Therefore, a therapeutically effective population of immunoreactive cells can be administered to an individual. Typically, a formulation comprising about 1 × 10⁴ to about 1 × 10¹⁰ immunoreactive cells is administered. In most cases, the formulation will contain about 1 × 10⁵ to about 1 × 10⁹ immunoreactive cells, about 5 × 10⁵ to about 5 × 10⁸ immunoreactive cells, or about 1 × 10⁶ to about 1 × 10⁷ immunoreactive cells. However, the number of CAR immunoreactive cells administered to the individual will vary between a wide range, depending on the location, source, identity, extent and severity of the cancer, the age and physical condition of the individual to be treated. A doctor will ultimately determine the appropriate dose to be used.

In some embodiments, a chimeric antigen receptor is used to stimulate an immune cell-mediated immune response. For example, a T cell-mediated immune response is an immune response involving T cell activation. Activated antigen-specific cytotoxic T cells are capable of inducing apoptosis in target cells that display exogenous antigen epitopes on the surface, such as cancer cells that display tumor antigens. In another embodiment, a chimeric antigen receptor is used to provide antitumor immunity in a mammal. The subject will develop anti-tumor immunity due to the T cell-mediated immune response.

In some cases, a method of treating a subject with cancer may involve administering one or more T cells described herein to a subject in need thereof. The T cells can bind to tumor target molecules and induce cancer cell death. As mentioned above, the invention also provides a method for treating a pathogen-caused infection in an individual, comprising administering to said individual a therapeutically effective amount of a T cell of the invention.

### Combination with anti-tumor drugs

In some embodiments, the T cells of the invention can be administered in combination with another therapeutic agent. In some embodiments, the another therapeutic agent is a chemotherapeutic agent. The chemotherapeutic agent that can be used in combination with T cells of the present invention includes, but not limited to, mitotic inhibitors (vinca alkaloids), including vinblastine, vinblastine, vinblastine, and novibin (TM) (vinorelbine, 5'-dehydrosulfide); topoisomerase I inhibitors, such as camptothecin compounds, including CamptosarTM (Irinotecan HCL), HycamtinTM (Topotecan HCL) and other compounds derived from camptothecin and its analogs; podophyllotoxin derivatives, such as etoposide, teniposide, and midazoxid; alkylating agents, cisplatin, cyclophosphamide, nitrogen mustard, trimethylenethiophosphoramid, carmustine, busulfan, chlorambucil, britazine, uracil mustard, cloprofen, and dacarbazine; antimetabolites, including cytarabine, fluorouracil, methotrexate, thiopurine, azathioprine and procarbazine; antibiotics, including but not limited to doxorubicin, bleomycin, dactamomycin, daunorubicin, mycinomycin, mitomycin, sarcomycin C, and daunomycin; and other chemotherapeutic drugs, including but not limited to antitumor antibodies, dacarbazine, azacytidine, amsacon, melphalan, ifosfamide and mitoxantrone.

In some embodiments, chemotherapeutic drugs that can be used in combination with T cells of the invention include, but not limited to, anti-angiogenic agents, including anti-VEGF antibodies (including humanized and chimeric antibodies, anti-VEGF aptamers, and antisense oligonucleotide) and other angiogenesis inhibitors, such as angiostatin, endostatin, interferon, retinoic acid, and tissue inhibitors of metalloproteinases-1 and -2.

### Kit

The invention also provides a kit comprising a T cell of the invention. The kit can be used to treat or prevent cancer, pathogen infection, immune disorder, or allograft. In one embodiment, a kit can include a therapeutic or prophylactic composition comprising one or more unit dosage forms of an effective amount of T cells.

In some embodiments, the kit comprises a sterile container that can contain the therapeutic or prophylactic composition.

In some cases, the kit can include about 1 × 10⁴ cells to about 1 × 10⁶ cells. In some cases, the kit can include at least about 1 × 10⁵ cells, at least about 1 × 10⁶ cells, at least about 1×10⁷ cells, at least about 4×10⁷ cells, at least about 5×10⁷ cells, at least about 6×10⁷ cells, at least about 6×10⁷ cells, 8×10⁷ cells, at least about 9×10⁷ cells, at least about 1×10⁸ cells, at least about 2×10⁸ cells, at least about 3×10⁸ cells, at least about 4×10⁸ cells, at least about 5×10⁸ cells, at least about 6×10⁸ cells, at least about 6×10⁸ cells, at least about 8×10⁸ cells, at least about 9×10⁸ cells, at least about 1×10⁹ cells, at least about 2×10⁹ cells, at least about 3×10⁹ cells, at least about 4×10⁹ cells, at least about 5×10⁹ cells, at least about 6×10⁹ cells, at least about 8×10⁹ cells, at least about 9×10⁹ cells, at least about 1×10¹⁰ cells, at least about 2×10¹⁰ cells, at least about 3×10¹⁰ cells, at least about 4×10¹⁰ cells, at least about 5×10¹⁰ cells, at least about 6×10¹⁰ cells, at least about 9×10¹⁰ cells, at least about 9×10¹⁰ cells, at least about 1×10¹¹ cells, at least about 2×10¹¹ cells, at least about 3×10¹¹ cells, at least about 4×10¹¹ cells, at least about 5×10¹¹ cells, at least about 8×10¹¹ cells, at least about 9×10¹¹ cells, or at least about 1× 10¹² cells. For example, the kit can include about 5×10¹⁰ cells.

In some cases, the kit may include allogeneic cells. In some cases, the kit can include cells that can include genomic modifications. In some cases, the kit may contain "ready-to-use" cells. In some cases, the kit can include cells that can be expanded for clinical use. In some cases, the kit may contain contents for research purposes.

Autologous lymphocyte infusion can be used for treatment. Peripheral blood mononuclear cells (PBMCs) can be collected from patients in need of treatment, and T cells can be activated and expanded using methods described herein and known in the art, and then injected into the patient. In other cases, allogeneic cells can be used to treat patients.

The methods disclosed herein may include transplantation. Transplantation can refer to adoptive transplantation of cell products. The transplant can be an autograft, allograft, xenograft, or any other transplant. For example, the transplant can be a xenograft. The transplant can also be an allograft.

### Example 1. Preparation of IL12-GPC3-CAR T cells

### 1. Construction of Plasmid

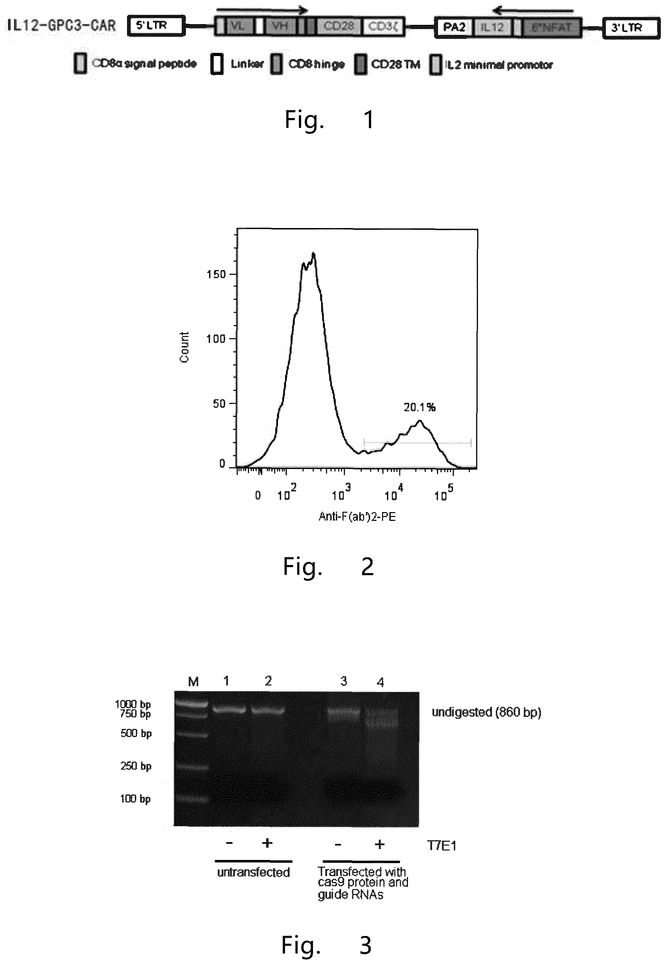

The scFv used in this example is an antibody targeting GPC3, and the nucleic acid sequence is shown in SEQ ID NO: 1. The chimeric antigen receptor used is a second-generation of chimeric antigen receptor, which has a transmembrane domain of CD28, intracellular domain of CD28 and CD3ζ. A plasmid for IL12-GPC3-CAR T cells was constructed according to the plasmid map as shown in Figure 1.

A lentiviral plasmid pRRLSIN.cPPT.EF-1α-GPC3-CAR-T expressing a second-generation of chimeric antigen receptor was constructed by using pRRLSIN.cPPT.EF-1α as a vector. The GPC3-CAR-T sequence consisted of a CD8α signal peptide (SEQ ID NO: 2), scFv targeting GPC3 (SEQ ID NO: 1), CD8 hinge (SEQ ID NO: 3) and CD28 transmembrane region (SEQ ID NO: 6), intracellular signaling domain (SEQ ID NO: 4) and intracellular segment CD3ζ of CD3 (SEQ ID NO: 5).

Regarding IL12-GPC3-CAR T cells, NFAT6-IL12 sequence was inserted into pRRLSIN.cPPT.EF-1α-GPC3-CAR-T plasmid, thereby constructing a lentivirus plasmid expressing a second generation of chimeric antigen receptor of GPC3 and of IL12, pRRLSIN.cPPT.EF-1α-IL12-GPC3-CAR T. The NFAT6-IL12 sequence consisted of 6 * NFAT binding motif (SEQ ID NO: 7), IL2 minimal promoter (SEQ ID NO: 8), IL12 signal peptide and IL12 p40 (SEQ ID NO: 10), (G4S)3 Linker (SEQ ID NO: 9), IL12 p35 (SEQ ID NO: 11), PA2 (SEQ ID NO: 12).

### 2. Lentivirus packaging, virus concentration and titer determination

### a. Lentiviral packaging

1) 293T cells cultured to the 6^{th} to 10^{th} generation was inoculated at a density of 5 × 10⁶ in a petri dish, and culture at 37°C, 5% CO₂ overnight. The culture medium was DMEM containing 10% fetal bovine serum (Gibico);
2) the target gene plasmid pRRLSIN.cPPT.EF-1α-IL12-GPC3-CAR T 5.4 µg, the packaging plasmids pRsv-REV 6.2 µg, RRE-PMDLg 6.2 µg, Vsvg 2.4 µg were dissolved into 800 µL of blank DMEM culture solution and mixed to obtain a plasmid mixture;
3) 60 µg PEI (1 µg / (µl) was dissolved in 800 µl serum-free DMEM medium, gently mixed (or vortexed at 1000 rpm for 5 seconds), and incubated at room temperature for 5 minutes to obtain a PEI mixture;
4) the plasmid mixture was added into the PEI mixture, homogeneously mixed and incubated for 20 min at room temperature to form a transfection complex;
5) 1.6 ml of the transfection complex was added dropwise to a 10cm petri dish containing 11 ml of DMEM medium. After 4-5 hours, the transfected 293T cells were exchanged with 10% FBS DMEM and incubated at 37°C for 72h. The supernatant of the virus solution was collected.

### b. Lentivirus concentration

1) preparation of 5 × PEG8000 NaCl: 8.766 g of NaCl and 50 g of PEG8000 was weighed and dissolved in 200 ml of Milli-Q pure water; sterilized at 121°C for 30 min, and stored at 4°C;
2) a 0.45 µm filter was used to filter the virus supernatant. 5 × PEG-8000 NaCl stock liquor 7.5 ml was added to each 30 ml of the filtered virus initial solution; mixed once every 20-30 minutes for 3-5 times; and placed at 4°C overnight;
3) the mixture obtained in step 2) was centrifuged at 4°C, 4000 g for 20 min; the supernatant was aspirated and discarded, the resulting precipitate stood and the residual liquid was removed; an appropriate amount of lentivirus lysing solution was added to dissolve the lentivirus precipitate; and the concentrated virus suspension was store at -80°C.

### c. Lentiviral titer determination

1) 293T cells was inoculated into a 6-well petri dish at 2 × 10⁵ cells, 2 ml / well; a polybrene solution at an initial concentration of 10 µg / µl was added at 0.6 µg/ml with a final concentration of 6 µg / ml; incubated at 37°C, 5% CO2 for 1 hour, and the culture medium was DMEM containing 10% fetal bovine serum;
2) 10 µL / well of virus concentrated solution was added for 5-fold dilution, 3 gradients, and cultured at 37°C, 5% CO₂;
3) After 72 hours of infection, 293T cells were digested with trypsinize (30s), 1 ml of DMEM (10% FBS) was added to quench the digestion, the cell suspension was transferred into a 2 ml centrifuge tube (halve), and centrifuged at 5000 rpm for 5 min; the supernatant was discarded; and washed twice with PBS (2% NBS);
4) 50 µl of PE-SA (1: 200 dilution) antibody was added to the cells of the control group and incubated on ice for 45 min, washed twice with PBS (2% NBS), and resuspended as a control; 50 µl of 1: 50 diluted biotin-Goat anti human IgG, F (ab')2 antibody was added to the cells of the test group, incubated on ice for 45min; and washed twice with PBS (2% NBS); and 50µl PE-SA (1: 200 dilution) antibody was added and incubated on ice for 45min;
5) the cells were resuspended by adding 2 ml PBS (2% NBS), and centrifuged at 5000 rpm / min, 4°C for 5 minutes, and the supernatant was discarded; the process was repeated twice; 500 µl PBS (2% NBS) was added and transfered to a flow tube. Flow cytometry was used to detect PE channels. The number of cells with a positive rate of 5-20% was appropriate. The titer (U / mL) was calculated = number of cells × positive rate / virus volume, and the virus titers after concentration are:
   IL12-GPC3-CAR T: 1×10⁸ U/ml.

### 3. Preparation of IL12-GPC3-CAR T cells

1) a 24-well plate was coated with RetroNectin: 380 µl of 5 µg / ml RetroNectin solution (PBS) was added to each well, and incubated at 4°C overnight;
2) the RetroNectin solution (PBS) in the 24-well plate was discarded and wash it twice with 1 ml of PBS; the cells were inoculated in a 24-well plate coated with Retronectin with the number of cells in each well of 5 × 10⁵, and the volume of the culture solution was 500 µl; the concentrated lentivirus was added to PBMC cells at MOI = 15, centrifuged at 1800 rpm, 32°C for 40 min, and transferred to a cell culture incubator;
3) Expansion culture: The infected cells were passaged every other day at a density of 5 × 10⁵ / mL, and at the same time, a final concentration of 500 U / mL of recombinant human IL-2 was added to the lymphocyte culture solution.

The determination of the positive rate (purity) of IL12-GPC3-CAR T cells was performed through flow cytometry using the conventional operation in the art (the determination method is a conventional method in the art, such as the method disclosed by Kowolik et al., 2006), and IL12-GPC3-CAR T is a T cell transfected with pRRLSIN.cPPT.EF-1α-9F2-28Z-NFAT6-IL12. The results are shown in Figure 2: after lentivirus infection, the proportion of positive T cells with a chimeric antigen receptor on the cell surface was 20.1%.

### Example 2. Construction of TCR-deficient IL12-GPC3-CAR T cells

### 1. Construction of CRISPR and in vitro transcription of corresponding gRNA

Two gRNAs used to knock out the TCRα chain targeted the first exon of the constant region of the TCRα chain. The two gRNA target sequences are:
TRAC-gRNA-1 (SEQ ID NO: 13): GAGTCTCTCAGCTGGTACA
TRAC-gRNA-2 (SEQ ID NO: 14): TGTGCTAGACATGAGGTCTA.

The T7 promoter-driven gRNA *in vitro* transcription box is as follows:
T7 Promoter + target gRNA + guide RNA Scaffold
TRAC-gRNA-1-IVT-Template (SEQ ID NO: 15): 
TAATACGACTCACTATAGAGAGTCTCTCAGCTGGTACAGTTTTAGAGCTAGAAAT
AGCAAGTTAAAATAAGGCTAGTCCGTTATCAACTTGAAAAAGTGGCACCGAGTCGGT
GCTTTTTTT
TRAC-gRNA-2-IVT-Template (SEQ ID NO: 16):

TAATACGACTCACTATAGTGTGCTAGACATGAGGTCTAGTTTTAGAGCTAGAAAT
AGCAAGTTAAAATAAGGCTAGTCCGTTATCAACTTGAAAAAGTGGCACCGAGTCGGT
GCTTTTTTT

The DNA sequence of the above TRAC-gRNA-1 / 2-IVT-Template was amplified and synthesized through Overlap PCR, cloned into MluI / SalI site on MSCV vector, and the vector was further confirmed by sequencing. The forward primers for Overlap PCR used to synthesize the T7 promoter-driven gRNA *in vitro* transcription frame are:
TRAC-gRNA-1-MluI-Overlap-Forward (SEQ ID NO: 17): 
CCGACGCGTTAATACGACTCACTATAGAGAGTCTCTCAGCTGGTACAGTTTTAGAGCT
AGAAATAGCAAGTTAAAATAAG
TRAC-gRNA-1-MluI-Overlap-Forward (SEQ ID NO: 18):

CCGACGCGTTAATACGACTCACTATAGTGTGCTAGACATGAGGTCTAGTTTTAGAGCT
AGAAATAGCAAGTTAAAATAAG

The reverse primers for Overlap PCR used to synthesize the T7 promoter-driven gRNA *in vitro* transcription frame are:
All-gRNA-SalI-Overlap-Reverse (SEQ ID NO: 19):

ACGCGTCGACAAAAAAAGCACCGACTCGGTGCCACTTTTTCAAGTTGATAACGGACTA
GCCTTATTTTAACTTGCTATTTC

The primer pairs used to amplify the above two transcription frames are: All-gRNA-MOF: CCGACGCGTTAATACGACT (SEQ ID NO: 20); All-gRNA-SOR: ACGCGTCGACAAAAAAAGC (SEQ ID NO: 21)

*In vitro* transcription of gRNA was performed using MEGAshortscript™ T7 Transcription Kit (Thermo fisher, AM1354). In *in vitro* transcription, the corresponding vector linearized by the restriction enzyme SalI was used as a template.

### 2.Knockout of α chain of IL12-GPC3-CAR T cell receptor (TCR)

The IL12-GPC3-CAR T-cell receptor (TCR) α chain was knocked out using Human T Cell Nucleofector® Kit (Lonza, VPA-1002). gRNAs (*in vitro* transcription products of TRAC-gRNA-1-IVT-Template and TRAC-gRNA-1-IVT-Template), Cas9 protein (NEB, M0646M) were electrotransformed into IL12-GPC3-CAR T cells prepared in Example 1 by using Electrotransformer (Lonza, Nucleofector IIb) under T-023 program.

Gene editing efficiency was tested by T7EN1 nuclease (NEB, M0302S) assay. Firstly, the gene editing target region was amplified by PCR with a primer pair as follows: TRAC-F (SEQ ID NO: 22): 5'-TCATGTCCTAACCCTGATCCTCTT-3'; TRAC-R (SEQ ID NO: 23): 5'-TTGGACTTTTCCCAGCTGACAGA-3'. After the PCR product was purified, it was denatured and renatured in 1 × NEBuffer 2. The thermal cycling program was: 95°C, 5 min; 95-85°C, -2°C/s; 85-25°C, -0.1°C/s; 4°C. The denatured and renatured PCR product was digested with T7EN1 nuclease (10 U / 20 µl system) at 37°C for 15 minutes, then detected by 2% agarose gel electrophoresis, and stained with ethidium bromide. As shown in Figure 3, a high percentage of gene mutations occurred at the first exon of the IL12-GPC3-CAR T-cell receptor (TCR) α chain.

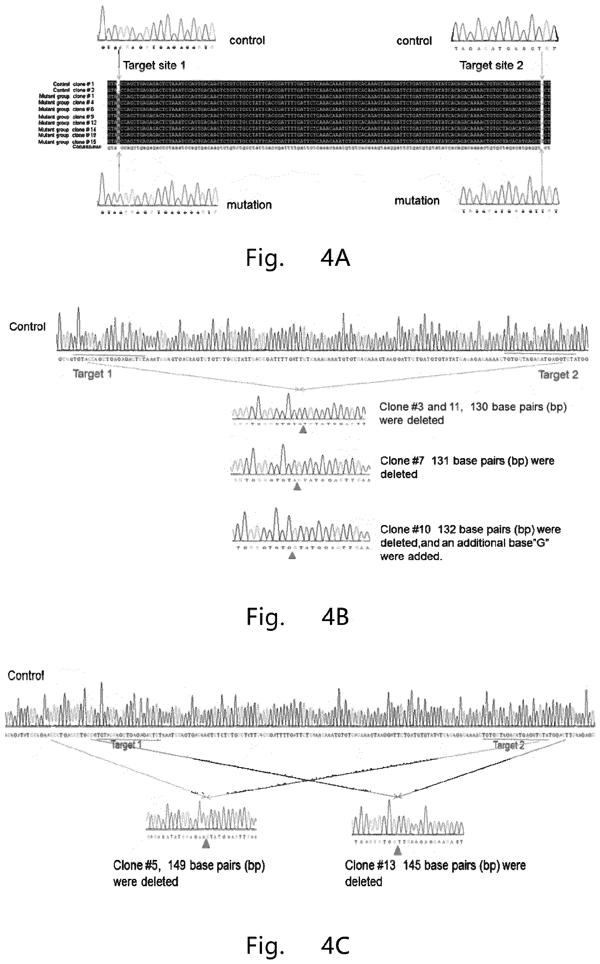

The purified PCR product was ligated into the pGEM-T vector, and the gene editing efficiency was preliminarily calculated by sequencing (picking 16 clones): in the 16 clones, 8 clones (Clone # 1, # 4, # 6, # 9, # 12, # 14, # 15 and # 19; Figure 4a) had insertion mutations, and 6 clones (Clone # 3, # 5, # 7, # 10, # 11, and # 13; Figures 4b and 4c) had deletion mutations.

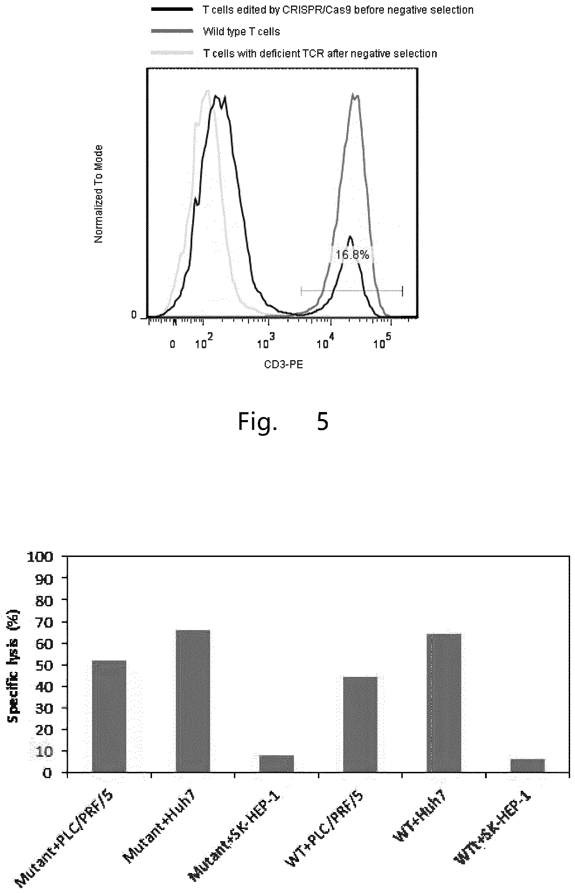

Flow cytometry was used to analyze the expression of CD3ε chain on the surface of IL12-GPC3-CAR T cells after electrotransformation. It was found that after gene editing by the CRISPR / Cas9 system, CD3ε chain could be detected on the cell surface in 16.8% of the cells (Figure 5).

CD3 negative cells were enriched and purified by negative magnetic sorting. Firstly, the cells to be sorted, which were washed with AutoMACS Buffer, were incubated with CD3 microbeads (Miltenyi Biotec, 130-050-101) for 15 minutes at 4°C, washed twice with AutoMACS Buffer, and then loaded on a LD column (Miltenyi Biotec). The effluent was collected, centrifuged at 300 × g for 10 min. Cells were collected, resuspended in AIM-V containing 2% human AB serum (Gemcell, 100-512), and cultured at 37°C, 5% CO2. After magnetic sorting, flow cytometry was used to analyze the expression of CD3ε chain on the surface of IL12-GPC3-CAR T cells, and it was found that the CD3ε chain on the surface of IL12-GPC3-CAR T cells without gene editing was positive; however, for all of the cells subjected to gene editing and magnetic sorting, the CD3ε chains on surface were negative (Figure 5).

### Example 3. Cytotoxicity determination of IL12-GPC3-CAR T cells before and after TCR inactivation

CytoTox 96 non-radioactive cytotoxicity detection kit (Promega) was used to detect cytotoxicity, and details can be found in CytoTox 96 non-radioactive cytotoxicity detection kit manual.
1) Target cells: 50 µL of 2 × 10⁵ / mL Huh-7, PLC/PRF/5, and SK-HEP-1 cells were inoculated into 96 well plates, in which Huh-7 cells and PLC/PRF/5 cells were GPC3 positive, and SK-HEP-1 cells were GPC3 negative;
2) Effector cells: IL12-GPC3-CAR T cells to which TCR + or TCR- was added at an effect target ratio of 1: 1;
3) 5 duplicate wells were set in each group, and the average value for 5 duplicate wells was taken. The detection time was 18h. The experimental groups and control groups are as follows:
   Each experimental group: each target cell + the above effector cell; control group 1: maximum release of LDH from target cells; Control group 2: spontaneous release of LDH from target cells; Control group 3: spontaneous release of LDH from effector cells.

The cytotoxicity calculation formula is: cytotoxicity % = [(experimental group-effector cell spontaneous group-target cell spontaneous group) / (target cell maximum-target cell spontaneous)] * 100.

The experimental results are shown in Figure 6. Compared with IL12-GPC3-CAR T cells with wild-type TCR, IL12-GPC3-CAR T cells with TCR being inactivated showed similar cytotoxicity *in vitro* toxicity experiments. It can be seen that the knockout of TCR does not affect anti-cancer activities of IL12-GPC3-CAR T cells.

### Example 4. Secretion of cytokine from GPC3- CAR-T/IL12-GPC3-CAR T

IL12-GPC3-CAR T was incubated with liver cancer cell lines Huh-7, PLC/PRF/5 and SK-HEP-1 cells at 1: 1 for 24 hours, and the supernatant was collected. The secretion level of cytokine was detected by ELISA.

The sample for detecting IL12 was not necessary to be diluted, and the samples for detecting IL2 and IFN-γ were diluted for 20 times. Double antibody sandwich enzyme-linked immunosorbent assay technology was adopted for ELISA kit. Specific anti-human IL-2, IFN-γ, and IL-12 monoclonal antibodies were pre-coated on a high-affinity microplate, respectively. Standards, samples to be tested, and biotinylated detection antibodies were added to the wells of the microplate. After incubation, the IL-2, IFN-γ, and IL-12 present in the samples bound to the solid-phase and detection antibodies, respectively. After unbound substances were washed off, horseradish peroxidase-labeled streptavidin-HRP was added. After the microplate was washed, the chromogenic substrate TMB was added for development, and the absorbance value was measured at a wavelength of 450 nm (reference wavelength 570-630nm).

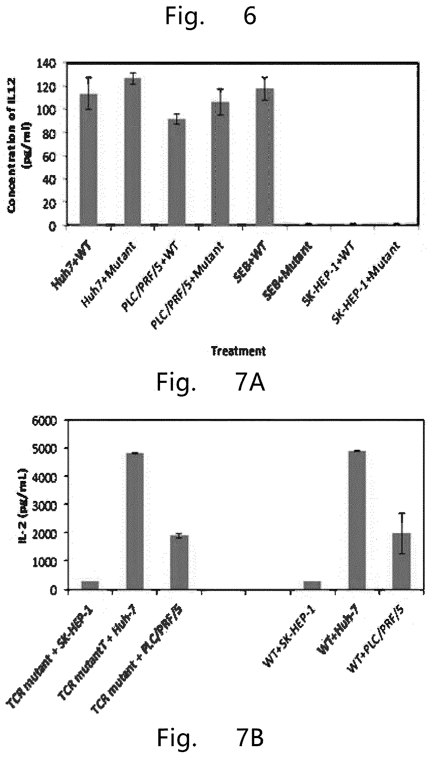

As shown in Figure 7a, when co-incubated with GPC3+ tumor cells Huh-7 and PLC/PRF/5, TCR + and TCR- IL12-GPC3-CAR T secreted higher levels of IL12, and when co-incubated with GPC3- tumor cells SK-HEP-1, IL12 was almost undetectable, indicating that the CAR-T cells secreted IL12 when recognizing the tumor-specific antigen GPC3, and that TCR knockout won't affect IL12 expression induced after tumor-specific antigen GPC3 was recognized by chimeric antigen receptor. However, after non-tumor-specific antigen Staphylococcal enterotoxin B (SEB, final concentration 1 µg / ml) was added to TCR+ and TCR- IL12-GPC3-CAR T culture, it was found that SEB can induce TCR+ IL12-GPC3-CAR T cells to express IL-12 (Figure 7a), but fail to induce TCR- IL12-GPC3-CAR T cells to express IL-12. Therefore, the knockout of TCR can eliminate non-specific expression of IL-12 from IL-12-GPC3-CAR T cells induced by non-tumor-specific antigens.

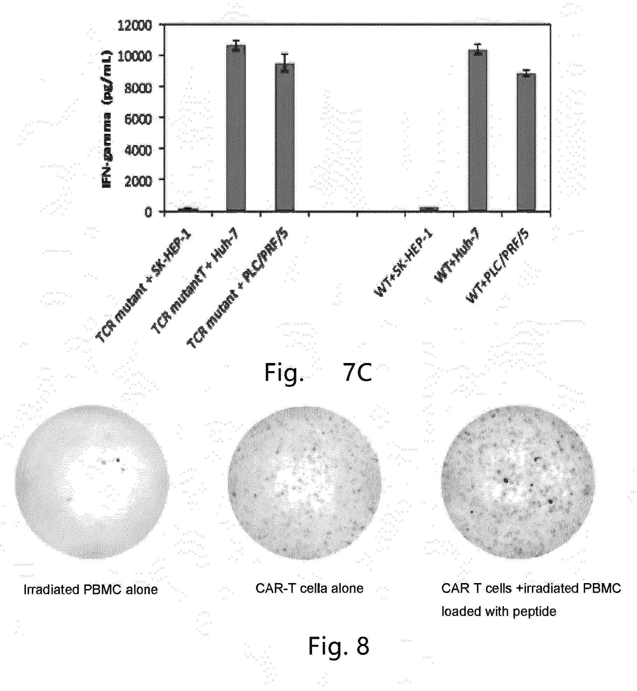

As shown in Figures 7b and c, after incubated with GPC3+ tumor cells Huh-7 and PLC/PRF/5 for 18 hours, the release amounts of IL-2 and IFN-γ from TCR+ and TCR-IL12-GPC3-CAR T were similar, which shows that TCR knockout won't affect the release of IL-2 and IFN-γ from IL12-GPC3-CAR T cells.

### Example 5. Detection of IL12 expression by ELISA

In order to detect the specificity of IL-12 expression in GPC3-CAR T cells (GPC3-28Z-NFAT-IL-12) with the IL-12 encoding gene driven by minimal IL2 promoter regulated by NFAT after TCR knockout, 35Gy-irradiated peripheral blood mononuclear cells loaded with antigen peptide libraries (from Clostridium tetanus, Epstain-Bar virus, cytomegalovirus, and influenza virus A) were co-cultured with 9F2-28Z-NFAT-IL-12 CAR T cells for 18 hours, and then subjected to enzyme-linked immunospot assay. The results are shown in Fig. 8, which show that, compared with GCR3-28Z-NFAT-IL-12 CAR T cells in which TCR was knocked out, production of IL-12 from wild-type GPC3-28Z-NFAT-IL-12 CAR T cells can be significantly induced by PBMC stimulation. Therefore, the specificity of IL-12 expression in GPC3-CAR T cells (GPC3-28Z-NFAT-IL-12) with the IL-12 encoding gene driven by minimal IL2 promoter regulated by NFAT will be enhanced by the knockout of TCR.

The sequences used in the present invention are summarized in the following table:

| SEQ ID NO | construct | Sequence |
|---|---|---|
| 1 | GPC3 scFv | Gaggtgcagctggtgcagagcggcgccgaggtgaagaagcccggcgccagcgtgaaggtgagctgcaaggccagcggctacacc Ttcagcgactacgagatgcactgggtgcggcaggcccccggccagggcctggagtggatgggcgccatccaccccggcagcggc Gacaccgcctacaaccagcggttcaagggccgggtgaccatcaccgccgacaagagcaccagcaccgcctacatggagctgagc Agcctgcggagcgaggacaccgccgtgtactactgcgcccggttctacagctacgcctactggggccagggcaccctggtgacc Gtgagcgccggtggaggcggttcaggcggaggtggttctggcggtggcggatcggacatcgtgatgacccagacccccctgagc Ctgcccgtgacccccggcgagcccgccagcatcagctgccggagcagccagagcctggtgcacagcaacggcaacacctacctg Cagtggtacctgcagaagcccggccagagcccccagctgctgatctacaaggtgagcaaccggttcagcggcgtgcccgaccgg Ttcagcggcagcggcagcggcaccgacttcaccctgaagatcagccgggtggaggccgaggacgtgggcgtgtactactgcagc cagagcatctacgtgccctacaccttcggccagggcaccaagctggagatcaaacgt |
| 2 | Human CD8α signal peptide | atggccttaccagtgaccgccttgctcctgccgctggccttgctgctccacgccgccaggccg |
| 3 | Human CD8α hinge region | Accacgacgccagcgccgcgaccaccaacaccggcgcccaccatcgcgtcgcagcccctgtccctgcgcccagaggcgtgcc gccagcggcgggggcgcagtgcacacgagggggctggacttcgcctgtgat |
| 4 | human CD28 intracellular domain | Aggagtaagaggagcaggctcctgcacagtgactacatgaacatgactccccgccgccccgggccaacccgcaagcattacc gccctatgccccaccacgcgacttcgcagcctatcgctcc |
| 5 | human CD3 ζ intracellular domain | Agagtgaagttcagcaggagcgcagacgcccccgcgtaccagcagggccagaaccagctctataacgagctcaatctaggac Aagagaggagtacgatgttttggacaagagacgtggccgggaccctgagatggggggaaagccgcagagaaggaagaaccct Aggaaggcctgtacaatgaactgcagaaagataagatggcggaggcctacagtgagattgggatgaaaggcgagcgccggag Ggcaaggggcacgatggcctttaccagggtctcagtacagccaccaaggacacctacgacgcccttcacatgcaggccctgc ccctcgc |
| 6 | Human CD28 transmembrane domain | ttttgggtgctggtggtggttggtggagtcctggcttgctatagcttgctagtaacagtggcctttattattttctgggtg |
| 7 | NFAT6 binding motif | Ggaggaaaaactgtttcatacagaaggcgtggaggaaaaactgtttcatacagaaggcgtggaggaaaaactgtttcataca Gaaggcgtcaattgtcctcgacggaggaaaaactgtttcatacagaaggcgtggaggaaaaactgtttcatacagaaggcgt ggaggaaaaactgtttcatacagaaggcgt |
| 8 | IL2 minimal promoter | Acattttgacacccccataatatttttccagaattaacagtataaattgcatctcttgttcaagagttccctatcactctct ttaatcactactcacagtaacctcaactcctg |
| 9 | (G₄S)₃ linker | ggtggaggcggttcaggcggaggtggttctggcggtggcggatcg |
| 10 | IL12 signal peptide and IL12p40 | Atgtgtcaccagcagttggtcatctcttggttttccctggtttttctggcatctcccctcgtggccatatgggaactgaaga Aagatgtttatgtcgtagaattggattggtatccggatgcccctggagaaatggtggtcctcacctgtgacacccctgaaga Agatggtatcacctggaccttggaccagagcagtgaggtcttaggctctggcaaaaccctgaccatccaagtcaaagagttt Ggagatgctggccagtacacctgtcacaaaggaggcgaggttctaagccattcgctcctgctgcttcacaaaaaggaagatg Gaatttggtccactgatattttaaaggaccagaaagaacccaaaaataagacctttctaagatgcgaggccaagaattattc Tggacgtttcacctgctggtggctgacgacaatcagtactgatttgacattcagtgtcaaaagcagcagaggctcttctgac Ccccaaggggtgacgtgcggagctgctacactctctgcagagagagtcagaggggacaacaaggagtatgagtactcagtgg Agtgccaggaggacagtgcctgcccagctgctgaggagagtctgcccattgaggtcatggtggatgccgttcacaagctcaa Gtatgaaaactacaccagcagcttcttcatcagggacatcatcaaacctgacccacccaagaacttgcagctgaagccatta Aagaattctcggcaggtggaggtcagctgggagtaccctgacacctggagtactccacattcctacttctccctgacattct Gcgttcaggtccagggcaagagcaagagagaaaagaaagatagagtcttcacggacaagacctcagccacggtcatctgccg caaaaatgccagcattagcgtgcgggcccaggaccgctactatagctcatcttggagcgaatgggcatctgtgccctgcagt |
| 11 | IL12p35 | Agaaacctccccgtggccactccagacccaggaatgttcccatgccttcaccactcccaaaacctgctgagggccgtcagc Aacatgctccagaaggccagacaaactctagaattttacccttgcacttctgaagagattgatcatgaagatatcacaaaa Gataaaaccagcacagtggaggcctgtttaccattggaattaaccaagaatgagagttgcctaaattccagagagacctct Ttcataactaatgggagttgcctggcctccagaaagacctcttttatgatggccctgtgccttagtagtatttatgaagac ttgaagatgtaccaggtggagttcaagaccatgaatgcaaagcttctgatggatcctaagaggcagatctttctagatcaa aacatgctggcagttattgatgagctgatgcaggccctgaatttcaacagtgagactgtgccacaaaaatcctcccttgaa gaaccggatttttataaaactaaaatcaagctctgcatacttcttcatgctttcagaattcgggcagtgactattgataga gtgatgagctatctgaatgcttcctaa |
| 12 | PA2 | aataaaatatctttattttcattacatctgtgtgttggttttttgtgtgag |
| 13 | TRAC-gRNA-1 | GAGTCTCTCAGCTGGTACA |
| 14 | TRAC-gRNA-2 | TGTGCTAGACATGAGGTCTA |
| 15 | TRAC-gRNA-1-IVT-template | TAATACGACTCACTATAGAGAGTCTCTCAGCTGGTACAGTTTTAGAGCTAGAAATAGCAAGTTAAAATAAGGCTAGTCCG TTATCAACTTGAAAAAGTGGCACCGAGTCGGTGCTTTTTTT |
| 16 | TRAC-gRNA-2-IVT-template | TAATACGACTCACTATAGTGTGCTAGACATGAGGTCTAGTTTTAGAGCTAGAAATAGCAAGTTAAAATAAGGCTAGTCC GTTATCAACTTGAAAAAGTGGCACCGAGTCGGTGCTTTTTTT |
| 17 | TRAC-gRNA-1-MluI-overlapping-forward | CCGACGCGTTAATACGACTCACTATAGAGAGTCTCTCAGCTGGTACAGTTTTAGAGCTAGAAATAGCAAGTTAAAATAAG |
| 18 | TRAC-gRNA-1-MluI-overlapping-forward | CCGACGCGTTAATACGACTCACTATAGTGTGCTAGACATGAGGTCTAGTTTTAGAGCTAGAAATAGCAAGTTAAAATAAG |
| 19 | All-gRNA-SalI-overlapping-reverse | ACGCGTCGACAAAAAAAGCACCGACTCGGTGCCACTTTTTCAAGTTGATAACGGACTAGCCTTATTTTAACTTGCTATTTC |
| 20 | All-gRNA-MOF | CCGACGCGTTAATACGACT |
| 21 | All-gRNA-SOR | ACGCGTCGACAAAAAAAGC |
| 22 | TRAC-F | TCATGTCCTAACCCTGATCCTCTT |
| 23 | TRAC-R | TTGGACTTTTCCCAGCT GACAGA |
| 24 | GPC3-28Z | EVQLVQSGAEVKKPGASVKVSCKASGYTFSDYEMHWVRQAPGQGLEWMGAIHPGSGDTAYNQRFKGRVTITADKSTSTA YMELSSLRSEDTAVYYCARFYSYAYWGQGTLVTVSAGGGGSGGGGSGGGGSDIVMTQTPLSLPVTPGEPASISCRSSQS LVHSNGNTYLQWYLQKPGQSPQLLIYKVSNRFSGVPDRFSGSGSGTDFTLKISRVEAEDVGVYYCSQSIYVPYTFGQGT KLEIKRTTTPAPRPPTPAPTIASQPLSLRPEACRPAAGGAVHTRGLDFACDFWVLVVVGGVLACYSLLVTVAFIIFWVR SKRSRLLHSDYMNMTPRRPGPTRKHYQPYAPPRDFAAYRSRVKFSRSADAPAYQQGQNQLYNELNLGRREEYDVLDKRR GRDPEMGGKPQRRKNPQEGLYNELQKDKMAEAYSEIGMKGERRRGKGHDGLYQGLSTATKDTYDALHMQALPPR |
| 25 | GPC3-BBZ | EVQLVQSGAEVKKPGASVKVSCKASGYTFSDYEMHWVRQAPGQGLEWMGAIHPGSGDTAYNQRFKGRVTITADKSTSTA YMELSSLRSEDTAVYYCARFYSYAYWGQGTLVTVSAGGGGSGGGGSGGGGSDIVMTQTPLSLPVTPGEPASISCRSSQS LVHSNGNTYLQWYLQKPGQSPQLLIYKVSNRFSGVPDRFSGSGSGTDFTLKISRVEAEDVGVYYCSQSIYVPYTFGQGT KLEIKRTTTPAPRPPTPAPTIASQPLSLRPEACRPAAGGAVHTRGLDFACDIYIWAPLAGTCGVLLLSLVITLYCKRGR KKLLYIFKQPFMRPVQTTQEEDGCSCRFPEEEEGGCELRVKFSRSADAPAYQQGQNQLYNELNLGRREEYDVLDKRRGR DPEMGGKPQRRKNPQEGLYNELQKDKMAEAYSEIGMKGERRRGKGHDGLYQGLSTATKDTYDALHMQALPPR |
| 26 | GPC3-28BBZ | EVQLVQSGAEVKKPGASVKVSCKASGYTFSDYEMHWVRQAPGQGLEWMGAIHPGSGDTAYNQRFKGRVTITADKSTSTA YMELSSLRSEDTAVYYCARFYSYAYWGQGTLVTVSAGGGGSGGGGSGGGGSDIVMTQTPLSLPVTPGEPASISCRSSQS LVHSNGNTYLQWYLQKPGQSPQLLIYKVSNRFSGVPDRFSGSGSGTDFTLKISRVEAEDVGVYYCSQSIYVPYTFGQGT KLEIKRTTTPAPRPPTPAPTIASQPLSLRPEACRPAAGGAVHTRGLDFACDFWVLVVVGGVLACYSLLVTVAFIIFWVR SKRSRLLHSDYMNMTPRRPGPTRKHYQPYAPPRDFAAYRSKRGRKKLLYIFKQPFMRPVQTTQEEDGCSCRFPEEEEGG CELRVKFSRSADAPAYQQGQNQLYNELNLGRREEYDVLDKRRGRDPEMGGKPQRRKNPQEGLYNELQKDKMAEAYSEIG MKGERRRGKGHDGLYQGLSTATKDTYDALHMQALPPR |
| 27 | GPC3-z | EVQLVQSGAEVKKPGASVKVSCKASGYTFSDYEMHWVRQAPGQGLEWMGAIHPGSGDTAYNQRFKGRVTITADKSTSTAY MELSSLRSEDTAVYYCARFYSYAYWGQGTLVTVSAGGGGSGGGGSGGGGSDIVMTQTPLSLPVTPGEPASISCRSSQSLV HSNGNTYLQWYLQKPGQSPQLLIYKVSNRFSGVPDRFSGSGSGTDFTLKISRVEAEDVGVYYCSQSIYVPYTFGQGTKLE IKRrvkfsrsadapayqqgqnqlynelnlgrreeydvldkrrgrdpemggkpqrrknpqeglynelqkdkmaeayseigm kgerrrgkghdglyqglstatkdtydalhmqalppr |
| 28 | GPC3-scFv | EVQLVQSGAEVKKPGASVKVSCKASGYTFSDYEMHWVRQAPGQGLEWMGAIHPGSGDTAYNQRFKGRVTITADKSTSTAY MELSSLRSEDTAVYYCARFYSYAYWGQGTLVTVSAGGGGSGGGGSGGGGSDIVMTQTPLSLPVTPGEPASISCRSSQSLV HSNGNTYLQWYLQKPGQSPQLLIYKVSNRFSGVPDRFSGSGSGTDFTLKISRVEAEDVGVYYCSQSIYVPYTFGQGTKLE IKR |
| 29 | 85-2-28Z | Qvqlqesgpglikpsqtlsltctvsggsissgynwhwirqppgkglewigyihytgstnynpalrsrvtisvdtsknqfs Lklssvtaadtaiyycariyngnsfpywgqgttvtvssggggsggggsggggsdivmtqspdslavslgeratinckssq Slfnsgnqknyltwyqqkpgqppklliywastresgvpdrfsgsgsgtdftltisslqaedvavyycqnaysfpytfggg tkleikrTttpaprpptpaptiasqplslrpeacrpaaggavhtrgldfacdFwvlvvvggvlacysllvtvafiifwvR skrsrllhsdymnmtprrpgptrkhyqpyapprdfaayrsrvkfsrsadapayqqgqnqlynelnlgrreeydvldkrrg rdpemggkpqrrknpqeglynelqkdkmaeayseigmkgerrrgkghdglyqglstatkdtydalhmqalppr |
| 30 | 85-28z | Qvqlqesgpglvkpsqtlsltctvsggsissgynwhwirqppgkglewigyihytgstnynpalrsrvtisvdtsknqfs Lklssvtaadtavyycariyngnsfpywgqgttvtvssggggsggggsggggsdivmtqspdslavslgeratinckssq Slfnsgnqknyltwyqqkpgqppklliywastresgvpdrfsgsgsgtdftltisslqaedvavyycqnaysfpytfggg tkleikrTttpaprpptpaptiasqplslrpeacrpaaggavhtrgldfacdFwvlvvvggvlacysllvtvafiifwvR skrsrllhsdymnmtprrpgptrkhyqpyapprdfaayrsrvkfsrsadapayqqgqnqlynelnlgrreeydvldkrrg rdpemggkpqrrknpqeglynelqkdkmaeayseigmkgerrrgkghdglyqglstatkdtydalhmqalppr |
| 31 | 22-28Z | DIQMTQSPSSLSASVGDRVTITCHASQDINVNIGWLQQKPGKAFKGLIYHGKNLEDGVPSRFSGSGSGTDFTLTISSLQP EDFATYYCNQYENIPLTFGQGTKVEIKRGGGGSGGGGSGGGGSDVQLVESGGGLVQPGGSLRLSCAVSGYSITSDYAWNW IRQAPGKGLEWLGYISYRGRTQYNPSLKSRISITRDNSKNTFFLQLNSLRAEDTAVYYCARMGKNWDYWGQGTLVTVSST ttpaprpptpaptiasqplslrpeacrpaaggavhtrgldfacdFwvlvvvggvlacysllvtvafiifwvRskrsrllh sdymnmtprrpgptrkhyqpyapprdfaayrsrvkfsrsadapayqqgqnqlynelnlgrreeydvldkrrgrdpemggk pqrrknpqeglynelqkdkmaeayseigmkgerrrgkghdglyqglstatkdtydalhmqalppr |
| 32 | 22-BBZ | DIQMTQSPSSLSASVGDRVTITCHASQDINVNIGWLQQKPGKAFKGLIYHGKNLEDGVPSRFSGSGSGTDFTLTISSLQP EDFATYYCNQYENIPLTFGQGTKVEIKRGGGGSGGGGSGGGGSDVQLVESGGGLVQPGGSLRLSCAVSGYSITSDYAWNW IRQAPGKGLEWLGYISYRGRTQYNPSLKSRISITRDNSKNTFFLQLNSLRAEDTAVYYCARMGKNWDYWGQGTLVTVSST TTPAPRPPTPAPTIASQPLSLRPEACRPAAGGAVHTRGLDFACDIYIWAPLAGTCGVLLLSLVITLYCKRGRKKLLYIFK QPFMRPVQTTQEEDGCSCRFPEEEEGGCELRVKFSRSADAPAYKQGQNQLYNELNLGRREEYDVLDKRRGRDPEMGGKPR RKNPQEGLYNELQKDKMAEAYSEIGMKGERRRGKGHDGLYQGLSTATKDTYDALHMQALPPR |
| 33 | 22-28BBZ | DIQMTQSPSSLSASVGDRVTITCHASQDINVNIGWLQQKPGKAFKGLIYHGKNLEDGVPSRFSGSGSGTDFTLTISSLQP EDFATYYCNQYENIPLTFGQGTKVEIKRGGGGSGGGGSGGGGSDVQLVESGGGLVQPGGSLRLSCAVSGYSITSDYAWNW IRQAPGKGLEWLGYISYRGRTQYNPSLKSRISITRDNSKNTFFLQLNSLRAEDTAVYYCARMGKNWDYWGQGTLVTVSST ttpaprpptpaptiasqplslrpeacrpaaggavhtrgldfacdFwvlvvvggvlacysllvtvafiifwvRskrsrllh sdymnmtprrpgptrkhyqpyapprdfaayrsKrgrkkllyifkqpfmrpvqttqeedgcscrfpeeeeggcelrvkfsr sadapayqqgqnqlynelnlgrreeydvldkrrgrdpemggkpqrrknpqeglynelqkdkmaeayseigmkgerrrgkg hdglyqglstatkdtydalhmqalppr |
| 34 | CD19-28Z | Qvqlvqsgaevkkpgssvkvsckasgytfssywmnwvrqapgqglewmgqiwpgdgdtnyngkfkgrvtitadeststay Melsslrsedtavyycarretttvgryyyamdywgqgttvtvssggggsggggsggggsdivltqspaslavspgqrati Tckasqsvdydgdsylnwyqqkpgqppklliydasnlvsgvparfsgsgsgtdftltinpveandtanyycqqstedpwt Fgqgtkveikrtttpaprpptpaptiasqplslrpeacrpaaggavhtrgldfacdfwvlvvvggvlacysllvtvafii Fwvrskrsrllhsdymnmtprrpgptrkhyqpyapprdfaayrsrvkfsrsadapayqqgqnqlynelnlgrreeydvld krrgrdpemggkpqrrknpqeglynelqkdkmaeayseigmkgerrrgkghdglyqglstatkdtydalhmqalppr |
| 35 | 33-28Z | Dvvmtqsplslpvtpgepasiscrssqslvhsnantylhwylqkpgqspqlliykvsnrfsgvpdrfsgsgsgtdftlkis Rveaedvgvyycsqnthvpptfgqgtkleikrggggsggggsggggsqvqlvqsgaevkkpgasvkvsckasgytftdyem Hwvrqapgqglewmgaldpktgdtaysqkfkgrvtltadeststaymelsslrsedtavyyctrfysytywgqgtlvtvss TttpaprpptpaptiasqplslrpeacrpaaggavhtrgldfacdFwvlvvvggvlacysllvtvafiifwvRskrsrllh Sdymnmtprrpgptrkhyqpyapprdfaayrsrvkfsrsadapayqqgqnqlynelnlgrreeydvldkrrgrdpemggkp qrrknpqeglynelqkdkmaeayseigmkgerrrgkghdglyqglstatkdtydalhmqalppr |
| 36 | 33-28BBZ | Dvvmtqsplslpvtpgepasiscrssqslvhsnantylhwylqkpgqspqlliykvsnrfsgvpdrfsgsgsgtdftlkis Rveaedvgvyycsqnthvpptfgqgtkleikrggggsggggsggggsqvqlvqsgaevkkpgasvkvsckasgytftdyem Hwvrqapgqglewmgaldpktgdtaysqkfkgrvtltadeststaymelsslrsedtavyyctrfysytywgqgtlvtvss TttpaprpptpaptiasqplslrpeacrpaaggavhtrgldfacdFwvlvvvggvlacysllvtvafiifwvRskrsrllh sdymnmtprrpgptrkhyqpyapprdfaayrsKrgrkkllyifkqpfmrpvqttqeedgcscrfpeeeeggcelrvkfsrs adapayqqgqnqlynelnlgrreeydvldkrrgrdpemggkpqrrknpqeglynelqkdkmaeayseigmkgerrrgkghd glyqglstatkdtydalhmqalppr |
| 37 | 163-28BBZ | Qiqlvqsgpelkkpgetvkisckasgytftnygmnwvkqapgkglkwmgwintntgeptyaeefkgrfafsletsastayl Qinnlknedtatyfcarlgfgnamdywgqgtsvtvssggggsggggsggggsdivmtqspssltvtagekvtmscksssqsl Lnsgnqknyltwyqqkpgqppklliywastresgvpdrftgsgsgtdftltissvqaedlavyycqndysypltfgagtkl elkrTTTPAPRPPTPAPTIASQPLSLRPEACRPAAGGAVHTRGLDFACDFWVLVVVGGVLACYSLLVTVAFIIFWVRSKRS RLLHSDYMNMTPRRPGPTRKHYQPYAPPRDFAAYRSKRGRKKLLYIFKQPFMRPVQTTQEEDGCSCRFPEEEEGGCELRVK |
| | | FSRSADAPAYQQGQNQLYNELNLGRREEYDVLDKRRGRDPEMGGKPQRRKNPQEGLYNELQKDKMAEAYSEIGMKGERRRG KGHDGLYQGLSTATKDTYDALHMQALPPR |
| 38 | 175-28BBZ | Qvqlqqpgaelvrpgasvklsckasgytftsywinwvkqrpgqglewigniypsdsytnynqkfkdkatltvdkssstaymq Lssptsedsavyyctrswrgnsfdywgqgttltvssggggsggggsggggsdivmtqspssltvtagekvtmsckssqslln Sgnqknyltwyqqkpgqppklliywastresgvpdrftgsgsgtdftltissvqaedlavyycqndysypftfgsgtkleik rTTTPAPRPPTPAPTIASQPLSLRPEACRPAAGGAVHTRGLDFACDFWVLVVVGGVLACYSLLVTVAFIIFWVRSKRSRLLH SDYMNMTPRRPGPTRKHYQPYAPPRDFAAYRSKRGRKKLLYIFKQPFMRPVQTTQEEDGCSCRFPEEEEGGCELRVKFSRSA DAPAYQQGQNQLYNELNLGRREEYDVLDKRRGRDPEMGGKPQRRKNPQEGLYNELQKDKMAEAYSEIGMKGERRRGKGHDGL YQGLSTATKDTYDALHMQALPPR |
| 39 | 16-28BBZ | QVQLEQSGLGLVKPSQTLSLTCAISGDTVSSDSAAWNWIRQSPSRGLEWLGRTYYRSKWFNDYAVSVKGRITINSDTSKNQF SLQLNSVTPEDTAVYYCARSNSYYYYAMDVWGQGTLVTVSSGGGGSGGGGSGGGGSQAVLTQPSSLSASPGASASLTCTLRS GINVGIYRIYWYQQRPGSPPQILLTYKSDSDKYQGSGVPSRFSGSKDASANAGILLISGLQSEDEADYYCMIWHSGGWVFGG GTKVTVLGTTTPAPRPPTPAPTIASQPLSLRPEACRPAAGGAVHTRGLDFACDFWVLVVVGGVLACYSLLVTVAFIIFWVRS KRSRLLHSDYMNMTPRRPGPTRKHYQPYAPPRDFAAYRSKRGRKKLLYIFKQPFMRPVQTTQEEDGCSCRFPEEEEGGCELR VKFSRSADAPAYQQGQNQLYNELNLGRREEYDVLDKRRGRDPEMGGKPQRRKNPQEGLYNELQKDKMAEAYSEIGMKGERRR GKGHDGLYQGLSTATKDTYDALHMQALPPR |
| 40 | 32-28Z | QMQLVQSGAEVKKPGASVKVSCKASGYTFTSYYMHWVRQAPGQGLEWMGIINPSGGSTSYAQKFQGRVTMTRDTSTSTVYME LSSLRSEDTAVYYCASSRSGTTVVNHDAFDIWGKGTTVTVSSGGGGSGGGGSGGGGSDIQLTQSPSSLSASVGDRVTITCRA SQVISRALAWYQQTPGKPPKLLIYDASNLQSGVPSRFSGSGSGTDFTLTISRLQPEDFATYYCQQFNSYPLTFGGGTKLEIK RTTTPAPRPPTPAPTIASQPLSLRPEACRPAAGGAVHTRGLDFACDFWVLVVVGGVLACYSLLVTVAFIIFWVRSKRSRLLH SDYMNMTPRRPGPTRKHYQPYAPPRDFAAYRSRVKFSRSADAPAYQQGQNQLYNELNLGRREEYDVLDKRRGRDPEMGGKPQ RRKNPQEGLYNELQKDKMAEAYSEIGMKGERRRGKGHDGLYQGLSTATKDTYDALHMQALPPR |
| 41 | 32-BBZ | QMQLVQSGAEVKKPGASVKVSCKASGYTFTSYYMHWVRQAPGQGLEWMGIINPSGGSTSYAQKFQGRVTMTRDTSTSTVYME LSSLRSEDTAVYYCASSRSGTTVVNHDAFDIWGKGTTVTVSSGGGGSGGGGSGGGGSDIQLTQSPSSLSASVGDRVTITCRA SQVISRALAWYQQTPGKPPKLLIYDASNLQSGVPSRFSGSGSGTDFTLTISRLQPEDFATYYCQQFNSYPLTFGGGTKLEIK RTTTPAPRPPTPAPTIASQPLSLRPEACRPAAGGAVHTRGLDFACDIYIWAPLAGTCGVLLLSLVITLYCKRGRKKLLYIFK QPFMRPVQTTQEEDGCSCRFPEEEEGGCELRVKFSRSADAPAYKQGQNQLYNELNLGRREEYDVLDKRRGRDPEMGGKPRRK NPQEGLYNELQKDKMAEAYSEIGMKGERRRGKGHDGLYQGLSTATKDTYDALHMQALPPR |
| 42 | 32-28BBZ | QMQLVQSGAEVKKPGASVKVSCKASGYTFTSYYMHWVRQAPGQGLEWMGIINPSGGSTSYAQKFQGRVTMTRDTSTSTVYME LSSLRSEDTAVYYCASSRSGTTVVNHDAFDIWGKGTTVTVSSGGGGSGGGGSGGGGSDIQLTQSPSSLSASVGDRVTITCRA SQVISRALAWYQQTPGKPPKLLIYDASNLQSGVPSRFSGSGSGTDFTLTISRLQPEDFATYYCQQFNSYPLTFGGGTKLEIK RTTTPAPRPPTPAPTIASQPLSLRPEACRPAAGGAVHTRGLDFACDFWVLVVVGGVLACYSLLVTVAFIIFWVRSKRSRLLH SDYMNMTPRRPGPTRKHYQPYAPPRDFAAYRSKRGRKKLLYIFKQPFMRPVQTTQEEDGCSCRFPEEEEGGCELRVKFSRSA DAPAYQQGQNQLYNELNLGRREEYDVLDKRRGRDPEMGGKPQRRKNPQEGLYNELQKDKMAEAYSEIGMKGERRRGKGHDGL YQGLSTATKDTYDALHMQALPPR |
| 43 | EGFR-CAR-28z | DILMTQSPSSMSVSLGDTVSITCHSSQDINSNIGWLQQRPGKSFKGLIYHGTNLDDEVPSRFSGSGSGADYSLTISSLESED FADYYCVQYAQFPWTFGGGTKLEIKRGGGGSGGGGSGGGGSADVQLQESGPSLVKPSQSLSLTCTVTGYSITSDFAWNWIRQ FPGNKLEWMGYISYSGNTRYNPSLKSRISITRDTSKNQFFLQLNSVTIEDTATYYCVTAGRGFPYWGQGTLVTVSATttpap rpptpaptiasqplslrpeacrpaaggavhtrgldfacdFwvlvvvggvlacysllvtvafiifwvRskrsrllhsdymnmt prrpgptrkhyqpyapprdfaayrsrvkfsrsadapayqqgqnqlynelnlgrreeydvldkrrgrdpemggkpqrrknpqe glynelqkdkmaeayseigmkgerrrgkghdglyqglstatkdtydalhmqalppr |
| 44 | FLT3L, amino acid sequence | Mtvlapawspttylllllllssglsgtqdcsfqhspissdfavkirelsdyllqdypvtvasnlqdeelcgglwrlvlaq Rwmerlktvagskmqgllervnteihfvtkcafqpppsclrfvqtnisrllqetseqlvalkpwitrqnfsrclelqcqp dsstlpppwsprpleataptapqppllllllpvglllllaaawclhwqrtrrrtprpgeqvppvpspqdlllveh |
| 45 | Human IL12, amino acid sequence | Mchqqlviswfslvflasplvaiwelkkdvyvveldwypdapgemvvltcdtpeedgitwtldqssevlgsgktltiqvke Fgdagqytchkggevlshslllllhkkedgiwstdilkdqkepknktflrceaknysgrftcwwlttistdltfsvkssrgs Sdpqgvtcgaatlsaervrgdnkeyeysvecqedsacpaaeeslpievmvdavhklkyenytssffirdiikpdppknlql Kplknsrqvevsweypdtwstphsyfsltfcvqvqgkskrekkdrvftdktsatvicrknasisvraqdryysssswsewas Vpcsgggggsgggsgggsrnlpvatpdpgmfpclhhsqnllravsnmlqkarqtlefypctseeidheditkdktstvea Clpleltknesclnsretsfitngsclasrktsfmmalclssiyedlkmyqvefktmnakllmdpkrqifldqnmlavide lmqalnfnsetvpqkssleepdfyktkiklcillhafriravtidrvmsylnas* |
| 46 | IFN β | MTNKCLLQIALLLCFSTTALSMSYNLLGFLQRSSNFQCQKLLWQLNGRLEYCLKDRMNFDIPEEIKQLQQFQKEDAALTIY EMLQNIFAIFRQDSSSTGWNETIVENLLANVYHQINHLKTVLEEKLEKEDFTRGKLMSSLHLKRYYGRILHYLKAKEYSHC AWTIVRVEILRNFYFINRLTGYLRN |

All documents mentioned in the present invention are incorporated by reference in this application, as if each document was individually incorporated by reference. In addition, it should be understood that after reading the above teaching content of the present invention, those skilled in the art can make various changes or modifications to the present invention, and these equivalent forms also fall within the scope defined by the claims appended to this application.

## Claims

1. A genetically engineered T cell, wherein the endogenous T cell receptor (TCR) in said T cell is inactivated or inactive; and
said T cell comprises an open reading frame, the open reading frame encodes an exogenous receptor capable of binding to a target antigen and is regulated by the exogenous receptor, and when the exogenous receptor binds to the target antigen, the expression of the open reading frame can be initiated.

2. The T cell of claim 1, wherein the open reading frame is regulated by the exogenous receptor through a promoter.

3. The T cell of claim 2, wherein the exogenous receptor can trigger the activation of CD3 signal, and the promoter activity is regulated by TCR signal activation.

4. The T cell of claim 2, wherein the T cell comprises the nucleic acid of an open reading frame operably linked to the promoter, the promoter activity is regulated by the activation of TCR signal, the exogenous receptor can trigger the activation of CD3 signal, and when the exogenous receptor binds to the target antigen, the promoter regulates the expression of the open reading frame.

5. The T cell of claim 1, wherein the T cell does not express an endogenous T cell receptor (TCR).

6. The T cell of claim 1, wherein the endogenous TCR is treated by gene knockout technology or gene silencing technology, so that the endogenous TCR is inactive;
preferably, the endogenous TCR gene is knocked out by gene site-directed knockout technology, the gene site-directed knockout technology includes CRISPR / Cas9 technology, Zinc Finger Nucleases (ZFN) technology, transcription activator-like effector (TALE) technology, or TALE-CRISPR / Cas9 technology;
and more preferably, CRISPR / Cas9 technology is used.

7. The T cell of claim 6, wherein the gene knockout or gene silencing is performed in the constant region of one or both of the α and β chains of the TCR;
preferably, the exon of the corresponding encoding gene in the constant region of one or both of the α and β chains of the TCR is subjected to gene knockout or gene silencing.

8. The T cell of claim 7, wherein gene knockout or gene silencing is performed in the constant region of the α chain of the TCR,
preferably, the first exon in the constant region of the endogenous TCRα chain is subjected to gene knockout or gene silencing.

9. The T cell of any one of claims 2-4, wherein the promoter comprises a binding motif of a transcription factor that depends on the activation of TCR signal.

10. The T cell of claim 9, wherein the promoter comprises a minimal promoter operably linked to a binding motif of a transcription factor that depends on the activation of TCR signal.

11. The T cell of claim 10, wherein the minimal promoter is a minimal promoter of cytokine, including a minimal promoter of interleukin, interferon, tumor necrosis factor superfamily, colony stimulating factor, chemokine, and growth factor; preferably minimal promoter of IFN -γ, TNF-α, or IL-2, more preferably minimal promoter of IL-2.

12. The T cell of any one of claims 9-11, wherein the binding motif includes a NFAT, NF-κB or AP-1 binding motif or a combination thereof; preferably a NFAT binding motif, more preferably two or more NFAT binding motifs; most preferably 6 NFAT-binding motifs.

13. The T cell of any one of claims 9-12, wherein the minimal promoter is encoded by a nucleotide sequence having 90% identity with SEQ ID NO: 8.

14. The T cell of any one of claims 1-13, wherein the open reading frame includes an open reading frame of cytokine, immunotoxin, cytotoxic protein, antibody drug, or bifunctional antibody;
the cytokine is preferably IFN-α, IFN-β, IFN -γ; Interleukin 2, 3, 4, 5, 6, 8, 12, 13, 22, 23, 24; TNF-α, GM-CSF, CD40L, CTLA-4, FLT3L, TRAIL or LIGHT; and
more preferably, the cytokine is IL-12.

15. The T cell of any one of claims 1-13, wherein the exogenous receptor is selected from chimeric antigen receptor (CAR), modified T cell (antigen) receptor (TCR), T cell fusion protein (TFP), and T cell antigen coupler (TAC) or a combination thereof.

16. The T cell of claim 15, wherein the modified TCR is selected from wild TCR, high affinity TCR, or chimeric TCR.

17. The T cell of claim 15, wherein the chimeric antigen receptor comprises an antigen-binding domain, transmembrane domain and intracellular domain.

18. The T cell of claim 17, wherein the chimeric antigen receptor includes:
(i) an antibody that specifically binds to said antigen, a transmembrane region of CD28, a co-stimulatory signal domain of CD28, and a CD3ζ fusion peptide; or
(ii) an antibody that specifically binds to said antigen, a transmembrane region of CD28, a co-stimulatory signal domain of CD137, and a CD3ζ fusion peptide; or
(iii) an antibody that specifically binds to the antigen, a transmembrane region of CD28, a co-stimulatory signal domain of CD28, a co-stimulatory signal domain of CD137, and a CD3ζ fusion peptide.

19. The T cell of claim 15, wherein the TFP includes:
(a) a TCR subunit comprising:
Part of TCR extracellular domain, transmembrane domain, and TCR intracellular domain, and said intracellular domain includs a stimulatory signaling domain;
(b) an antibody domain having an antigen-binding domain;
wherein the TCR subunit is operably connected to the antibody domain, the extracellular, transmembrane, and intracellular signaling domains of the TCR subunit are derived from CD3ε or CD3γ, and the TFP is integrated into the TCR expressed on T cells.

20. The T cell of claim 15, wherein the TAC includes:
(a) an extracellular domain: the extracellular domain includes an antibody domain having an antigen-binding domain, and a single-chain antibody that binds to CD3;
(b) a transmembrane region;
(c) an intracellular domain connected to the protein kinase LCK.

21. The T cell of any one of claims 1-20, wherein the target antigen includes a tumor antigen or a pathogen antigen.

22. The T cell of any one of claims 2-20, wherein the nucleic acid of the exogenous receptor that can bind to the target antigen and trigger the activation of CD3 signal are linked togethe with the nucleic acid of the open reading frame operably connected to the promoterr directly or through a linker molecule.

23. The T cell of any one of claims 1-20, wherein the amino acid sequence of the exogenous receptor that can bind to the target antigen and trigger the activation of CD3 signal is a sequence having at least 90% identity with the sequence as shown in SEQ ID NO: 24, 25, 26, 27, 29, 30, 31, 32, 33, 34, 35, 36, 37, 38, 39, 40, 41, 42, or 43.

24. The T cell of any one of claims 2-22, wherein the nucleic acid of the open reading frame operably connected to the promoter is encoded by a nucleotide sequence having 90% identity with SEQ ID NO: 44, 45, or 46.

25. Use of the T cell of any one of claims 2-24 for preparing a pharmaceutical composition for treating a tumor, pathogen infection in an individual in need thereof, or enhancing the immune tolerance of an individual.

26. An expression construct, comprising: the expression cassette of the exogenous receptor that can bind to the target antigen and trigger the activation of CD3 signal of any one of the preceding claims linked together with the expression cassette of the open reading frame operably connected to the promoter directly or through a linker molecule.

27. A method for preparing the T cell of any one of claims 1-24, including following steps:
1) the endogenous TCR is treated by gene knockout technology or gene silencing technology, so that the endogenous TCR is inactive; preferably, the endogenous TCR gene is knocked out by gene site-directed knockout technology; the gene site-directed knockout technology includes CRISPR / Cas9 technology, Zinc Finger Nucleases (ZFN) technology, transcription activator-like effector (TALE) technology, or TALE-CRISPR / Cas9 technology; and more preferably, CRISPR / Cas9 technology is used;
2) the T cells obtained as said above are infected by a virus carrying a nucleic acid encoding an exogenous receptor capable of binding to a target antigen and a nucleic acid of an open reading frame regulated by the exogenous receptor, wherein when the exogenous receptor binds to the target antigen, the expression of the open reading frame can be initiated.

28. The method of claim 27, wherein the exon of the corresponding encoding gene in the constant region of one or both of the α and β chains of the TCR is subjected to gene site-directed knockout technology, so that the endogenous TCR is inactive, and preferably the first exon in the constant region of α chain of the endogenous TCR is site-directed knocked out.

29. The method of claim 27 or 28, wherein the T cells are derived from PBMC, umbilical cord blood cells, purified T cell population, T cell line, and / or genetically engineered T cells.

30. A method for treating tumors, pathogen infections in an individual in need thereof, or enhancing immune tolerance in an individual, comprising administering a therapeutically effective amount of the T cells of any one of claims 1-24, or administering a therapeutically effective amount of a pharmaceutical composition comprising the T cells of any one of claims 1-24.

31. The method of claim 30, wherein the subject is a human.

32. The use of claim 25, wherein the tumor includes leukemia (such as acute leukemia, acute lymphocytic leukemia, acute myeloid leukemia, acute myeloid leukemia, acute promyelocytic leukemia, acute myelo-monocytic leukemia, acute monocyte leukemia, acute leukemia, chronic leukemia, chronic myelogenous leukemia, chronic lymphocytic leukemia, polycythemia vera), lymphoma (Hodgkin's disease, non-Hodgkin's disease), primary macroglobulinemia, heavy chain disease, solid tumors such as sarcomas and cancers (such as fibrosarcoma, myxosarcoma, liposarcoma, chondrosarcoma, osteosarcoma, chordoma, endothelial sarcoma, lymphangiosarcoma, angiosarcoma, lymphangiothelioma, synovial tumor, mesothelioma, Ewing's tumor, leiomyosarcoma, rhabdomyosarcoma, colon cancer, pancreatic cancer, breast cancer, ovarian cancer, prostate cancer, squamous cell carcinoma, basal cell carcinoma, adenocarcinoma, sweat adenocarcinoma, sebaceous adenocarcinoma, papillary carcinoma, papillary adenocarcinoma, carcinoma, bronchial carcinoma, myeloid cancer, renal cell carcinoma, liver cancer, nile duct cancer, choriocarcinoma, seminal cell tumor, embryo cancer, nephroblastoma, cervical cancer, uterine cancer, testicular cancer, lung cancer, small cell lung cancer, bladder cancer, epithelial cancer, glue Plasma tumors, astrocytomas, medulloblastomas, craniopharyngiomas, ependymal tumors, pineal tumors, hemangioblastomas, acoustic neuromas, oligodendroglioma, schwannomas, meningiomas, melanoma, neuroblastoma, retinoblastoma), esophageal cancer, gallbladder cancer , kidney cancer, multiple myeloma; preferably, the "tumor" includes but is not limited to: pancreatic cancer, liver cancer, lung cancer, gastric cancer, esophageal cancer, head and neck squamous cell carcinoma, prostate cancer, colon cancer, breast cancer, lymphoma, gallbladder cancer, kidney cancer, leukemia, multiple myeloma, ovarian cancer, cervical cancer, and glioma, and any combination thereof; or
the pathogens include: viruses, bacteria, fungi, protozoa or parasites; preferably, the viruses include: cytomegalovirus, Epstein-Barr virus, human immunodeficiency virus or influenza virus.

33. A pharmaceutical composition, comprising: the T cell of any one of claims 1-24; and a pharmaceutically acceptable carrier or excipient.

34. A kit, comprising:
the T cell of any one of claims 1-24; and
an instruction on how to administer the immune effector cells to an individual.


Sequence listing

<110>  CARSGEN THERAPEUTICS CO., LTD.
       SHANGHAI CANCER INSTITUTE

<120>  GENETICALLY ENGINEERED T CELL AND APPLICATION THEREOF

<130>  BET 20M1075

<140>  EP 18854823.4
<141>  2018-09-07

<150>  CN 201710805991.4
<151>  2017-09-08

<150>  CN 201710867308.X
<151>  2017-09-22

<150>  CN 201711287300.2
<151>  2017-12-07

<160>  46

<170>  PatentIn version 3.5

<210>  1
<211>  729
<212>  DNA
<213>  Artificial sequence

<220>
<223>  GPC3 scFv

<400>  1
gaggtgcagc tggtgcagag cggcgccgag gtgaagaagc ccggcgccag cgtgaaggtg      60

agctgcaagg ccagcggcta caccttcagc gactacgaga tgcactgggt gcggcaggcc     120

cccggccagg gcctggagtg gatgggcgcc atccaccccg gcagcggcga caccgcctac     180

aaccagcggt tcaagggccg ggtgaccatc accgccgaca agagcaccag caccgcctac     240

atggagctga gcagcctgcg gagcgaggac accgccgtgt actactgcgc ccggttctac     300

agctacgcct actggggcca gggcaccctg gtgaccgtga gcgccggtgg aggcggttca     360

ggcggaggtg gttctggcgg tggcggatcg gacatcgtga tgacccagac ccccctgagc     420

ctgcccgtga cccccggcga gcccgccagc atcagctgcc ggagcagcca gagcctggtg     480

cacagcaacg gcaacaccta cctgcagtgg tacctgcaga agcccggcca gagcccccag     540

ctgctgatct acaaggtgag caaccggttc agcggcgtgc ccgaccggtt cagcggcagc     600

ggcagcggca ccgacttcac cctgaagatc agccgggtgg aggccgagga cgtgggcgtg     660

tactactgca gccagagcat ctacgtgccc tacaccttcg gccagggcac caagctggag     720

atcaaacgt                                                             729


<210>  2
<211>  63

<212>  DNA
<213>  Artificial sequence

<220>
<223>  human CD8alpha signal peptide

<400>  2
atggccttac cagtgaccgc cttgctcctg ccgctggcct tgctgctcca cgccgccagg      60

ccg                                                                    63


<210>  3
<211>  135
<212>  DNA
<213>  Artificial sequence

<220>
<223>  human CD8alpha hinge region

<400>  3
accacgacgc cagcgccgcg accaccaaca ccggcgccca ccatcgcgtc gcagcccctg      60

tccctgcgcc cagaggcgtg ccggccagcg gcggggggcg cagtgcacac gagggggctg     120

gacttcgcct gtgat                                                      135


<210>  4
<211>  123
<212>  DNA
<213>  Artificial sequence

<220>
<223>  human CD28 intracellular domain

<400>  4
aggagtaaga ggagcaggct cctgcacagt gactacatga acatgactcc ccgccgcccc      60

gggccaaccc gcaagcatta ccagccctat gccccaccac gcgacttcgc agcctatcgc     120

tcc                                                                   123


<210>  5
<211>  339
<212>  DNA
<213>  Artificial sequence

<220>
<223>  human CD3zeta intracellular domain

<400>  5
agagtgaagt tcagcaggag cgcagacgcc cccgcgtacc agcagggcca gaaccagctc      60

tataacgagc tcaatctagg acgaagagag gagtacgatg ttttggacaa gagacgtggc     120

cgggaccctg agatgggggg aaagccgcag agaaggaaga accctcagga aggcctgtac     180

aatgaactgc agaaagataa gatggcggag gcctacagtg agattgggat gaaaggcgag     240

cgccggaggg gcaaggggca cgatggcctt taccagggtc tcagtacagc caccaaggac     300

acctacgacg cccttcacat gcaggccctg ccccctcgc 339

<210> 6
<211> 81
<212> DNA
<213> Artificial sequence

<220>
<223> human CD28 transmembrane domain

<400> 6
ttttgggtgc tggtggtggt tggtggagtc ctggcttgct atagcttgct agtaacagtg 60

gcctttatta ttttctgggt g 81

<210> 7
<211> 194
<212> DNA
<213> Artificial sequence

<220>
<223> NFAT6 binding motif

<400> 7
ggaggaaaaa ctgtttcata cagaaggcgt ggaggaaaaa ctgtttcata cagaaggcgt 60

ggaggaaaaa ctgtttcata cagaaggcgt caattgtcct cgacggagga aaaactgttt 120

catacagaag gcgtggagga aaaactgttt catacagaag gcgtggagga aaaactgttt 180

catacagaag gcgt 194

<210> 8
<211> 114
<212> DNA
<213> Artificial sequence

<220>
<223> IL2 minimal promoter

<400> 8
acattttgac acccccataa tatttttcca gaattaacag tataaattgc atctcttgtt 60

caagagttcc ctatcactct ctttaatcac tactcacagt aacctcaact cctg 114

<210> 9
<211> 45
<212> DNA
<213> Artificial sequence

<220>
<223> (G4S)3 linker

<400> 9
ggtggaggcg gttcaggcgg aggtggttct ggcggtggcg gatcg 45

<210> 10
<211> 984

<212>  DNA
<213>  Artificial sequence

<220>
<223>  IL12 signal peptide and IL12p40

<400>  10
atgtgtcacc agcagttggt catctcttgg ttttccctgg tttttctggc atctcccctc      60

gtggccatat gggaactgaa gaaagatgtt tatgtcgtag aattggattg gtatccggat     120

gcccctggag aaatggtggt cctcacctgt gacacccctg aagaagatgg tatcacctgg     180

accttggacc agagcagtga ggtcttaggc tctggcaaaa ccctgaccat ccaagtcaaa     240

gagtttggag atgctggcca gtacacctgt cacaaaggag gcgaggttct aagccattcg     300

ctcctgctgc ttcacaaaaa ggaagatgga atttggtcca ctgatatttt aaaggaccag     360

aaagaaccca aaaataagac ctttctaaga tgcgaggcca agaattattc tggacgtttc     420

acctgctggt ggctgacgac aatcagtact gatttgacat tcagtgtcaa aagcagcaga     480

ggctcttctg acccccaagg ggtgacgtgc ggagctgcta cactctctgc agagagagtc     540

agaggggaca acaaggagta tgagtactca gtggagtgcc aggaggacag tgcctgccca     600

gctgctgagg agagtctgcc cattgaggtc atggtggatg ccgttcacaa gctcaagtat     660

gaaaactaca ccagcagctt cttcatcagg gacatcatca aacctgaccc acccaagaac     720

ttgcagctga agccattaaa gaattctcgg caggtggagg tcagctggga gtaccctgac     780

acctggagta ctccacattc ctacttctcc ctgacattct gcgttcaggt ccagggcaag     840

agcaagagag aaaagaaaga tagagtcttc acggacaaga cctcagccac ggtcatctgc     900

cgcaaaaatg ccagcattag cgtgcgggcc caggaccgct actatagctc atcttggagc     960

gaatgggcat ctgtgccctg cagt                                            984


<210>  11
<211>  594
<212>  DNA
<213>  Artificial sequence

<220>
<223>  IL12p35

<400>  11
agaaacctcc ccgtggccac tccagaccca ggaatgttcc catgccttca ccactcccaa      60

aacctgctga gggccgtcag caacatgctc cagaaggcca gacaaactct agaattttac     120

ccttgcactt ctgaagagat tgatcatgaa gatatcacaa aagataaaac cagcacagtg     180

gaggcctgtt taccattgga attaaccaag aatgagagtt gcctaaattc cagagagacc     240

tctttcataa ctaatgggag ttgcctggcc tccagaaaga cctcttttat gatggccctg     300

tgccttagta gtatttatga agacttgaag atgtaccagg tggagttcaa gaccatgaat     360

gcaaagcttc tgatggatcc taagaggcag atctttctag atcaaaacat gctggcagtt 420

attgatgagc tgatgcaggc cctgaatttc aacagtgaga ctgtgccaca aaaatcctcc 480

cttgaagaac cggattttta taaaactaaa atcaagctct gcatacttct tcatgctttc 540

agaattcggg cagtgactat tgatagagtg atgagctatc tgaatgcttc ctaa 594

<210> 12
<211> 51
<212> DNA
<213> Artificial sequence

<220>
<223> PA2

<400> 12
aataaaatat ctttattttc attacatctg tgtgttggtt ttttgtgtga g 51

<210> 13
<211> 19
<212> DNA
<213> Artificial sequence

<220>
<223> TRAC-gRNA-1

<400> 13
gagtctctca gctggtaca 19

<210> 14
<211> 20
<212> DNA
<213> Artificial sequence

<220>
<223> TRAC-gRNA-2

<400> 14
tgtgctagac atgaggtcta 20

<210> 15
<211> 121
<212> DNA
<213> Artificial sequence

<220>
<223> TRAC-gRNA-1-IVT-template

<400> 15
taatacgact cactatagag agtctctcag ctggtacagt tttagagcta gaaatagcaa 60

gttaaaataa ggctagtccg ttatcaactt gaaaaagtgg caccgagtcg gtgctttttt 120

t 121

<210> 16
<211> 121

<212>  DNA
<213>  Artificial sequence

<220>
<223>  TRAC-gRNA-2-IVT-template

<400>  16
taatacgact cactatagtg tgctagacat gaggtctagt tttagagcta gaaatagcaa      60

gttaaaataa ggctagtccg ttatcaactt gaaaaagtgg caccgagtcg gtgctttttt     120

t                                                                     121


<210>  17
<211>  80
<212>  DNA
<213>  Artificial sequence

<220>
<223>  TRAC-gRNA-1-MluI-overlapping-forward

<400>  17
ccgacgcgtt aatacgactc actatagaga gtctctcagc tggtacagtt ttagagctag      60

aaatagcaag ttaaaataag                                                  80


<210>  18
<211>  80
<212>  DNA
<213>  Artificial sequence

<220>
<223>  TRAC-gRNA-1-MluI-overlapping-forward

<400>  18
ccgacgcgtt aatacgactc actatagtgt gctagacatg aggtctagtt ttagagctag      60

aaatagcaag ttaaaataag                                                  80


<210>  19
<211>  81
<212>  DNA
<213>  Artificial sequence

<220>
<223>  All-gRNA-SalI-overlapping-reverse

<400>  19
acgcgtcgac aaaaaaagca ccgactcggt gccacttttt caagttgata acggactagc      60

cttattttaa cttgctattt c                                                81


<210>  20
<211>  19
<212>  DNA
<213>  Artificial sequence

<220>
<223>  All-gRNA-MOF

<400>  20
ccgacgcgtt aatacgact                                                    19


<210>  21
<211>  19
<212>  DNA
<213>  Artificial sequence

<220>
<223>  All-gRNA-SOR

<400>  21
acgcgtcgac aaaaaaagc                                                    19


<210>  22
<211>  24
<212>  DNA
<213>  Artificial sequence

<220>
<223>  TRAC-F

<400>  22
tcatgtccta accctgatcc tctt                                              24


<210>  23
<211>  23
<212>  DNA
<213>  Artificial sequence

<220>
<223>  TRAC-R

<400>  23
ttggactttt cccagctgac aga                                               23


<210>  24
<211>  469
<212>  PRT
<213>  Artificial sequence

<220>
<223>  GPC3-28Z

<400>  24

Glu Val Gln Leu Val Gln Ser Gly Ala Glu Val Lys Lys Pro Gly Ala
1               5                   10                  15


Ser Val Lys Val Ser Cys Lys Ala Ser Gly Tyr Thr Phe Ser Asp Tyr
            20                  25                  30


Glu Met His Trp Val Arg Gln Ala Pro Gly Gln Gly Leu Glu Trp Met
        35                  40                  45

Gly Ala Ile His Pro Gly Ser Gly Asp Thr Ala Tyr Asn Gln Arg Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Phe Tyr Ser Tyr Ala Tyr Trp Gly Gln Gly Thr Leu Val Thr
            100                 105                 110

Val Ser Ala Gly Gly Gly Gly Ser Gly Gly Gly Gly Ser Gly Gly Gly
        115                 120                 125

Gly Ser Asp Ile Val Met Thr Gln Thr Pro Leu Ser Leu Pro Val Thr
    130                 135                 140

Pro Gly Glu Pro Ala Ser Ile Ser Cys Arg Ser Ser Gln Ser Leu Val
145                 150                 155                 160

His Ser Asn Gly Asn Thr Tyr Leu Gln Trp Tyr Leu Gln Lys Pro Gly
                165                 170                 175

Gln Ser Pro Gln Leu Leu Ile Tyr Lys Val Ser Asn Arg Phe Ser Gly
            180                 185                 190

Val Pro Asp Arg Phe Ser Gly Ser Gly Ser Gly Thr Asp Phe Thr Leu
        195                 200                 205

Lys Ile Ser Arg Val Glu Ala Glu Asp Val Gly Val Tyr Tyr Cys Ser
    210                 215                 220

Gln Ser Ile Tyr Val Pro Tyr Thr Phe Gly Gln Gly Thr Lys Leu Glu
225                 230                 235                 240

Ile Lys Arg Thr Thr Thr Pro Ala Pro Arg Pro Pro Thr Pro Ala Pro
                245                 250                 255

Thr Ile Ala Ser Gln Pro Leu Ser Leu Arg Pro Glu Ala Cys Arg Pro
            260                 265                 270

Ala Ala Gly Gly Ala Val His Thr Arg Gly Leu Asp Phe Ala Cys Asp
        275                 280                 285

Phe Trp Val Leu Val Val Val Gly Gly Val Leu Ala Cys Tyr Ser Leu
    290                 295                 300

Leu Val Thr Val Ala Phe Ile Ile Phe Trp Val Arg Ser Lys Arg Ser
305                 310                 315                 320

Arg Leu Leu His Ser Asp Tyr Met Asn Met Thr Pro Arg Arg Pro Gly
                325                 330                 335

Pro Thr Arg Lys His Tyr Gln Pro Tyr Ala Pro Pro Arg Asp Phe Ala
            340                 345                 350

Ala Tyr Arg Ser Arg Val Lys Phe Ser Arg Ser Ala Asp Ala Pro Ala
        355                 360                 365

Tyr Gln Gln Gly Gln Asn Gln Leu Tyr Asn Glu Leu Asn Leu Gly Arg
    370                 375                 380

Arg Glu Glu Tyr Asp Val Leu Asp Lys Arg Arg Gly Arg Asp Pro Glu
385                 390                 395                 400

Met Gly Gly Lys Pro Gln Arg Arg Lys Asn Pro Gln Glu Gly Leu Tyr
                405                 410                 415

Asn Glu Leu Gln Lys Asp Lys Met Ala Glu Ala Tyr Ser Glu Ile Gly
            420                 425                 430

Met Lys Gly Glu Arg Arg Arg Gly Lys Gly His Asp Gly Leu Tyr Gln
        435                 440                 445

Gly Leu Ser Thr Ala Thr Lys Asp Thr Tyr Asp Ala Leu His Met Gln
    450                 455                 460

Ala Leu Pro Pro Arg
465

<210>  25
<211>  467
<212>  PRT
<213>  Artificial sequence

<220>
<223>  GPC3-BBZ

<400>  25

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Ser Asp Tyr
            20                  25                  30

Glu Met His Trp Val Arg Gln Ala Pro Gly Gln Gly Leu Glu Trp Met
        35                  40                  45

Gly Ala Ile His Pro Gly Ser Gly Asp Thr Ala Tyr Asn Gln Arg Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Phe Tyr Ser Tyr Ala Tyr Trp Gly Gln Gly Thr Leu Val Thr
            100                 105                 110

Val Ser Ala Gly Gly Gly Gly Ser Gly Gly Gly Gly Ser Gly Gly Gly
        115                 120                 125

Gly Ser Asp Ile Val Met Thr Gln Thr Pro Leu Ser Leu Pro Val Thr
    130                 135                 140

Pro Gly Glu Pro Ala Ser Ile Ser Cys Arg Ser Ser Gln Ser Leu Val
145                 150                 155                 160

His Ser Asn Gly Asn Thr Tyr Leu Gln Trp Tyr Leu Gln Lys Pro Gly
                165                 170                 175

Gln Ser Pro Gln Leu Leu Ile Tyr Lys Val Ser Asn Arg Phe Ser Gly
            180                 185                 190

Val Pro Asp Arg Phe Ser Gly Ser Gly Ser Gly Thr Asp Phe Thr Leu
        195                 200                 205

Lys Ile Ser Arg Val Glu Ala Glu Asp Val Gly Val Tyr Tyr Cys Ser
    210                 215                 220

Gln Ser Ile Tyr Val Pro Tyr Thr Phe Gly Gln Gly Thr Lys Leu Glu
225                 230                 235                 240

Ile Lys Arg Thr Thr Thr Pro Ala Pro Arg Pro Pro Thr Pro Ala Pro
                245                 250                 255

Thr Ile Ala Ser Gln Pro Leu Ser Leu Arg Pro Glu Ala Cys Arg Pro
            260                 265                 270

Ala Ala Gly Gly Ala Val His Thr Arg Gly Leu Asp Phe Ala Cys Asp
        275                 280                 285

Ile Tyr Ile Trp Ala Pro Leu Ala Gly Thr Cys Gly Val Leu Leu Leu
    290                 295                 300

Ser Leu Val Ile Thr Leu Tyr Cys Lys Arg Gly Arg Lys Lys Leu Leu
305                 310                 315                 320

Tyr Ile Phe Lys Gln Pro Phe Met Arg Pro Val Gln Thr Thr Gln Glu
                325                 330                 335

Glu Asp Gly Cys Ser Cys Arg Phe Pro Glu Glu Glu Glu Gly Gly Cys
            340                 345                 350

Glu Leu Arg Val Lys Phe Ser Arg Ser Ala Asp Ala Pro Ala Tyr Gln
        355                 360                 365

Gln Gly Gln Asn Gln Leu Tyr Asn Glu Leu Asn Leu Gly Arg Arg Glu
    370                 375                 380

Glu Tyr Asp Val Leu Asp Lys Arg Arg Gly Arg Asp Pro Glu Met Gly
385                 390                 395                 400

Gly Lys Pro Gln Arg Arg Lys Asn Pro Gln Glu Gly Leu Tyr Asn Glu
                405                 410                 415

Leu Gln Lys Asp Lys Met Ala Glu Ala Tyr Ser Glu Ile Gly Met Lys
            420                 425                 430

Gly Glu Arg Arg Arg Gly Lys Gly His Asp Gly Leu Tyr Gln Gly Leu
        435                 440                 445

Ser Thr Ala Thr Lys Asp Thr Tyr Asp Ala Leu His Met Gln Ala Leu
    450                 455                 460

Pro Pro Arg
465

<210>  26
<211>  511
<212>  PRT
<213>  Artificial sequence

<220>
<223>  GPC3-28BBZ

<400>  26

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Ser Asp Tyr
            20                  25                  30

Glu Met His Trp Val Arg Gln Ala Pro Gly Gln Gly Leu Glu Trp Met
        35                  40                  45

Gly Ala Ile His Pro Gly Ser Gly Asp Thr Ala Tyr Asn Gln Arg Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Phe Tyr Ser Tyr Ala Tyr Trp Gly Gln Gly Thr Leu Val Thr
            100                 105                 110

Val Ser Ala Gly Gly Gly Gly Ser Gly Gly Gly Gly Ser Gly Gly Gly
        115                 120                 125

Gly Ser Asp Ile Val Met Thr Gln Thr Pro Leu Ser Leu Pro Val Thr
    130                 135                 140

Pro Gly Glu Pro Ala Ser Ile Ser Cys Arg Ser Ser Gln Ser Leu Val
145                 150                 155                 160

His Ser Asn Gly Asn Thr Tyr Leu Gln Trp Tyr Leu Gln Lys Pro Gly
                165                 170                 175

Gln Ser Pro Gln Leu Leu Ile Tyr Lys Val Ser Asn Arg Phe Ser Gly
            180                 185                 190

Val Pro Asp Arg Phe Ser Gly Ser Gly Ser Gly Thr Asp Phe Thr Leu
        195                 200                 205

Lys Ile Ser Arg Val Glu Ala Glu Asp Val Gly Val Tyr Tyr Cys Ser
    210                 215                 220

Gln Ser Ile Tyr Val Pro Tyr Thr Phe Gly Gln Gly Thr Lys Leu Glu
225                 230                 235                 240

Ile Lys Arg Thr Thr Thr Pro Ala Pro Arg Pro Pro Thr Pro Ala Pro
                245                 250                 255

Thr Ile Ala Ser Gln Pro Leu Ser Leu Arg Pro Glu Ala Cys Arg Pro
            260                 265                 270

Ala Ala Gly Gly Ala Val His Thr Arg Gly Leu Asp Phe Ala Cys Asp
        275                 280                 285

Phe Trp Val Leu Val Val Val Gly Gly Val Leu Ala Cys Tyr Ser Leu
    290                 295                 300

Leu Val Thr Val Ala Phe Ile Ile Phe Trp Val Arg Ser Lys Arg Ser
305                 310                 315                 320

Arg Leu Leu His Ser Asp Tyr Met Asn Met Thr Pro Arg Arg Pro Gly
                325                 330                 335

Pro Thr Arg Lys His Tyr Gln Pro Tyr Ala Pro Pro Arg Asp Phe Ala
            340                 345                 350

Ala Tyr Arg Ser Lys Arg Gly Arg Lys Lys Leu Leu Tyr Ile Phe Lys
        355                 360                 365

Gln Pro Phe Met Arg Pro Val Gln Thr Thr Gln Glu Glu Asp Gly Cys
    370                 375                 380

Ser Cys Arg Phe Pro Glu Glu Glu Glu Gly Gly Cys Glu Leu Arg Val
385                 390                 395                 400

Lys Phe Ser Arg Ser Ala Asp Ala Pro Ala Tyr Gln Gln Gly Gln Asn
                405                 410                 415

Gln Leu Tyr Asn Glu Leu Asn Leu Gly Arg Arg Glu Glu Tyr Asp Val
            420                 425                 430

Leu Asp Lys Arg Arg Gly Arg Asp Pro Glu Met Gly Gly Lys Pro Gln
        435                 440                 445

Arg Arg Lys Asn Pro Gln Glu Gly Leu Tyr Asn Glu Leu Gln Lys Asp
    450                 455                 460

Lys Met Ala Glu Ala Tyr Ser Glu Ile Gly Met Lys Gly Glu Arg Arg
465                 470                 475                 480

Arg Gly Lys Gly His Asp Gly Leu Tyr Gln Gly Leu Ser Thr Ala Thr
                485                 490                 495

Lys Asp Thr Tyr Asp Ala Leu His Met Gln Ala Leu Pro Pro Arg
            500                 505                 510

<210>  27
<211>  356

<212>  PRT
<213>  Artificial sequence

<220>
<223>  GPC3-z

<400>  27

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Ser Asp Tyr
            20                  25                  30

Glu Met His Trp Val Arg Gln Ala Pro Gly Gln Gly Leu Glu Trp Met
        35                  40                  45

Gly Ala Ile His Pro Gly Ser Gly Asp Thr Ala Tyr Asn Gln Arg Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Phe Tyr Ser Tyr Ala Tyr Trp Gly Gln Gly Thr Leu Val Thr
            100                 105                 110

Val Ser Ala Gly Gly Gly Gly Ser Gly Gly Gly Gly Ser Gly Gly Gly
        115                 120                 125

Gly Ser Asp Ile Val Met Thr Gln Thr Pro Leu Ser Leu Pro Val Thr
    130                 135                 140

Pro Gly Glu Pro Ala Ser Ile Ser Cys Arg Ser Ser Gln Ser Leu Val
145                 150                 155                 160

His Ser Asn Gly Asn Thr Tyr Leu Gln Trp Tyr Leu Gln Lys Pro Gly
                165                 170                 175

Gln Ser Pro Gln Leu Leu Ile Tyr Lys Val Ser Asn Arg Phe Ser Gly
            180                 185                 190

Val Pro Asp Arg Phe Ser Gly Ser Gly Ser Gly Thr Asp Phe Thr Leu
        195                 200                 205

Lys Ile Ser Arg Val Glu Ala Glu Asp Val Gly Val Tyr Tyr Cys Ser
    210                 215                 220

Gln Ser Ile Tyr Val Pro Tyr Thr Phe Gly Gln Gly Thr Lys Leu Glu
225                 230                 235                 240

Ile Lys Arg Arg Val Lys Phe Ser Arg Ser Ala Asp Ala Pro Ala Tyr
                245                 250                 255

Gln Gln Gly Gln Asn Gln Leu Tyr Asn Glu Leu Asn Leu Gly Arg Arg
            260                 265                 270

Glu Glu Tyr Asp Val Leu Asp Lys Arg Arg Gly Arg Asp Pro Glu Met
        275                 280                 285

Gly Gly Lys Pro Gln Arg Arg Lys Asn Pro Gln Glu Gly Leu Tyr Asn
    290                 295                 300

Glu Leu Gln Lys Asp Lys Met Ala Glu Ala Tyr Ser Glu Ile Gly Met
305                 310                 315                 320

Lys Gly Glu Arg Arg Arg Gly Lys Gly His Asp Gly Leu Tyr Gln Gly
                325                 330                 335

Leu Ser Thr Ala Thr Lys Asp Thr Tyr Asp Ala Leu His Met Gln Ala
            340                 345                 350

Leu Pro Pro Arg
        355

<210>  28
<211>  243
<212>  PRT
<213>  Artificial sequence

<220>
<223>  GPC3-scFv

<400>  28

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Ser Asp Tyr
            20                  25                  30

Glu Met His Trp Val Arg Gln Ala Pro Gly Gln Gly Leu Glu Trp Met
        35                  40                  45

Gly Ala Ile His Pro Gly Ser Gly Asp Thr Ala Tyr Asn Gln Arg Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Phe Tyr Ser Tyr Ala Tyr Trp Gly Gln Gly Thr Leu Val Thr
            100                 105                 110

Val Ser Ala Gly Gly Gly Gly Ser Gly Gly Gly Gly Ser Gly Gly Gly
        115                 120                 125

Gly Ser Asp Ile Val Met Thr Gln Thr Pro Leu Ser Leu Pro Val Thr
    130                 135                 140

Pro Gly Glu Pro Ala Ser Ile Ser Cys Arg Ser Ser Gln Ser Leu Val
145                 150                 155                 160

His Ser Asn Gly Asn Thr Tyr Leu Gln Trp Tyr Leu Gln Lys Pro Gly
                165                 170                 175

Gln Ser Pro Gln Leu Leu Ile Tyr Lys Val Ser Asn Arg Phe Ser Gly
            180                 185                 190

Val Pro Asp Arg Phe Ser Gly Ser Gly Ser Gly Thr Asp Phe Thr Leu
        195                 200                 205

Lys Ile Ser Arg Val Glu Ala Glu Asp Val Gly Val Tyr Tyr Cys Ser
    210                 215                 220

Gln Ser Ile Tyr Val Pro Tyr Thr Phe Gly Gln Gly Thr Lys Leu Glu
225                 230                 235                 240

Ile Lys Arg

<210>  29
<211>  473
<212>  PRT
<213>  Artificial sequence

<220>
<223>  85-2-28Z

<400>  29

Gln Val Gln Leu Gln Glu Ser Gly Pro Gly Leu Ile Lys Pro Ser Gln
1               5                   10                  15

Thr Leu Ser Leu Thr Cys Thr Val Ser Gly Gly Ser Ile Ser Ser Gly

20                  25                  30

Tyr Asn Trp His Trp Ile Arg Gln Pro Pro Gly Lys Gly Leu Glu Trp
        35                  40                  45

Ile Gly Tyr Ile His Tyr Thr Gly Ser Thr Asn Tyr Asn Pro Ala Leu
    50                  55                  60

Arg Ser Arg Val Thr Ile Ser Val Asp Thr Ser Lys Asn Gln Phe Ser
65                  70                  75                  80

Leu Lys Leu Ser Ser Val Thr Ala Ala Asp Thr Ala Ile Tyr Tyr Cys
                85                  90                  95

Ala Arg Ile Tyr Asn Gly Asn Ser Phe Pro Tyr Trp Gly Gln Gly Thr
            100                 105                 110

Thr Val Thr Val Ser Ser Gly Gly Gly Gly Ser Gly Gly Gly Gly Ser
        115                 120                 125

Gly Gly Gly Gly Ser Asp Ile Val Met Thr Gln Ser Pro Asp Ser Leu
    130                 135                 140

Ala Val Ser Leu Gly Glu Arg Ala Thr Ile Asn Cys Lys Ser Ser Gln
145                 150                 155                 160

Ser Leu Phe Asn Ser Gly Asn Gln Lys Asn Tyr Leu Thr Trp Tyr Gln
                165                 170                 175

Gln Lys Pro Gly Gln Pro Pro Lys Leu Leu Ile Tyr Trp Ala Ser Thr
            180                 185                 190

Arg Glu Ser Gly Val Pro Asp Arg Phe Ser Gly Ser Gly Ser Gly Thr
        195                 200                 205

Asp Phe Thr Leu Thr Ile Ser Ser Leu Gln Ala Glu Asp Val Ala Val
    210                 215                 220

Tyr Tyr Cys Gln Asn Ala Tyr Ser Phe Pro Tyr Thr Phe Gly Gly Gly
225                 230                 235                 240

Thr Lys Leu Glu Ile Lys Arg Thr Thr Thr Pro Ala Pro Arg Pro Pro
                245                 250                 255

Thr Pro Ala Pro Thr Ile Ala Ser Gln Pro Leu Ser Leu Arg Pro Glu
            260                 265                 270

Ala Cys Arg Pro Ala Ala Gly Gly Ala Val His Thr Arg Gly Leu Asp
        275                 280                 285

Phe Ala Cys Asp Phe Trp Val Leu Val Val Val Gly Gly Val Leu Ala
    290                 295                 300

Cys Tyr Ser Leu Leu Val Thr Val Ala Phe Ile Ile Phe Trp Val Arg
305                 310                 315                 320

Ser Lys Arg Ser Arg Leu Leu His Ser Asp Tyr Met Asn Met Thr Pro
                325                 330                 335

Arg Arg Pro Gly Pro Thr Arg Lys His Tyr Gln Pro Tyr Ala Pro Pro
            340                 345                 350

Arg Asp Phe Ala Ala Tyr Arg Ser Arg Val Lys Phe Ser Arg Ser Ala
        355                 360                 365

Asp Ala Pro Ala Tyr Gln Gln Gly Gln Asn Gln Leu Tyr Asn Glu Leu
    370                 375                 380

Asn Leu Gly Arg Arg Glu Glu Tyr Asp Val Leu Asp Lys Arg Arg Gly
385                 390                 395                 400

Arg Asp Pro Glu Met Gly Gly Lys Pro Gln Arg Arg Lys Asn Pro Gln
                405                 410                 415

Glu Gly Leu Tyr Asn Glu Leu Gln Lys Asp Lys Met Ala Glu Ala Tyr
            420                 425                 430

Ser Glu Ile Gly Met Lys Gly Glu Arg Arg Arg Gly Lys Gly His Asp
        435                 440                 445

Gly Leu Tyr Gln Gly Leu Ser Thr Ala Thr Lys Asp Thr Tyr Asp Ala
    450                 455                 460

Leu His Met Gln Ala Leu Pro Pro Arg
465                 470

<210>  30
<211>  473
<212>  PRT
<213>  Artificial sequence

<220>
<223>  85-28z

<400>  30

Gln Val Gln Leu Gln Glu Ser Gly Pro Gly Leu Val Lys Pro Ser Gln

1               5                   10                  15

Thr Leu Ser Leu Thr Cys Thr Val Ser Gly Gly Ser Ile Ser Ser Gly
            20                  25                  30

Tyr Asn Trp His Trp Ile Arg Gln Pro Pro Gly Lys Gly Leu Glu Trp
        35                  40                  45

Ile Gly Tyr Ile His Tyr Thr Gly Ser Thr Asn Tyr Asn Pro Ala Leu
    50                  55                  60

Arg Ser Arg Val Thr Ile Ser Val Asp Thr Ser Lys Asn Gln Phe Ser
65                  70                  75                  80

Leu Lys Leu Ser Ser Val Thr Ala Ala Asp Thr Ala Val Tyr Tyr Cys
                85                  90                  95

Ala Arg Ile Tyr Asn Gly Asn Ser Phe Pro Tyr Trp Gly Gln Gly Thr
            100                 105                 110

Thr Val Thr Val Ser Ser Gly Gly Gly Gly Ser Gly Gly Gly Gly Ser
        115                 120                 125

Gly Gly Gly Gly Ser Asp Ile Val Met Thr Gln Ser Pro Asp Ser Leu
    130                 135                 140

Ala Val Ser Leu Gly Glu Arg Ala Thr Ile Asn Cys Lys Ser Ser Gln
145                 150                 155                 160

Ser Leu Phe Asn Ser Gly Asn Gln Lys Asn Tyr Leu Thr Trp Tyr Gln
                165                 170                 175

Gln Lys Pro Gly Gln Pro Pro Lys Leu Leu Ile Tyr Trp Ala Ser Thr
            180                 185                 190

Arg Glu Ser Gly Val Pro Asp Arg Phe Ser Gly Ser Gly Ser Gly Thr
        195                 200                 205

Asp Phe Thr Leu Thr Ile Ser Ser Leu Gln Ala Glu Asp Val Ala Val
    210                 215                 220

Tyr Tyr Cys Gln Asn Ala Tyr Ser Phe Pro Tyr Thr Phe Gly Gly Gly
225                 230                 235                 240

Thr Lys Leu Glu Ile Lys Arg Thr Thr Thr Pro Ala Pro Arg Pro Pro
                245                 250                 255

Thr Pro Ala Pro Thr Ile Ala Ser Gln Pro Leu Ser Leu Arg Pro Glu
            260                 265                 270

Ala Cys Arg Pro Ala Ala Gly Gly Ala Val His Thr Arg Gly Leu Asp
        275                 280                 285

Phe Ala Cys Asp Phe Trp Val Leu Val Val Val Gly Gly Val Leu Ala
    290                 295                 300

Cys Tyr Ser Leu Leu Val Thr Val Ala Phe Ile Ile Phe Trp Val Arg
305                 310                 315                 320

Ser Lys Arg Ser Arg Leu Leu His Ser Asp Tyr Met Asn Met Thr Pro
                325                 330                 335

Arg Arg Pro Gly Pro Thr Arg Lys His Tyr Gln Pro Tyr Ala Pro Pro
            340                 345                 350

Arg Asp Phe Ala Ala Tyr Arg Ser Arg Val Lys Phe Ser Arg Ser Ala
        355                 360                 365

Asp Ala Pro Ala Tyr Gln Gln Gly Gln Asn Gln Leu Tyr Asn Glu Leu
    370                 375                 380

Asn Leu Gly Arg Arg Glu Glu Tyr Asp Val Leu Asp Lys Arg Arg Gly
385                 390                 395                 400

Arg Asp Pro Glu Met Gly Gly Lys Pro Gln Arg Arg Lys Asn Pro Gln
                405                 410                 415

Glu Gly Leu Tyr Asn Glu Leu Gln Lys Asp Lys Met Ala Glu Ala Tyr
            420                 425                 430

Ser Glu Ile Gly Met Lys Gly Glu Arg Arg Arg Gly Lys Gly His Asp
        435                 440                 445

Gly Leu Tyr Gln Gly Leu Ser Thr Ala Thr Lys Asp Thr Tyr Asp Ala
    450                 455                 460

Leu His Met Gln Ala Leu Pro Pro Arg
465                 470

<210>  31
<211>  465
<212>  PRT
<213>  Artificial sequence

<220>
<223>  22-28Z

<400>  31

Asp Ile Gln Met Thr Gln Ser Pro Ser Ser Leu Ser Ala Ser Val Gly
1               5                   10                  15

Asp Arg Val Thr Ile Thr Cys His Ala Ser Gln Asp Ile Asn Val Asn
            20                  25                  30

Ile Gly Trp Leu Gln Gln Lys Pro Gly Lys Ala Phe Lys Gly Leu Ile
        35                  40                  45

Tyr His Gly Lys Asn Leu Glu Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Asn Gln Tyr Glu Asn Ile Pro Leu
                85                  90                  95

Thr Phe Gly Gln Gly Thr Lys Val Glu Ile Lys Arg Gly Gly Gly Gly
            100                 105                 110

Ser Gly Gly Gly Gly Ser Gly Gly Gly Gly Ser Asp Val Gln Leu Val
        115                 120                 125

Glu Ser Gly Gly Gly Leu Val Gln Pro Gly Gly Ser Leu Arg Leu Ser
    130                 135                 140

Cys Ala Val Ser Gly Tyr Ser Ile Thr Ser Asp Tyr Ala Trp Asn Trp
145                 150                 155                 160

Ile Arg Gln Ala Pro Gly Lys Gly Leu Glu Trp Leu Gly Tyr Ile Ser
                165                 170                 175

Tyr Arg Gly Arg Thr Gln Tyr Asn Pro Ser Leu Lys Ser Arg Ile Ser
            180                 185                 190

Ile Thr Arg Asp Asn Ser Lys Asn Thr Phe Phe Leu Gln Leu Asn Ser
        195                 200                 205

Leu Arg Ala Glu Asp Thr Ala Val Tyr Tyr Cys Ala Arg Met Gly Lys
    210                 215                 220

Asn Trp Asp Tyr Trp Gly Gln Gly Thr Leu Val Thr Val Ser Ser Thr
225                 230                 235                 240

Thr Thr Pro Ala Pro Arg Pro Pro Thr Pro Ala Pro Thr Ile Ala Ser
                245                 250                 255

Gln Pro Leu Ser Leu Arg Pro Glu Ala Cys Arg Pro Ala Ala Gly Gly
            260                 265                 270

Ala Val His Thr Arg Gly Leu Asp Phe Ala Cys Asp Phe Trp Val Leu
        275                 280                 285

Val Val Val Gly Gly Val Leu Ala Cys Tyr Ser Leu Leu Val Thr Val
    290                 295                 300

Ala Phe Ile Ile Phe Trp Val Arg Ser Lys Arg Ser Arg Leu Leu His
305                 310                 315                 320

Ser Asp Tyr Met Asn Met Thr Pro Arg Arg Pro Gly Pro Thr Arg Lys
                325                 330                 335

His Tyr Gln Pro Tyr Ala Pro Pro Arg Asp Phe Ala Ala Tyr Arg Ser
            340                 345                 350

Arg Val Lys Phe Ser Arg Ser Ala Asp Ala Pro Ala Tyr Gln Gln Gly
        355                 360                 365

Gln Asn Gln Leu Tyr Asn Glu Leu Asn Leu Gly Arg Arg Glu Glu Tyr
    370                 375                 380

Asp Val Leu Asp Lys Arg Arg Gly Arg Asp Pro Glu Met Gly Gly Lys
385                 390                 395                 400

Pro Gln Arg Arg Lys Asn Pro Gln Glu Gly Leu Tyr Asn Glu Leu Gln
                405                 410                 415

Lys Asp Lys Met Ala Glu Ala Tyr Ser Glu Ile Gly Met Lys Gly Glu
            420                 425                 430

Arg Arg Arg Gly Lys Gly His Asp Gly Leu Tyr Gln Gly Leu Ser Thr
        435                 440                 445

Ala Thr Lys Asp Thr Tyr Asp Ala Leu His Met Gln Ala Leu Pro Pro
    450                 455                 460

Arg
465

<210>  32
<211>  462
<212>  PRT

<213>  Artificial sequence

<220>
<223>  22-BBZ

<400>  32

Asp Ile Gln Met Thr Gln Ser Pro Ser Ser Leu Ser Ala Ser Val Gly
1               5                   10                  15

Asp Arg Val Thr Ile Thr Cys His Ala Ser Gln Asp Ile Asn Val Asn
            20                  25                  30

Ile Gly Trp Leu Gln Gln Lys Pro Gly Lys Ala Phe Lys Gly Leu Ile
        35                  40                  45

Tyr His Gly Lys Asn Leu Glu Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Asn Gln Tyr Glu Asn Ile Pro Leu
                85                  90                  95

Thr Phe Gly Gln Gly Thr Lys Val Glu Ile Lys Arg Gly Gly Gly Gly
            100                 105                 110

Ser Gly Gly Gly Gly Ser Gly Gly Gly Gly Ser Asp Val Gln Leu Val
        115                 120                 125

Glu Ser Gly Gly Gly Leu Val Gln Pro Gly Gly Ser Leu Arg Leu Ser
    130                 135                 140

Cys Ala Val Ser Gly Tyr Ser Ile Thr Ser Asp Tyr Ala Trp Asn Trp
145                 150                 155                 160

Ile Arg Gln Ala Pro Gly Lys Gly Leu Glu Trp Leu Gly Tyr Ile Ser
                165                 170                 175

Tyr Arg Gly Arg Thr Gln Tyr Asn Pro Ser Leu Lys Ser Arg Ile Ser
            180                 185                 190

Ile Thr Arg Asp Asn Ser Lys Asn Thr Phe Phe Leu Gln Leu Asn Ser
        195                 200                 205

Leu Arg Ala Glu Asp Thr Ala Val Tyr Tyr Cys Ala Arg Met Gly Lys
    210                 215                 220

Asn Trp Asp Tyr Trp Gly Gln Gly Thr Leu Val Thr Val Ser Ser Thr
225                 230                 235                 240

Thr Thr Pro Ala Pro Arg Pro Pro Thr Pro Ala Pro Thr Ile Ala Ser
                245                 250                 255

Gln Pro Leu Ser Leu Arg Pro Glu Ala Cys Arg Pro Ala Ala Gly Gly
            260                 265                 270

Ala Val His Thr Arg Gly Leu Asp Phe Ala Cys Asp Ile Tyr Ile Trp
        275                 280                 285

Ala Pro Leu Ala Gly Thr Cys Gly Val Leu Leu Leu Ser Leu Val Ile
    290                 295                 300

Thr Leu Tyr Cys Lys Arg Gly Arg Lys Lys Leu Leu Tyr Ile Phe Lys
305                 310                 315                 320

Gln Pro Phe Met Arg Pro Val Gln Thr Thr Gln Glu Glu Asp Gly Cys
                325                 330                 335

Ser Cys Arg Phe Pro Glu Glu Glu Glu Gly Gly Cys Glu Leu Arg Val
            340                 345                 350

Lys Phe Ser Arg Ser Ala Asp Ala Pro Ala Tyr Lys Gln Gly Gln Asn
        355                 360                 365

Gln Leu Tyr Asn Glu Leu Asn Leu Gly Arg Arg Glu Glu Tyr Asp Val
    370                 375                 380

Leu Asp Lys Arg Arg Gly Arg Asp Pro Glu Met Gly Gly Lys Pro Arg
385                 390                 395                 400

Arg Lys Asn Pro Gln Glu Gly Leu Tyr Asn Glu Leu Gln Lys Asp Lys
                405                 410                 415

Met Ala Glu Ala Tyr Ser Glu Ile Gly Met Lys Gly Glu Arg Arg Arg
            420                 425                 430

Gly Lys Gly His Asp Gly Leu Tyr Gln Gly Leu Ser Thr Ala Thr Lys
        435                 440                 445

Asp Thr Tyr Asp Ala Leu His Met Gln Ala Leu Pro Pro Arg
    450                 455                 460

<210>  33
<211>  507
<212>  PRT

<213>  Artificial sequence

<220>
<223>  22-28BBZ

<400>  33

Asp Ile Gln Met Thr Gln Ser Pro Ser Ser Leu Ser Ala Ser Val Gly
1               5                   10                  15

Asp Arg Val Thr Ile Thr Cys His Ala Ser Gln Asp Ile Asn Val Asn
            20                  25                  30

Ile Gly Trp Leu Gln Gln Lys Pro Gly Lys Ala Phe Lys Gly Leu Ile
        35                  40                  45

Tyr His Gly Lys Asn Leu Glu Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Asn Gln Tyr Glu Asn Ile Pro Leu
                85                  90                  95

Thr Phe Gly Gln Gly Thr Lys Val Glu Ile Lys Arg Gly Gly Gly Gly
            100                 105                 110

Ser Gly Gly Gly Gly Ser Gly Gly Gly Gly Ser Asp Val Gln Leu Val
        115                 120                 125

Glu Ser Gly Gly Gly Leu Val Gln Pro Gly Gly Ser Leu Arg Leu Ser
    130                 135                 140

Cys Ala Val Ser Gly Tyr Ser Ile Thr Ser Asp Tyr Ala Trp Asn Trp
145                 150                 155                 160

Ile Arg Gln Ala Pro Gly Lys Gly Leu Glu Trp Leu Gly Tyr Ile Ser
                165                 170                 175

Tyr Arg Gly Arg Thr Gln Tyr Asn Pro Ser Leu Lys Ser Arg Ile Ser
            180                 185                 190

Ile Thr Arg Asp Asn Ser Lys Asn Thr Phe Phe Leu Gln Leu Asn Ser
        195                 200                 205

Leu Arg Ala Glu Asp Thr Ala Val Tyr Tyr Cys Ala Arg Met Gly Lys
    210                 215                 220

Asn Trp Asp Tyr Trp Gly Gln Gly Thr Leu Val Thr Val Ser Ser Thr
225                 230                 235                 240

Thr Thr Pro Ala Pro Arg Pro Pro Thr Pro Ala Pro Thr Ile Ala Ser
                245                 250                 255

Gln Pro Leu Ser Leu Arg Pro Glu Ala Cys Arg Pro Ala Ala Gly Gly
            260                 265                 270

Ala Val His Thr Arg Gly Leu Asp Phe Ala Cys Asp Phe Trp Val Leu
        275                 280                 285

Val Val Val Gly Gly Val Leu Ala Cys Tyr Ser Leu Leu Val Thr Val
    290                 295                 300

Ala Phe Ile Ile Phe Trp Val Arg Ser Lys Arg Ser Arg Leu Leu His
305                 310                 315                 320

Ser Asp Tyr Met Asn Met Thr Pro Arg Arg Pro Gly Pro Thr Arg Lys
                325                 330                 335

His Tyr Gln Pro Tyr Ala Pro Pro Arg Asp Phe Ala Ala Tyr Arg Ser
            340                 345                 350

Lys Arg Gly Arg Lys Lys Leu Leu Tyr Ile Phe Lys Gln Pro Phe Met
        355                 360                 365

Arg Pro Val Gln Thr Thr Gln Glu Glu Asp Gly Cys Ser Cys Arg Phe
    370                 375                 380

Pro Glu Glu Glu Glu Gly Gly Cys Glu Leu Arg Val Lys Phe Ser Arg
385                 390                 395                 400

Ser Ala Asp Ala Pro Ala Tyr Gln Gln Gly Gln Asn Gln Leu Tyr Asn
                405                 410                 415

Glu Leu Asn Leu Gly Arg Arg Glu Glu Tyr Asp Val Leu Asp Lys Arg
            420                 425                 430

Arg Gly Arg Asp Pro Glu Met Gly Gly Lys Pro Gln Arg Arg Lys Asn
        435                 440                 445

Pro Gln Glu Gly Leu Tyr Asn Glu Leu Gln Lys Asp Lys Met Ala Glu
    450                 455                 460

Ala Tyr Ser Glu Ile Gly Met Lys Gly Glu Arg Arg Arg Gly Lys Gly
465                 470                 475                 480

His Asp Gly Leu Tyr Gln Gly Leu Ser Thr Ala Thr Lys Asp Thr Tyr
                485                 490                 495

Asp Ala Leu His Met Gln Ala Leu Pro Pro Arg
            500                 505

<210> 34
<211> 477
<212> PRT
<213> Artificial sequence

<220>
<223> CD19-28Z

<400> 34

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Phe Ser Ser Tyr
            20                  25                  30

Trp Met Asn Trp Val Arg Gln Ala Pro Gly Gln Gly Leu Glu Trp Met
        35                  40                  45

Gly Gln Ile Trp Pro Gly Asp Gly Asp Thr Asn Tyr Asn Gly Lys Phe
    50                  55                  60

Lys Gly Arg Val Thr Ile Thr Ala Asp Glu Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Arg Glu Thr Thr Thr Val Gly Arg Tyr Tyr Tyr Ala Met Asp
            100                 105                 110

Tyr Trp Gly Gln Gly Thr Thr Val Thr Val Ser Ser Gly Gly Gly Gly
        115                 120                 125

Ser Gly Gly Gly Gly Ser Gly Gly Gly Gly Ser Asp Ile Val Leu Thr
    130                 135                 140

Gln Ser Pro Ala Ser Leu Ala Val Ser Pro Gly Gln Arg Ala Thr Ile
145                 150                 155                 160

Thr Cys Lys Ala Ser Gln Ser Val Asp Tyr Asp Gly Asp Ser Tyr Leu
                165                 170                 175

Asn Trp Tyr Gln Gln Lys Pro Gly Gln Pro Pro Lys Leu Leu Ile Tyr
        180                 185                 190

Asp Ala Ser Asn Leu Val Ser Gly Val Pro Ala Arg Phe Ser Gly Ser
    195                 200                 205

Gly Ser Gly Thr Asp Phe Thr Leu Thr Ile Asn Pro Val Glu Ala Asn
    210                 215                 220

Asp Thr Ala Asn Tyr Tyr Cys Gln Gln Ser Thr Glu Asp Pro Trp Thr
225                 230                 235                 240

Phe Gly Gln Gly Thr Lys Val Glu Ile Lys Arg Thr Thr Thr Pro Ala
                245                 250                 255

Pro Arg Pro Pro Thr Pro Ala Pro Thr Ile Ala Ser Gln Pro Leu Ser
            260                 265                 270

Leu Arg Pro Glu Ala Cys Arg Pro Ala Ala Gly Gly Ala Val His Thr
        275                 280                 285

Arg Gly Leu Asp Phe Ala Cys Asp Phe Trp Val Leu Val Val Val Gly
    290                 295                 300

Gly Val Leu Ala Cys Tyr Ser Leu Leu Val Thr Val Ala Phe Ile Ile
305                 310                 315                 320

Phe Trp Val Arg Ser Lys Arg Ser Arg Leu Leu His Ser Asp Tyr Met
                325                 330                 335

Asn Met Thr Pro Arg Arg Pro Gly Pro Thr Arg Lys His Tyr Gln Pro
            340                 345                 350

Tyr Ala Pro Pro Arg Asp Phe Ala Ala Tyr Arg Ser Arg Val Lys Phe
        355                 360                 365

Ser Arg Ser Ala Asp Ala Pro Ala Tyr Gln Gln Gly Gln Asn Gln Leu
    370                 375                 380

Tyr Asn Glu Leu Asn Leu Gly Arg Arg Glu Glu Tyr Asp Val Leu Asp
385                 390                 395                 400

Lys Arg Arg Gly Arg Asp Pro Glu Met Gly Gly Lys Pro Gln Arg Arg
                405                 410                 415

Lys Asn Pro Gln Glu Gly Leu Tyr Asn Glu Leu Gln Lys Asp Lys Met
            420                 425                 430

Ala Glu Ala Tyr Ser Glu Ile Gly Met Lys Gly Glu Arg Arg Arg Gly
        435                 440                 445

Lys Gly His Asp Gly Leu Tyr Gln Gly Leu Ser Thr Ala Thr Lys Asp
    450                 455                 460

Thr Tyr Asp Ala Leu His Met Gln Ala Leu Pro Pro Arg
465                 470                 475

<210>  35
<211>  469
<212>  PRT
<213>  Artificial sequence

<220>
<223>  33-28Z

<400>  35

Asp Val Val Met Thr Gln Ser Pro Leu Ser Leu Pro Val Thr Pro Gly
1               5                   10                  15

Glu Pro Ala Ser Ile Ser Cys Arg Ser Ser Gln Ser Leu Val His Ser
            20                  25                  30

Asn Ala Asn Thr Tyr Leu His Trp Tyr Leu Gln Lys Pro Gly Gln Ser
        35                  40                  45

Pro Gln Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ser Gln Asn
                85                  90                  95

Thr His Val Pro Pro Thr Phe Gly Gln Gly Thr Lys Leu Glu Ile Lys
            100                 105                 110

Arg Gly Gly Gly Gly Ser Gly Gly Gly Gly Ser Gly Gly Gly Gly Ser
        115                 120                 125

Gln Val Gln Leu Val Gln Ser Gly Ala Glu Val Lys Lys Pro Gly Ala
    130                 135                 140

Ser Val Lys Val Ser Cys Lys Ala Ser Gly Tyr Thr Phe Thr Asp Tyr
145                 150                 155                 160

Glu Met His Trp Val Arg Gln Ala Pro Gly Gln Gly Leu Glu Trp Met
                165                 170                 175

Gly Ala Leu Asp Pro Lys Thr Gly Asp Thr Ala Tyr Ser Gln Lys Phe
            180                 185                 190

Lys Gly Arg Val Thr Leu Thr Ala Asp Glu Ser Thr Ser Thr Ala Tyr
        195                 200                 205

Met Glu Leu Ser Ser Leu Arg Ser Glu Asp Thr Ala Val Tyr Tyr Cys
    210                 215                 220

Thr Arg Phe Tyr Ser Tyr Thr Tyr Trp Gly Gln Gly Thr Leu Val Thr
225                 230                 235                 240

Val Ser Ser Thr Thr Thr Pro Ala Pro Arg Pro Pro Thr Pro Ala Pro
                245                 250                 255

Thr Ile Ala Ser Gln Pro Leu Ser Leu Arg Pro Glu Ala Cys Arg Pro
            260                 265                 270

Ala Ala Gly Gly Ala Val His Thr Arg Gly Leu Asp Phe Ala Cys Asp
        275                 280                 285

Phe Trp Val Leu Val Val Val Gly Gly Val Leu Ala Cys Tyr Ser Leu
    290                 295                 300

Leu Val Thr Val Ala Phe Ile Ile Phe Trp Val Arg Ser Lys Arg Ser
305                 310                 315                 320

Arg Leu Leu His Ser Asp Tyr Met Asn Met Thr Pro Arg Arg Pro Gly
                325                 330                 335

Pro Thr Arg Lys His Tyr Gln Pro Tyr Ala Pro Pro Arg Asp Phe Ala
            340                 345                 350

Ala Tyr Arg Ser Arg Val Lys Phe Ser Arg Ser Ala Asp Ala Pro Ala
        355                 360                 365

Tyr Gln Gln Gly Gln Asn Gln Leu Tyr Asn Glu Leu Asn Leu Gly Arg
    370                 375                 380

Arg Glu Glu Tyr Asp Val Leu Asp Lys Arg Arg Gly Arg Asp Pro Glu
385                 390                 395                 400

Met Gly Gly Lys Pro Gln Arg Arg Lys Asn Pro Gln Glu Gly Leu Tyr
                405                 410                 415

Asn Glu Leu Gln Lys Asp Lys Met Ala Glu Ala Tyr Ser Glu Ile Gly
            420                 425                 430

Met Lys Gly Glu Arg Arg Arg Gly Lys Gly His Asp Gly Leu Tyr Gln
        435                 440                 445

Gly Leu Ser Thr Ala Thr Lys Asp Thr Tyr Asp Ala Leu His Met Gln
    450                 455                 460

Ala Leu Pro Pro Arg
465

<210>  36
<211>  511
<212>  PRT
<213>  Artificial sequence

<220>
<223>  33-28BBZ

<400>  36

Asp Val Val Met Thr Gln Ser Pro Leu Ser Leu Pro Val Thr Pro Gly
1               5                   10                  15

Glu Pro Ala Ser Ile Ser Cys Arg Ser Ser Gln Ser Leu Val His Ser
            20                  25                  30

Asn Ala Asn Thr Tyr Leu His Trp Tyr Leu Gln Lys Pro Gly Gln Ser
        35                  40                  45

Pro Gln Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ser Gln Asn
                85                  90                  95

Thr His Val Pro Pro Thr Phe Gly Gln Gly Thr Lys Leu Glu Ile Lys
            100                 105                 110

Arg Gly Gly Gly Gly Ser Gly Gly Gly Gly Ser Gly Gly Gly Gly Ser
        115                 120                 125

Gln Val Gln Leu Val Gln Ser Gly Ala Glu Val Lys Lys Pro Gly Ala
    130                 135                 140

Ser Val Lys Val Ser Cys Lys Ala Ser Gly Tyr Thr Phe Thr Asp Tyr
145                 150                 155                 160

Glu Met His Trp Val Arg Gln Ala Pro Gly Gln Gly Leu Glu Trp Met
                165                 170                 175

Gly Ala Leu Asp Pro Lys Thr Gly Asp Thr Ala Tyr Ser Gln Lys Phe
            180                 185                 190

Lys Gly Arg Val Thr Leu Thr Ala Asp Glu Ser Thr Ser Thr Ala Tyr
        195                 200                 205

Met Glu Leu Ser Ser Leu Arg Ser Glu Asp Thr Ala Val Tyr Tyr Cys
    210                 215                 220

Thr Arg Phe Tyr Ser Tyr Thr Tyr Trp Gly Gln Gly Thr Leu Val Thr
225                 230                 235                 240

Val Ser Ser Thr Thr Thr Pro Ala Pro Arg Pro Pro Thr Pro Ala Pro
                245                 250                 255

Thr Ile Ala Ser Gln Pro Leu Ser Leu Arg Pro Glu Ala Cys Arg Pro
            260                 265                 270

Ala Ala Gly Gly Ala Val His Thr Arg Gly Leu Asp Phe Ala Cys Asp
        275                 280                 285

Phe Trp Val Leu Val Val Val Gly Gly Val Leu Ala Cys Tyr Ser Leu
    290                 295                 300

Leu Val Thr Val Ala Phe Ile Ile Phe Trp Val Arg Ser Lys Arg Ser
305                 310                 315                 320

Arg Leu Leu His Ser Asp Tyr Met Asn Met Thr Pro Arg Arg Pro Gly
                325                 330                 335

Pro Thr Arg Lys His Tyr Gln Pro Tyr Ala Pro Pro Arg Asp Phe Ala
            340                 345                 350

Ala Tyr Arg Ser Lys Arg Gly Arg Lys Lys Leu Leu Tyr Ile Phe Lys
        355                 360                 365

Gln Pro Phe Met Arg Pro Val Gln Thr Thr Gln Glu Glu Asp Gly Cys
    370                 375                 380

Ser Cys Arg Phe Pro Glu Glu Glu Glu Gly Gly Cys Glu Leu Arg Val
385                 390                 395                 400

Lys Phe Ser Arg Ser Ala Asp Ala Pro Ala Tyr Gln Gln Gly Gln Asn
                405                 410                 415

Gln Leu Tyr Asn Glu Leu Asn Leu Gly Arg Arg Glu Glu Tyr Asp Val
            420                 425                 430

Leu Asp Lys Arg Arg Gly Arg Asp Pro Glu Met Gly Gly Lys Pro Gln
        435                 440                 445

Arg Arg Lys Asn Pro Gln Glu Gly Leu Tyr Asn Glu Leu Gln Lys Asp
    450                 455                 460

Lys Met Ala Glu Ala Tyr Ser Glu Ile Gly Met Lys Gly Glu Arg Arg
465                 470                 475                 480

Arg Gly Lys Gly His Asp Gly Leu Tyr Gln Gly Leu Ser Thr Ala Thr
                485                 490                 495

Lys Asp Thr Tyr Asp Ala Leu His Met Gln Ala Leu Pro Pro Arg
            500                 505                 510

<210>  37
<211>  515
<212>  PRT
<213>  Artificial sequence

<220>
<223>  163-28BBZ

<400>  37
   Gln Ile Gln Leu Val Gln Ser Gly Pro Glu Leu Lys Lys Pro Gly Glu
   1               5                   10                  15
   Thr Val Lys Ile Ser Cys Lys Ala Ser Gly Tyr Thr Phe Thr Asn Tyr
               20                  25                  30
   Gly Met Asn Trp Val Lys Gln Ala Pro Gly Lys Gly Leu Lys Trp Met
           35                  40                  45
   Gly Trp Ile Asn Thr Asn Thr Gly Glu Pro Thr Tyr Ala Glu Glu Phe
       50                  55                  60
   Lys Gly Arg Phe Ala Phe Ser Leu Glu Thr Ser Ala Ser Thr Ala Tyr
   65                  70                  75                  80
   Leu Gln Ile Asn Asn Leu Lys Asn Glu Asp Thr Ala Thr Tyr Phe Cys
                   85                  90                  95
   Ala Arg Leu Gly Phe Gly Asn Ala Met Asp Tyr Trp Gly Gln Gly Thr
   100 105 110
   Ser Val Thr Val Ser Ser Gly Gly Gly Gly Ser Gly Gly Gly Gly Ser
   115 120 125
   Gly Gly Gly Gly Ser Asp Ile Val Met Thr Gln Ser Pro Ser Ser Leu
   130 135 140
   Thr Val Thr Ala Gly Glu Lys Val Thr Met Ser Cys Lys Ser Ser Gln
   145 150 155 160
   Ser Leu Leu Asn Ser Gly Asn Gln Lys Asn Tyr Leu Thr Trp Tyr Gln
   165 170 175
   Gln Lys Pro Gly Gln Pro Pro Lys Leu Leu Ile Tyr Trp Ala Ser Thr
   180 185 190
   Arg Glu Ser Gly Val Pro Asp Arg Phe Thr Gly Ser Gly Ser Gly Thr
   195 200 205
   Asp Phe Thr Leu Thr Ile Ser Ser Val Gln Ala Glu Asp Leu Ala Val
   210 215 220
   Tyr Tyr Cys Gln Asn Asp Tyr Ser Tyr Pro Leu Thr Phe Gly Ala Gly
   225 230 235 240
   Thr Lys Leu Glu Leu Lys Arg Thr Thr Thr Pro Ala Pro Arg Pro Pro
   245 250 255
   Thr Pro Ala Pro Thr Ile Ala Ser Gln Pro Leu Ser Leu Arg Pro Glu
   260 265 270
   Ala Cys Arg Pro Ala Ala Gly Gly Ala Val His Thr Arg Gly Leu Asp
   275 280 285
   Phe Ala Cys Asp Phe Trp Val Leu Val Val Val Gly Gly Val Leu Ala
   290 295 300
   Cys Tyr Ser Leu Leu Val Thr Val Ala Phe Ile Ile Phe Trp Val Arg
   305 310 315 320
   Ser Lys Arg Ser Arg Leu Leu His Ser Asp Tyr Met Asn Met Thr Pro
   325 330 335
   Arg Arg Pro Gly Pro Thr Arg Lys His Tyr Gln Pro Tyr Ala Pro Pro
   340 345 350
   Arg Asp Phe Ala Ala Tyr Arg Ser Lys Arg Gly Arg Lys Lys Leu Leu
   355 360 365
   Tyr Ile Phe Lys Gln Pro Phe Met Arg Pro Val Gln Thr Thr Gln Glu
   370 375 380
   Glu Asp Gly Cys Ser Cys Arg Phe Pro Glu Glu Glu Glu Gly Gly Cys
   385 390 395 400
   Glu Leu Arg Val Lys Phe Ser Arg Ser Ala Asp Ala Pro Ala Tyr Gln
   405 410 415
   Gln Gly Gln Asn Gln Leu Tyr Asn Glu Leu Asn Leu Gly Arg Arg Glu
   420 425 430
   Glu Tyr Asp Val Leu Asp Lys Arg Arg Gly Arg Asp Pro Glu Met Gly
   435 440 445
   Gly Lys Pro Gln Arg Arg Lys Asn Pro Gln Glu Gly Leu Tyr Asn Glu
   450 455 460
   Leu Gln Lys Asp Lys Met Ala Glu Ala Tyr Ser Glu Ile Gly Met Lys
   465 470 475 480
   Gly Glu Arg Arg Arg Gly Lys Gly His Asp Gly Leu Tyr Gln Gly Leu
   485 490 495
   Ser Thr Ala Thr Lys Asp Thr Tyr Asp Ala Leu His Met Gln Ala Leu
   500 505 510
   Pro Pro Arg
   515

<210>  38
<211>  515
<212>  PRT
<213>  Artificial sequence

<220>
<223>  175-28BBZ

<400>  38

Gln Val Gln Leu Gln Gln Pro Gly Ala Glu Leu Val Arg Pro Gly Ala
1               5                   10                  15

Ser Val Lys Leu Ser Cys Lys Ala Ser Gly Tyr Thr Phe Thr Ser Tyr
            20                  25                  30

Trp Ile Asn Trp Val Lys Gln Arg Pro Gly Gln Gly Leu Glu Trp Ile
        35                  40                  45

Gly Asn Ile Tyr Pro Ser Asp Ser Tyr Thr Asn Tyr Asn Gln Lys Phe
    50                  55                  60

Lys Asp Lys Ala Thr Leu Thr Val Asp Lys Ser Ser Ser Thr Ala Tyr
65                  70                  75                  80

Met Gln Leu Ser Ser Pro Thr Ser Glu Asp Ser Ala Val Tyr Tyr Cys
                85                  90                  95

Thr Arg Ser Trp Arg Gly Asn Ser Phe Asp Tyr Trp Gly Gln Gly Thr
            100                 105                 110

Thr Leu Thr Val Ser Ser Gly Gly Gly Gly Ser Gly Gly Gly Gly Ser
        115                 120                 125

Gly Gly Gly Gly Ser Asp Ile Val Met Thr Gln Ser Pro Ser Ser Leu
    130                 135                 140

Thr Val Thr Ala Gly Glu Lys Val Thr Met Ser Cys Lys Ser Ser Gln
145                 150                 155                 160

Ser Leu Leu Asn Ser Gly Asn Gln Lys Asn Tyr Leu Thr Trp Tyr Gln
                165                 170                 175

Gln Lys Pro Gly Gln Pro Pro Lys Leu Leu Ile Tyr Trp Ala Ser Thr
            180                 185                 190

Arg Glu Ser Gly Val Pro Asp Arg Phe Thr Gly Ser Gly Ser Gly Thr
        195                 200                 205

Asp Phe Thr Leu Thr Ile Ser Ser Val Gln Ala Glu Asp Leu Ala Val
    210                 215                 220

Tyr Tyr Cys Gln Asn Asp Tyr Ser Tyr Pro Phe Thr Phe Gly Ser Gly
225                 230                 235                 240

Thr Lys Leu Glu Ile Lys Arg Thr Thr Thr Pro Ala Pro Arg Pro Pro
                245                 250                 255

Thr Pro Ala Pro Thr Ile Ala Ser Gln Pro Leu Ser Leu Arg Pro Glu
            260                 265                 270

Ala Cys Arg Pro Ala Ala Gly Gly Ala Val His Thr Arg Gly Leu Asp
        275                 280                 285

Phe Ala Cys Asp Phe Trp Val Leu Val Val Val Gly Gly Val Leu Ala
    290                 295                 300

Cys Tyr Ser Leu Leu Val Thr Val Ala Phe Ile Ile Phe Trp Val Arg
305                 310                 315                 320

Ser Lys Arg Ser Arg Leu Leu His Ser Asp Tyr Met Asn Met Thr Pro
                325                 330                 335

Arg Arg Pro Gly Pro Thr Arg Lys His Tyr Gln Pro Tyr Ala Pro Pro
            340                 345                 350

Arg Asp Phe Ala Ala Tyr Arg Ser Lys Arg Gly Arg Lys Lys Leu Leu
        355                 360                 365

Tyr Ile Phe Lys Gln Pro Phe Met Arg Pro Val Gln Thr Thr Gln Glu
    370                 375                 380

Glu Asp Gly Cys Ser Cys Arg Phe Pro Glu Glu Glu Glu Gly Gly Cys
385                 390                 395                 400

Glu Leu Arg Val Lys Phe Ser Arg Ser Ala Asp Ala Pro Ala Tyr Gln
                405                 410                 415

Gln Gly Gln Asn Gln Leu Tyr Asn Glu Leu Asn Leu Gly Arg Arg Glu
            420                 425                 430

Glu Tyr Asp Val Leu Asp Lys Arg Arg Gly Arg Asp Pro Glu Met Gly
        435                 440                 445

Gly Lys Pro Gln Arg Arg Lys Asn Pro Gln Glu Gly Leu Tyr Asn Glu
    450                 455                 460

Leu Gln Lys Asp Lys Met Ala Glu Ala Tyr Ser Glu Ile Gly Met Lys
465                 470                 475                 480

Gly Glu Arg Arg Arg Gly Lys Gly His Asp Gly Leu Tyr Gln Gly Leu
                485                 490                 495

Ser Thr Ala Thr Lys Asp Thr Tyr Asp Ala Leu His Met Gln Ala Leu
            500                 505                 510

Pro Pro Arg
        515

<210>  39
<211>  522

<212>  PRT
<213>  Artificial sequence

<220>
<223>  16-28BBZ

<400>  39

Gln Val Gln Leu Glu Gln Ser Gly Leu Gly Leu Val Lys Pro Ser Gln
1               5                   10                  15

Thr Leu Ser Leu Thr Cys Ala Ile Ser Gly Asp Thr Val Ser Ser Asp
            20                  25                  30

Ser Ala Ala Trp Asn Trp Ile Arg Gln Ser Pro Ser Arg Gly Leu Glu
        35                  40                  45

Trp Leu Gly Arg Thr Tyr Tyr Arg Ser Lys Trp Phe Asn Asp Tyr Ala
    50                  55                  60

Val Ser Val Lys Gly Arg Ile Thr Ile Asn Ser Asp Thr Ser Lys Asn
65                  70                  75                  80

Gln Phe Ser Leu Gln Leu Asn Ser Val Thr Pro Glu Asp Thr Ala Val
                85                  90                  95

Tyr Tyr Cys Ala Arg Ser Asn Ser Tyr Tyr Tyr Tyr Ala Met Asp Val
            100                 105                 110

Trp Gly Gln Gly Thr Leu Val Thr Val Ser Ser Gly Gly Gly Gly Ser
        115                 120                 125

Gly Gly Gly Gly Ser Gly Gly Gly Gly Ser Gln Ala Val Leu Thr Gln
    130                 135                 140

Pro Ser Ser Leu Ser Ala Ser Pro Gly Ala Ser Ala Ser Leu Thr Cys
145                 150                 155                 160

Thr Leu Arg Ser Gly Ile Asn Val Gly Ile Tyr Arg Ile Tyr Trp Tyr
                165                 170                 175

Gln Gln Arg Pro Gly Ser Pro Pro Gln Ile Leu Leu Thr Tyr Lys Ser
            180                 185                 190

Asp Ser Asp Lys Tyr Gln Gly Ser Gly Val Pro Ser Arg Phe Ser Gly
        195                 200                 205

Ser Lys Asp Ala Ser Ala Asn Ala Gly Ile Leu Leu Ile Ser Gly Leu
    210                 215                 220

Gln Ser Glu Asp Glu Ala Asp Tyr Tyr Cys Met Ile Trp His Ser Gly
225                 230                 235                 240

Gly Trp Val Phe Gly Gly Gly Thr Lys Val Thr Val Leu Gly Thr Thr
                245                 250                 255

Thr Pro Ala Pro Arg Pro Pro Thr Pro Ala Pro Thr Ile Ala Ser Gln
            260                 265                 270

Pro Leu Ser Leu Arg Pro Glu Ala Cys Arg Pro Ala Ala Gly Gly Ala
        275                 280                 285

Val His Thr Arg Gly Leu Asp Phe Ala Cys Asp Phe Trp Val Leu Val
    290                 295                 300

Val Val Gly Gly Val Leu Ala Cys Tyr Ser Leu Leu Val Thr Val Ala
305                 310                 315                 320

Phe Ile Ile Phe Trp Val Arg Ser Lys Arg Ser Arg Leu Leu His Ser
                325                 330                 335

Asp Tyr Met Asn Met Thr Pro Arg Arg Pro Gly Pro Thr Arg Lys His
            340                 345                 350

Tyr Gln Pro Tyr Ala Pro Pro Arg Asp Phe Ala Ala Tyr Arg Ser Lys
        355                 360                 365

Arg Gly Arg Lys Lys Leu Leu Tyr Ile Phe Lys Gln Pro Phe Met Arg
    370                 375                 380

Pro Val Gln Thr Thr Gln Glu Glu Asp Gly Cys Ser Cys Arg Phe Pro
385                 390                 395                 400

Glu Glu Glu Glu Gly Gly Cys Glu Leu Arg Val Lys Phe Ser Arg Ser
                405                 410                 415

Ala Asp Ala Pro Ala Tyr Gln Gln Gly Gln Asn Gln Leu Tyr Asn Glu
            420                 425                 430

Leu Asn Leu Gly Arg Arg Glu Glu Tyr Asp Val Leu Asp Lys Arg Arg
        435                 440                 445

Gly Arg Asp Pro Glu Met Gly Gly Lys Pro Gln Arg Arg Lys Asn Pro
    450                 455                 460

Gln Glu Gly Leu Tyr Asn Glu Leu Gln Lys Asp Lys Met Ala Glu Ala
465                 470                 475                 480

Tyr Ser Glu Ile Gly Met Lys Gly Glu Arg Arg Arg Gly Lys Gly His
                485                 490                 495

Asp Gly Leu Tyr Gln Gly Leu Ser Thr Ala Thr Lys Asp Thr Tyr Asp
            500                 505                 510

Ala Leu His Met Gln Ala Leu Pro Pro Arg
        515                 520

<210> 40
<211> 473
<212> PRT
<213> Artificial sequence

<220>
<223> 32-28Z

<400> 40

Gln Met Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Tyr Met His Trp Val Arg Gln Ala Pro Gly Gln Gly Leu Glu Trp Met
        35                  40                  45

Gly Ile Ile Asn Pro Ser Gly Gly Ser Thr Ser Tyr Ala Gln Lys Phe
    50                  55                  60

Gln Gly Arg Val Thr Met Thr Arg Asp Thr Ser Thr Ser Thr Val Tyr
65                  70                  75                  80

Met Glu Leu Ser Ser Leu Arg Ser Glu Asp Thr Ala Val Tyr Tyr Cys
                85                  90                  95

Ala Ser Ser Arg Ser Gly Thr Thr Val Val Asn His Asp Ala Phe Asp
            100                 105                 110

Ile Trp Gly Lys Gly Thr Thr Val Thr Val Ser Ser Gly Gly Gly Gly
        115                 120                 125

Ser Gly Gly Gly Gly Ser Gly Gly Gly Gly Ser Asp Ile Gln Leu Thr
    130                 135                 140

Gln Ser Pro Ser Ser Leu Ser Ala Ser Val Gly Asp Arg Val Thr Ile
145                 150                 155                 160

Thr Cys Arg Ala Ser Gln Val Ile Ser Arg Ala Leu Ala Trp Tyr Gln
                165                 170                 175

Gln Thr Pro Gly Lys Pro Pro Lys Leu Leu Ile Tyr Asp Ala Ser Asn
            180                 185                 190

Leu Gln Ser Gly Val Pro Ser Arg Phe Ser Gly Ser Gly Ser Gly Thr
        195                 200                 205

Asp Phe Thr Leu Thr Ile Ser Arg Leu Gln Pro Glu Asp Phe Ala Thr
    210                 215                 220

Tyr Tyr Cys Gln Gln Phe Asn Ser Tyr Pro Leu Thr Phe Gly Gly Gly
225                 230                 235                 240

Thr Lys Leu Glu Ile Lys Arg Thr Thr Thr Pro Ala Pro Arg Pro Pro
                245                 250                 255

Thr Pro Ala Pro Thr Ile Ala Ser Gln Pro Leu Ser Leu Arg Pro Glu
            260                 265                 270

Ala Cys Arg Pro Ala Ala Gly Gly Ala Val His Thr Arg Gly Leu Asp
        275                 280                 285

Phe Ala Cys Asp Phe Trp Val Leu Val Val Val Gly Gly Val Leu Ala
    290                 295                 300

Cys Tyr Ser Leu Leu Val Thr Val Ala Phe Ile Ile Phe Trp Val Arg
305                 310                 315                 320

Ser Lys Arg Ser Arg Leu Leu His Ser Asp Tyr Met Asn Met Thr Pro
                325                 330                 335

Arg Arg Pro Gly Pro Thr Arg Lys His Tyr Gln Pro Tyr Ala Pro Pro
            340                 345                 350

Arg Asp Phe Ala Ala Tyr Arg Ser Arg Val Lys Phe Ser Arg Ser Ala
        355                 360                 365

Asp Ala Pro Ala Tyr Gln Gln Gly Gln Asn Gln Leu Tyr Asn Glu Leu
    370                 375                 380

Asn Leu Gly Arg Arg Glu Glu Tyr Asp Val Leu Asp Lys Arg Arg Gly
385                 390                 395                 400

Arg Asp Pro Glu Met Gly Gly Lys Pro Gln Arg Arg Lys Asn Pro Gln
                405                 410                 415

Glu Gly Leu Tyr Asn Glu Leu Gln Lys Asp Lys Met Ala Glu Ala Tyr
            420                 425                 430

Ser Glu Ile Gly Met Lys Gly Glu Arg Arg Arg Gly Lys Gly His Asp
        435                 440                 445

Gly Leu Tyr Gln Gly Leu Ser Thr Ala Thr Lys Asp Thr Tyr Asp Ala
    450                 455                 460

Leu His Met Gln Ala Leu Pro Pro Arg
465                 470

<210>  41
<211>  470
<212>  PRT
<213>  Artificial sequence

<220>
<223>  32-BBZ

<400>  41

Gln Met Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Tyr Met His Trp Val Arg Gln Ala Pro Gly Gln Gly Leu Glu Trp Met
        35                  40                  45

Gly Ile Ile Asn Pro Ser Gly Gly Ser Thr Ser Tyr Ala Gln Lys Phe
    50                  55                  60

Gln Gly Arg Val Thr Met Thr Arg Asp Thr Ser Thr Ser Thr Val Tyr
65                  70                  75                  80

Met Glu Leu Ser Ser Leu Arg Ser Glu Asp Thr Ala Val Tyr Tyr Cys
                85                  90                  95

Ala Ser Ser Arg Ser Gly Thr Thr Val Val Asn His Asp Ala Phe Asp
            100                 105                 110

Ile Trp Gly Lys Gly Thr Thr Val Thr Val Ser Ser Gly Gly Gly Gly
        115                 120                 125

Ser Gly Gly Gly Gly Ser Gly Gly Gly Gly Ser Asp Ile Gln Leu Thr
    130                 135                 140

Gln Ser Pro Ser Ser Leu Ser Ala Ser Val Gly Asp Arg Val Thr Ile
145                 150                 155                 160

Thr Cys Arg Ala Ser Gln Val Ile Ser Arg Ala Leu Ala Trp Tyr Gln
                165                 170                 175

Gln Thr Pro Gly Lys Pro Pro Lys Leu Leu Ile Tyr Asp Ala Ser Asn
            180                 185                 190

Leu Gln Ser Gly Val Pro Ser Arg Phe Ser Gly Ser Gly Ser Gly Thr
        195                 200                 205

Asp Phe Thr Leu Thr Ile Ser Arg Leu Gln Pro Glu Asp Phe Ala Thr
    210                 215                 220

Tyr Tyr Cys Gln Gln Phe Asn Ser Tyr Pro Leu Thr Phe Gly Gly Gly
225                 230                 235                 240

Thr Lys Leu Glu Ile Lys Arg Thr Thr Thr Pro Ala Pro Arg Pro Pro
                245                 250                 255

Thr Pro Ala Pro Thr Ile Ala Ser Gln Pro Leu Ser Leu Arg Pro Glu
            260                 265                 270

Ala Cys Arg Pro Ala Ala Gly Gly Ala Val His Thr Arg Gly Leu Asp
        275                 280                 285

Phe Ala Cys Asp Ile Tyr Ile Trp Ala Pro Leu Ala Gly Thr Cys Gly
    290                 295                 300

Val Leu Leu Leu Ser Leu Val Ile Thr Leu Tyr Cys Lys Arg Gly Arg
305                 310                 315                 320

Lys Lys Leu Leu Tyr Ile Phe Lys Gln Pro Phe Met Arg Pro Val Gln
                325                 330                 335

Thr Thr Gln Glu Glu Asp Gly Cys Ser Cys Arg Phe Pro Glu Glu Glu
            340                 345                 350

Glu Gly Gly Cys Glu Leu Arg Val Lys Phe Ser Arg Ser Ala Asp Ala
        355                 360                 365

Pro Ala Tyr Lys Gln Gly Gln Asn Gln Leu Tyr Asn Glu Leu Asn Leu
    370                 375                 380

Gly Arg Arg Glu Glu Tyr Asp Val Leu Asp Lys Arg Arg Gly Arg Asp
385                 390                 395                 400

Pro Glu Met Gly Gly Lys Pro Arg Arg Lys Asn Pro Gln Glu Gly Leu
                405                 410                 415

Tyr Asn Glu Leu Gln Lys Asp Lys Met Ala Glu Ala Tyr Ser Glu Ile
            420                 425                 430

Gly Met Lys Gly Glu Arg Arg Arg Gly Lys Gly His Asp Gly Leu Tyr
        435                 440                 445

Gln Gly Leu Ser Thr Ala Thr Lys Asp Thr Tyr Asp Ala Leu His Met
    450                 455                 460

Gln Ala Leu Pro Pro Arg
465                 470

<210>  42
<211>  515
<212>  PRT
<213>  Artificial sequence

<220>
<223>  32-28BBZ

<400>  42
   Gln Met Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Ser Tyr
               20                  25                  30
   Tyr Met His Trp Val Arg Gln Ala Pro Gly Gln Gly Leu Glu Trp Met
           35                  40                  45
   Gly Ile Ile Asn Pro Ser Gly Gly Ser Thr Ser Tyr Ala Gln Lys Phe
       50                  55                  60
   Gln Gly Arg Val Thr Met Thr Arg Asp Thr Ser Thr Ser Thr Val Tyr
   65                  70                  75                  80
   Met Glu Leu Ser Ser Leu Arg Ser Glu Asp Thr Ala Val Tyr Tyr Cys
                   85                  90                  95
   Ala Ser Ser Arg Ser Gly Thr Thr Val Val Asn His Asp Ala Phe Asp
               100                 105                 110
   Ile Trp Gly Lys Gly Thr Thr Val Thr Val Ser Ser Gly Gly Gly Gly
           115                 120                 125
   Ser Gly Gly Gly Gly Ser Gly Gly Gly Gly Ser Asp Ile Gln Leu Thr
       130                 135                 140
   Gln Ser Pro Ser Ser Leu Ser Ala Ser Val Gly Asp Arg Val Thr Ile
   145                 150                 155                 160
   Thr Cys Arg Ala Ser Gln Val Ile Ser Arg Ala Leu Ala Trp Tyr Gln
                   165                 170                 175
   Gln Thr Pro Gly Lys Pro Pro Lys Leu Leu Ile Tyr Asp Ala Ser Asn
               180                 185                 190
   Leu Gln Ser Gly Val Pro Ser Arg Phe Ser Gly Ser Gly Ser Gly Thr
           195                 200                 205
   Asp Phe Thr Leu Thr Ile Ser Arg Leu Gln Pro Glu Asp Phe Ala Thr
       210                 215                 220
   Tyr Tyr Cys Gln Gln Phe Asn Ser Tyr Pro Leu Thr Phe Gly Gly Gly
   225                 230                 235                 240
   Thr Lys Leu Glu Ile Lys Arg Thr Thr Thr Pro Ala Pro Arg Pro Pro
                   245                 250                 255
   Thr Pro Ala Pro Thr Ile Ala Ser Gln Pro Leu Ser Leu Arg Pro Glu
               260                 265                 270
   Ala Cys Arg Pro Ala Ala Gly Gly Ala Val His Thr Arg Gly Leu Asp
           275                 280                 285
   Phe Ala Cys Asp Phe Trp Val Leu Val Val Val Gly Gly Val Leu Ala
       290                 295                 300
   Cys Tyr Ser Leu Leu Val Thr Val Ala Phe Ile Ile Phe Trp Val Arg
   305                 310                 315                 320
   Ser Lys Arg Ser Arg Leu Leu His Ser Asp Tyr Met Asn Met Thr Pro
                   325                 330                 335
   Arg Arg Pro Gly Pro Thr Arg Lys His Tyr Gln Pro Tyr Ala Pro Pro
               340                 345                 350
   Arg Asp Phe Ala Ala Tyr Arg Ser Lys Arg Gly Arg Lys Lys Leu Leu
           355                 360                 365
   Tyr Ile Phe Lys Gln Pro Phe Met Arg Pro Val Gln Thr Thr Gln Glu
       370                 375                 380
   Glu Asp Gly Cys Ser Cys Arg Phe Pro Glu Glu Glu Glu Gly Gly Cys
   385                 390                 395                 400
   Glu Leu Arg Val Lys Phe Ser Arg Ser Ala Asp Ala Pro Ala Tyr Gln
                   405                 410                 415
   Gln Gly Gln Asn Gln Leu Tyr Asn Glu Leu Asn Leu Gly Arg Arg Glu
               420                 425                 430
   Glu Tyr Asp Val Leu Asp Lys Arg Arg Gly Arg Asp Pro Glu Met Gly
           435                 440                 445
   Gly Lys Pro Gln Arg Arg Lys Asn Pro Gln Glu Gly Leu Tyr Asn Glu
       450                 455                 460
   Leu Gln Lys Asp Lys Met Ala Glu Ala Tyr Ser Glu Ile Gly Met Lys
   465                 470                 475                 480
   Gly Glu Arg Arg Arg Gly Lys Gly His Asp Gly Leu Tyr Gln Gly Leu
                   485                 490                 495
   Ser Thr Ala Thr Lys Asp Thr Tyr Asp Ala Leu His Met Gln Ala Leu
               500                 505                 510
   Pro Pro Arg
           515

<210>  43
<211>  466
<212>  PRT
<213>  Artificial sequence

<220>
<223>  EGFR-CAR-28z

<400>  43

Asp Ile Leu Met Thr Gln Ser Pro Ser Ser Met Ser Val Ser Leu Gly
1               5                   10                  15

Asp Thr Val Ser Ile Thr Cys His Ser Ser Gln Asp Ile Asn Ser Asn
            20                  25                  30

Ile Gly Trp Leu Gln Gln Arg Pro Gly Lys Ser Phe Lys Gly Leu Ile
        35                  40                  45

Tyr His Gly Thr Asn Leu Asp Asp Glu Val Pro Ser Arg Phe Ser Gly
    50                  55                  60

Ser Gly Ser Gly Ala Asp Tyr Ser Leu Thr Ile Ser Ser Leu Glu Ser
65                  70                  75                  80

Glu Asp Phe Ala Asp Tyr Tyr Cys Val Gln Tyr Ala Gln Phe Pro Trp
                85                  90                  95

Thr Phe Gly Gly Gly Thr Lys Leu Glu Ile Lys Arg Gly Gly Gly Gly
            100                 105                 110

Ser Gly Gly Gly Gly Ser Gly Gly Gly Gly Ser Ala Asp Val Gln Leu
        115                 120                 125

Gln Glu Ser Gly Pro Ser Leu Val Lys Pro Ser Gln Ser Leu Ser Leu
    130                 135                 140

Thr Cys Thr Val Thr Gly Tyr Ser Ile Thr Ser Asp Phe Ala Trp Asn
145                 150                 155                 160

Trp Ile Arg Gln Phe Pro Gly Asn Lys Leu Glu Trp Met Gly Tyr Ile
                165                 170                 175

Ser Tyr Ser Gly Asn Thr Arg Tyr Asn Pro Ser Leu Lys Ser Arg Ile
            180                 185                 190

Ser Ile Thr Arg Asp Thr Ser Lys Asn Gln Phe Phe Leu Gln Leu Asn
        195                 200                 205

Ser Val Thr Ile Glu Asp Thr Ala Thr Tyr Tyr Cys Val Thr Ala Gly
    210                 215                 220

Arg Gly Phe Pro Tyr Trp Gly Gln Gly Thr Leu Val Thr Val Ser Ala
225                 230                 235                 240

Thr Thr Thr Pro Ala Pro Arg Pro Pro Thr Pro Ala Pro Thr Ile Ala
                245                 250                 255

Ser Gln Pro Leu Ser Leu Arg Pro Glu Ala Cys Arg Pro Ala Ala Gly
            260                 265                 270

Gly Ala Val His Thr Arg Gly Leu Asp Phe Ala Cys Asp Phe Trp Val
        275                 280                 285

Leu Val Val Val Gly Gly Val Leu Ala Cys Tyr Ser Leu Leu Val Thr
    290                 295                 300

Val Ala Phe Ile Ile Phe Trp Val Arg Ser Lys Arg Ser Arg Leu Leu
305                 310                 315                 320

His Ser Asp Tyr Met Asn Met Thr Pro Arg Arg Pro Gly Pro Thr Arg
                325                 330                 335

Lys His Tyr Gln Pro Tyr Ala Pro Pro Arg Asp Phe Ala Ala Tyr Arg
            340                 345                 350

Ser Arg Val Lys Phe Ser Arg Ser Ala Asp Ala Pro Ala Tyr Gln Gln
        355                 360                 365

Gly Gln Asn Gln Leu Tyr Asn Glu Leu Asn Leu Gly Arg Arg Glu Glu
    370                 375                 380

Tyr Asp Val Leu Asp Lys Arg Arg Gly Arg Asp Pro Glu Met Gly Gly
385                 390                 395                 400

Lys Pro Gln Arg Arg Lys Asn Pro Gln Glu Gly Leu Tyr Asn Glu Leu
                405                 410                 415

Gln Lys Asp Lys Met Ala Glu Ala Tyr Ser Glu Ile Gly Met Lys Gly
            420                 425                 430

Glu Arg Arg Arg Gly Lys Gly His Asp Gly Leu Tyr Gln Gly Leu Ser
        435                 440                 445

Thr Ala Thr Lys Asp Thr Tyr Asp Ala Leu His Met Gln Ala Leu Pro
    450                 455                 460

Pro Arg
465

<210>  44
<211>  235
<212>  PRT
<213>  Artificial sequence

<220>
<223>  FLT3L amino acid sequence

<400>  44

Met Thr Val Leu Ala Pro Ala Trp Ser Pro Thr Thr Tyr Leu Leu Leu
1               5                   10                  15

Leu Leu Leu Leu Ser Ser Gly Leu Ser Gly Thr Gln Asp Cys Ser Phe
            20                  25                  30

Gln His Ser Pro Ile Ser Ser Asp Phe Ala Val Lys Ile Arg Glu Leu
        35                  40                  45

Ser Asp Tyr Leu Leu Gln Asp Tyr Pro Val Thr Val Ala Ser Asn Leu
    50                  55                  60

Gln Asp Glu Glu Leu Cys Gly Gly Leu Trp Arg Leu Val Leu Ala Gln
65                  70                  75                  80

Arg Trp Met Glu Arg Leu Lys Thr Val Ala Gly Ser Lys Met Gln Gly
                85                  90                  95

Leu Leu Glu Arg Val Asn Thr Glu Ile His Phe Val Thr Lys Cys Ala
            100                 105                 110

Phe Gln Pro Pro Pro Ser Cys Leu Arg Phe Val Gln Thr Asn Ile Ser
        115                 120                 125

Arg Leu Leu Gln Glu Thr Ser Glu Gln Leu Val Ala Leu Lys Pro Trp
    130                 135                 140

Ile Thr Arg Gln Asn Phe Ser Arg Cys Leu Glu Leu Gln Cys Gln Pro
145                 150                 155                 160

Asp Ser Ser Thr Leu Pro Pro Pro Trp Ser Pro Arg Pro Leu Glu Ala
                165                 170                 175

Thr Ala Pro Thr Ala Pro Gln Pro Pro Leu Leu Leu Leu Leu Leu Leu
            180                 185                 190

Pro Val Gly Leu Leu Leu Leu Ala Ala Ala Trp Cys Leu His Trp Gln
        195                 200                 205

Arg Thr Arg Arg Arg Thr Pro Arg Pro Gly Glu Gln Val Pro Pro Val
    210                 215                 220

Pro Ser Pro Gln Asp Leu Leu Leu Val Glu His
225                 230                 235

<210>  45
<211>  540
<212>  PRT
<213>  Artificial sequence

<220>
<223>  human IL12 amino acid sequence

<400>  45

Met Cys His Gln Gln Leu Val Ile Ser Trp Phe Ser Leu Val Phe Leu
1               5                   10                  15

Ala Ser Pro Leu Val Ala Ile Trp Glu Leu Lys Lys Asp Val Tyr Val
            20                  25                  30

Val Glu Leu Asp Trp Tyr Pro Asp Ala Pro Gly Glu Met Val Val Leu
        35                  40                  45

Thr Cys Asp Thr Pro Glu Glu Asp Gly Ile Thr Trp Thr Leu Asp Gln
    50                  55                  60

Ser Ser Glu Val Leu Gly Ser Gly Lys Thr Leu Thr Ile Gln Val Lys
65                  70                  75                  80

Glu Phe Gly Asp Ala Gly Gln Tyr Thr Cys His Lys Gly Gly Glu Val
                85                  90                  95

Leu Ser His Ser Leu Leu Leu Leu His Lys Lys Glu Asp Gly Ile Trp
            100                 105                 110

Ser Thr Asp Ile Leu Lys Asp Gln Lys Glu Pro Lys Asn Lys Thr Phe
        115                 120                 125

Leu Arg Cys Glu Ala Lys Asn Tyr Ser Gly Arg Phe Thr Cys Trp Trp
    130                 135                 140

Leu Thr Thr Ile Ser Thr Asp Leu Thr Phe Ser Val Lys Ser Ser Arg
145                 150                 155                 160

Gly Ser Ser Asp Pro Gln Gly Val Thr Cys Gly Ala Ala Thr Leu Ser
                165                 170                 175

Ala Glu Arg Val Arg Gly Asp Asn Lys Glu Tyr Glu Tyr Ser Val Glu
            180                 185                 190

Cys Gln Glu Asp Ser Ala Cys Pro Ala Ala Glu Glu Ser Leu Pro Ile
        195                 200                 205

Glu Val Met Val Asp Ala Val His Lys Leu Lys Tyr Glu Asn Tyr Thr
    210                 215                 220

Ser Ser Phe Phe Ile Arg Asp Ile Ile Lys Pro Asp Pro Pro Lys Asn
225                 230                 235                 240

Leu Gln Leu Lys Pro Leu Lys Asn Ser Arg Gln Val Glu Val Ser Trp
                245                 250                 255

Glu Tyr Pro Asp Thr Trp Ser Thr Pro His Ser Tyr Phe Ser Leu Thr
            260                 265                 270

Phe Cys Val Gln Val Gln Gly Lys Ser Lys Arg Glu Lys Lys Asp Arg
        275                 280                 285

Val Phe Thr Asp Lys Thr Ser Ala Thr Val Ile Cys Arg Lys Asn Ala
    290                 295                 300

Ser Ile Ser Val Arg Ala Gln Asp Arg Tyr Tyr Ser Ser Ser Trp Ser
305                 310                 315                 320

Glu Trp Ala Ser Val Pro Cys Ser Gly Gly Gly Gly Ser Gly Gly Gly
                325                 330                 335

Gly Ser Gly Gly Gly Gly Ser Arg Asn Leu Pro Val Ala Thr Pro Asp
            340                 345                 350

Pro Gly Met Phe Pro Cys Leu His His Ser Gln Asn Leu Leu Arg Ala
        355                 360                 365

Val Ser Asn Met Leu Gln Lys Ala Arg Gln Thr Leu Glu Phe Tyr Pro
    370                 375                 380

Cys Thr Ser Glu Glu Ile Asp His Glu Asp Ile Thr Lys Asp Lys Thr
385                 390                 395                 400

Ser Thr Val Glu Ala Cys Leu Pro Leu Glu Leu Thr Lys Asn Glu Ser
                405                 410                 415

Cys Leu Asn Ser Arg Glu Thr Ser Phe Ile Thr Asn Gly Ser Cys Leu
            420                 425                 430

Ala Ser Arg Lys Thr Ser Phe Met Met Ala Leu Cys Leu Ser Ser Ile
        435                 440                 445

Tyr Glu Asp Leu Lys Met Tyr Gln Val Glu Phe Lys Thr Met Asn Ala
    450                 455                 460

Lys Leu Leu Met Asp Pro Lys Arg Gln Ile Phe Leu Asp Gln Asn Met
465                 470                 475                 480

Leu Ala Val Ile Asp Glu Leu Met Gln Ala Leu Asn Phe Asn Ser Glu
                485                 490                 495

Thr Val Pro Gln Lys Ser Ser Leu Glu Glu Pro Asp Phe Tyr Lys Thr
            500                 505                 510

Lys Ile Lys Leu Cys Ile Leu Leu His Ala Phe Arg Ile Arg Ala Val
        515                 520                 525

Thr Ile Asp Arg Val Met Ser Tyr Leu Asn Ala Ser
    530                 535                 540

<210>  46
<211>  187
<212>  PRT
<213>  Artificial sequence

<220>
<223>  IFNbeta

<400>  46

Met Thr Asn Lys Cys Leu Leu Gln Ile Ala Leu Leu Leu Cys Phe Ser
1               5                   10                  15

Thr Thr Ala Leu Ser Met Ser Tyr Asn Leu Leu Gly Phe Leu Gln Arg
            20                  25                  30

Ser Ser Asn Phe Gln Cys Gln Lys Leu Leu Trp Gln Leu Asn Gly Arg
        35                  40                  45

Leu Glu Tyr Cys Leu Lys Asp Arg Met Asn Phe Asp Ile Pro Glu Glu
    50                  55                  60

Ile Lys Gln Leu Gln Gln Phe Gln Lys Glu Asp Ala Ala Leu Thr Ile
65                  70                  75                  80

Tyr Glu Met Leu Gln Asn Ile Phe Ala Ile Phe Arg Gln Asp Ser Ser
                85                  90                  95

Ser Thr Gly Trp Asn Glu Thr Ile Val Glu Asn Leu Leu Ala Asn Val
            100                 105                 110

Tyr His Gln Ile Asn His Leu Lys Thr Val Leu Glu Glu Lys Leu Glu
        115                 120                 125

Lys Glu Asp Phe Thr Arg Gly Lys Leu Met Ser Ser Leu His Leu Lys
    130                 135                 140

Arg Tyr Tyr Gly Arg Ile Leu His Tyr Leu Lys Ala Lys Glu Tyr Ser
145                 150                 155                 160

His Cys Ala Trp Thr Ile Val Arg Val Glu Ile Leu Arg Asn Phe Tyr
                165                 170                 175

Phe Ile Asn Arg Leu Thr Gly Tyr Leu Arg Asn
            180                 185